# EUROPEAN PATENT SPECIFICATION

(11) **EP 2 566 979 B1**
(45) Date of publication and mention of the grant of the patent: **25.02.2015**
(21) Application number: 11727131.2
(22) Date of filing: 05.05.2011
(51) Int. Cl.: C12Q 1/68

(54) **ASSAYS, COMPOSITIONS AND METHODS FOR DETECTING DRUG RESISTANT MICRO-ORGANISMS**
TESTS, ZUSAMMENSETZUNGEN UND VERFAHREN FÜR DEN NACHWEIS VON WIRKSTOFFRESISTENTEN MIKROORGANISMEN
DOSAGES, COMPOSITIONS ET MÉTHODES DE DÉTECTION DE MICRO-ORGANISMES PHARMACORÉSISTANTS

(30) Priority: 25.06.2010 WO PCT/EP2010/059105; 05.05.2010 WO PCT/EP2010/056122
(43) Date of publication of application: 13.03.2013
(73) Proprietor: Check-Points Holding B.V., 6709 PD Wageningen (NL)
(72) Inventor: VOS, Pieter, 3911 VA Rhenen (NL)
(74) Representative: Michalík, Andrej
(86) International application number: PCT/EP2011/057217
(87) International publication number: WO 2011/138402

(56) References cited:
- WO-A1-02/086153
- WO-A2-2004/106547
- NIEDERHAUSER C ET AL: "Use of the ligase detection reaction-polymerase chain reaction to identify point mutations in extended-spectrum beta-lactamases.", EUROPEAN JOURNAL OF CLINICAL MICROBIOLOGY & INFECTIOUS DISEASES : OFFICIAL PUBLICATION OF THE EUROPEAN SOCIETY OF CLINICAL MICROBIOLOGY JUN 2000 LNKD- PUBMED:10947227, vol. 19, no. 6, June 2000 (2000-06), pages 477-480, XP002606625, ISSN: 0934-9723
- KIM J ET AL: "Rapid discriminatory detection of genes coding for SHV beta-lactamases by ligase chain reaction", ANTIMICROBIAL AGENTS AND CHEMOTHERAPY, AMERICAN SOCIETY FOR MICROBIOLOGY, WASHINGTON, DC, US LNKD- DOI:10.1128/AAC.44.7.1860-1864.2000, vol. 44, no. 7, 1 July 2000 (2000-07-01), pages 1860-1864, XP002402613, ISSN: 0066-4804
- RANDEGGER C C ET AL: "Real-time PCR and melting curve analysis for reliable and rapid detection of SHV extended-spectrum beta-lactamases", ANTIMICROBIAL AGENTS AND CHEMOTHERAPY, AMERICAN SOCIETY FOR MICROBIOLOGY, WASHINGTON, DC, US LNKD- DOI:10.1128/AAC.45.6.1730-1736.2001, vol. 45, no. 6, 1 June 2001 (2001-06-01), pages 1730-1736, XP002420863, ISSN: 0066-4804
- GRIMM VERENA ET AL: "Use of DNA microarrays for rapid genotyping of TEM beta-lactamases that confer resistance", JOURNAL OF CLINICAL MICROBIOLOGY, AMERICAN SOCIETY FOR MICROBIOLOGY, WASHINGTON, DC, US LNKD- DOI:10.1128/JCM.42.8.3766-3774.2004, vol. 42, no. 8, 1 August 2004 (2004-08-01) , pages 3766-3774, XP002391573, ISSN: 0095-1137
- NAAS T ET AL: "Evaluation of a DNA Microarray, the Check-Points ESBL/KPC Array, for Rapid Detection of TEM, SHV, and CTX-M Extended-Spectrum beta-Lactamases and KPC Carbapenemases", ANTIMICROBIAL AGENTS AND CHEMOTHERAPY, vol. 54, no. 8, 14 June 2010 (2010-06-14), pages 3086-3092, XP009140424,
- STUART J COHEN ET AL: "Rapid detection of TEM, SHV and CTX-M extended-spectrum beta-lactamases in Enterobacteriaceae using ligation-mediated amplification with microarray analysis", JOURNAL OF ANTIMICROBIAL CHEMOTHERAPY, vol. 65, no. 7, 12 May 2010 (2010-05-12), pages 1377-1381, XP002606455, ISSN: 0305-7453
- MIKHAILOVICH V ET AL: "Identification of rifampin-resistant mycobacterium tuberculosis strains by hybridization, PCR and ligase detection reaction on oligonucleotide microchips", JOURNAL OF CLINICAL MICROBIOLOGY, AMERICAN SOCIETY FOR MICROBIOLOGY, WASHINGTON, DC, US LNKD- DOI:10.1128/JCM.39.7.2531-2540.2001, vol. 39, no. 7, 1 July 2001 (2001-07-01), pages 2531-2540, XP002970585, ISSN: 0095-1137
- BUSTI E ET AL: "Bacterial discrimination by means of a universal array approach mediated by LDR (ligase detection reaction)", BMC MICROBIOLOGY, BIOMED CENTRAL, LONDON, GB LNKD- DOI:10.1186/1471-2180-2-27, vol. 2, 20 September 2002 (2002-09-20), pages 1-12, XP002262041, ISSN: 1471-2180
- PITOUT JOHANN D D: "Molecular detection of bacteria producing newer of beta-lactamases", CURRENT GENOMICS, vol. 7, no. 3, May 2006 (2006-05), pages 171-177, XP009140429, ISSN: 1389-2029
- BRADFORD P A: "Extended-spectrum beta-lactamases in the 21st century: characterization, epidemiology, and detection of this important resistance threat", CLINICAL MICROBIOLOGY REVIEWS, WASHINGTON, DC, US LNKD- DOI:10.1128/CMR.14.4.933-951.2001, vol. 14, no. 4, 1 October 2001 (2001-10-01), pages 933-951, XP002391572, ISSN: 0893-8512

## Description

### Field of the invention

The present invention relates to assays, compositions and methods for the detection and discrimination of specific gene sequences encoding antibiotic resistance in a sample. The presence of such gene sequences is a very reliable indicator of antibiotic resistance in bacteria having these genes, which results in highly reduced susceptibility to antibiotics. Fast and accurate knowledge of the antibiotic resistance profiles of bacteria causing specific infections in patients enables the clinician to choose the best treatment options, and may contribute to prevent the spreading of such bacteria to other persons.

### Background of the invention

Beta-lactams are one of the most used and most effective antibiotics in treatment of infections in humans and animals. However, over the years many bacteria have become more and more insensitive to beta-lactam antibiotics. An important cause for this is that many bacteria have acquired beta-lactamase genes through horizontal gene transfer, and therefore now express beta-lactamases that break down beta-lactam antibiotics. This is a particular problem in gram negative bacteria, where various strains express beta-lactamases that are capable of hydrolyzing a wide range of beta-lactam antibiotics. Such bacteria are also named ESBLs, which is short for Extended Spectrum Beta-Lactamases. Typically, ESBLs are responsible for the resistance to beta-lactam (β-lactam) antibiotics such as penicillins, cephalosporins, monobactams cephamycins and carbapenems, including cefotaxime, ceftriaxone, ceftazidime, the oxyimino-monobactam aztreonam, and the carbapenems imipenem, meropenem, ertapenem and doripenem.

Many different beta-lactamases have been found in ESBLs. The table below depicts many of the beta-lactamases found in gram negative bacteria, but is not exhaustive, and it should be noted that this application also includes beta-lactamases that are not presented in this table. An important omission in this table is NDM-1, a metallo-beta-lactamase, capable of breaking down most carbapenems.

Gram negative bacteria that carry ESBL pose a serious health risk when people become infected with them, because infections may be very difficult to treat. A particular problem is also that such bacteria are easily transmitted from human to human, from human to animal and from animal to human. Implicating that a person carrying an ESBL may easily transmit the bacteria to others with which he/she is in close contact. This is a major problem in hospital wards. Therefore, accurate detection of ESBL is very important, both for patient treatment as well as hospital hygiene.

The spread of beta-lactamases between bacteria has increased the resistance of bacteria to beta-lactam drugs. The administration of beta-lactam drugs to patients with bacteria resistant to those drugs selects for those bacteria and leads to an increase in the transmission of beta-lactamases. Thus, there is a need to rapidly detect bacteria expressing specific beta-lactamases so that an appropriate therapeutic regimen is selected for a given patient and the likelihood of the spread of resistant bacteria is reduced.

| Acquired β-lactamases with hydrolytic activity against extended-spectrum cephalosporins and/or carbapenems | | | |
|---|---|---|---|
| β-Lactamase classes | ESBL_{A} | ESBL_{M} | ESBL_{CARBA} |
| | High prevalent ESBL_{A} | ESBL_{M-C}(Plasmid-mediated AmpC) | ESBL_{CARBA-A} |
| | CTX-M | | KPC |
| | TEM-ESBLs | CMY | GES-2, -4, -5, -6, -8 |
| | SHV-ESBLs | FOX | NMC |
| | VEB | MIR | SME |
| | PER | MOX | IMI-1, -2 |
| | | DHA | |
| | | LAT | |
| | | BIL | |
| | | ACT | |
| | | ACC | |
| | Low prevalent ESBL_{A} | ESBL_{M-D}(OXA-ESBL) | ESBL_{CARBA-B}(MBL) |
| | GES-1, -3, -7, -9 | OXA-10-group | IMP |
| | SFO-1 | OXA-13-group | VIM |
| | BES-1 | OXA-2-group | SPM-1 |
| | BEL-1 | OXA-18 | GIM-1 |
| | TLA | OXA-45 | SIM-1 |
| | IBC | | AIM-1 |
| | CMT^{a} | | ESBL_{CARBA-D}(OXA-carbapenemases) |
| | | | OXA-23-group |
| | | | OXA-24-group |
| | | | OXA-48^{b} |
| | | | OXA-58-group |
| Operational definition | Non-susceptibility to extend ed-spectru m cephalosporins | Non-susceptibility to extend ed-spectru m cephalosporins | Non-susceptibility to extend ed-spectru m cephalosporins and at least one carbapenem |
| | AND | AND | |
| | clavulanate synergy | phenotypic detection (ESBL_{M-C}) | AND |
| | | | ESBL_{CARBA} detected with phenotypic and/or genotypic methods |
| | | OR | |
| | | genotypic detection (ESBL_{M-D}) | |

| | | | |
|---|---|---|---|
| Table showing the most important ESBL genes, categorized in terms of prevalence, and enzyme characteristics. (N.B.: is not included in this table. NDM-1 is an MBL, and groups together with IMP, VIM, SPM, GIM SIM and AIM). | | | |

Classical methods for detecting ESBL rely on phenotyping using growth of ESBL-suspected bacteria in media supplemented with different types of beta-lactam antibiotics. These methods are inherently slow and are unable to discriminate clearly between the various beta-lactamases. Furthermore, these methods require a high level of expertise to execute and to interpret the results. These tests are therefore performed in specialized laboratories. The main disadvantage of this approach is that the results of the further identification of positive samples are very often too late for practical use as well as being relatively expensive. Such methods are therefore inadequate for advice on patient treatment. Such methods are also unable to assess whether two ESBL-carrying patients would have the same ESBL or two different ESBLs. Therefore, these methods cannot be used for hospital hygiene applications.

Various DNA-based assays and screening methods known in the art, such as real-time PCR, enable fast screening and provide a method for testing on presence or absence of pathogenic bacteria. However, e.g., after detecting the presence of micro-organisms, further identification requires multiparameter testing, which generally cannot be provided by these screening methods. For instance, real-time PCR allows only limited multiplexing of biomarkers. In addition, detection of small changes in DNA sequences such as SNPs is often difficult. Indeed, PCR detection of ESBL would generally be limited to one or very few beta-lactamase genes. Moreover, many ESBL genes have various gene variants, and such gene variants may have very different ESBL characteristics. An example of this is the TEM-gene. This gene has many variants that are either ESBL or non-ESBL. ESBL-variants would typically be able to hydrolyze third generation cephalosporins like Ceftazidim and Cefotaxime. Non-ESBL variants would not be capable of breaking down such third generation cephalosporins. The same holds through for the SHV-gene, that just as TEM, has many SHV ESBL as well as SHV non-ESBL variants. Another example is the GES gene. This gene has a number of variants, and one particular variant GES-2, is capable of breaking down carbapenems, while the other GES gene variants do not break down carbapenems, but third generation cephalosporins. It will be clear to a person skilled in the art that many PCRs and additional DNA sequencing of PCR products would be needed to cover all relevant ESBLs and in addition to discriminate between the various phenotypically different ESBL gene variants.

In view of time and cost considerations, the present invention provides optimized assays, compositions and methods for the detection, identification and characterization of ESBL nucleic acids, hence determining micro-organisms in samples resistant to beta-lactam antibiotics. The method detects whether or not antibiotic resistant micro-organisms are present in the sample and provides information on the nature of the genes responsible for the antibiotic resistance. This enables the clinician to treat patients in the best possible way and take adequate measures to prevent spreading of antibiotic bacteria.

Niederhauser C et al. (Eur J Clin Microbiol Infect Dis. 2000, vol. 19(6), 477-80) concern a ligase detection reaction-polymerase chain reaction (LDR-PCR) method to identify the Gly-Ser mutation at position 238 of the bla SHV-ESBL gene. To detect the LDR-PCR product, D1 Niederhauser C et al. employ gel electrophoresis and ethidium bromide staining.

WO 2004/106547 concerns methods for determining the presence of micro-organisms in samples, such as more specifically in food samples.

### Summary of the invention

The present invention relates to assays, compositions and methods for the detection and discrimination of specific gene sequences encoding antibiotic resistance in a sample. The nucleotide sequences encoding antibiotic resistance genes are uniquely identified. In particular, these sequences are hybridized to capture probes, enabling real-time PCR. For this purpose, the capture probes may also be covalently linked to amplification primers. Alternatively, detection of amplified products with hybridization to capture probes may be performed after amplification by hybridization to capture probes bound to a solid support such as microarrays and microspheres (beads). Finally, detection of amplification products during amplification in real-time using capture probes is combined with detection of such amplification products after amplification using the same and/or different capture probes.

### Legends to the figures and tables

- **Table 1**: Phenotypes of variants and corresponding amino acid substitutions that may be detected by the array system.
Table 1a: TEM variants
Table 1b: SHV variants
- **Table 2**: Detection of ESBL associated substitutions by microarray in 106 ESBL positive isolates.
- **Table 3**: Primers for PCR and sequencing of TEM, SHV and CTX-M genes.
- **Table 4**: Target ESBL nucleic acids for first and/or second nucleic acid probes.
- **Table 5**: ESBL real-time PCR detection and microarray-based typing of E.coli and K.pneumoniae strains.

### Supplementary Table 1

Nucleotide sequences of probes for ligation mediated amplification. The part of the probe complementary to TEM, SHV and CTX-M genes is shown. The part of the probe containing the primer binding sites and ZIP codes for hybridization to micro-array are not shown. To the left, probe names are shown with gene name (TEM, SHV, CTX-M1, 2, 9 and 8/25) and relevant amino acid Position of the mutations followed by a single letter indicating the amino acid codon detected by the probe. The upstream and downstream target-specific segments of each probe are shown. Boxes in the TEM and SHV probes represent codons detected by the probes and are respectively TEM-104 glutamic acid (GAG) and lysine (AAA), TEM-164 arginine (CGT), serine (AGT), cysteine (TGT) and histidine (CAT), TEM-84 isoleucine (ATT), TEM-100 serine (AGT), TEM-238 glycine (GGT) and serine (AGT), SHV-130 serine (AGC), SHV-238 glycine (GGC), serine (AGC) and alanine (GCC), SHV-240 glutamic acid (AGG) or lysine (AAA), SHV-179 aspartic acid (GAC), alanine (GCC), glycine (GGC) and asparagine (AAC). Note that probes for TEM-238, SHV-238 and SHV-240 are in antisense orientation. The CTX-M probes for families 1, 2, 8, 9 and 25 were chosen in regions were there is near sequence identity within each family and strong discrimination between families. For families 8 and 25 a combined probe was chosen detecting both families.

### Supplementary Table 2

Characteristics of the 213 test isolates (bacterial species, beta-lactamases, ESBL genotype, ESBL phenotype, and Array Result).
Supplementary Table 2a: ESBL Positive isolates (n=106)
Supplementary Table 2b: ESBL negative isolates (n=107)

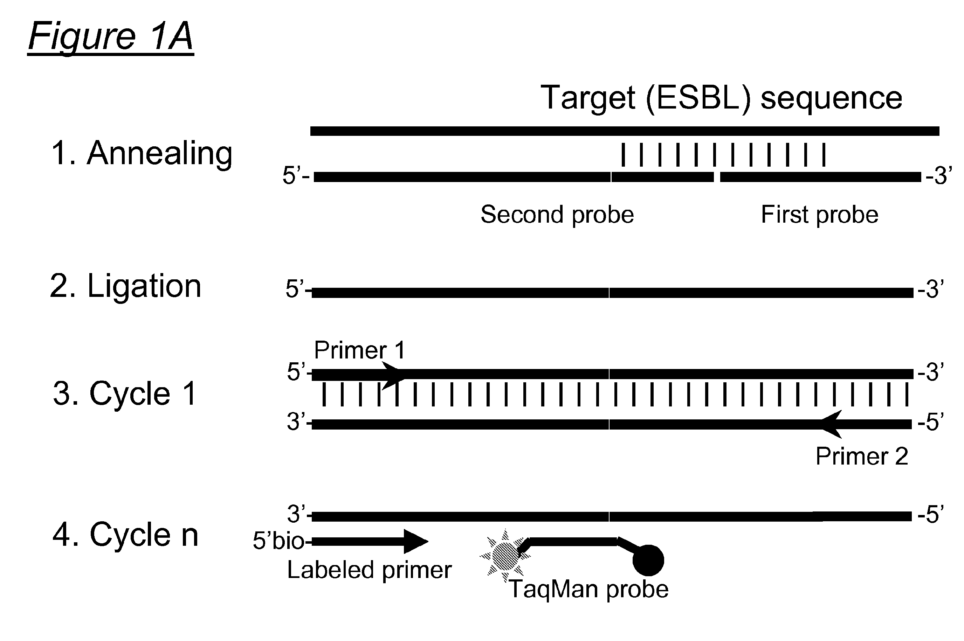

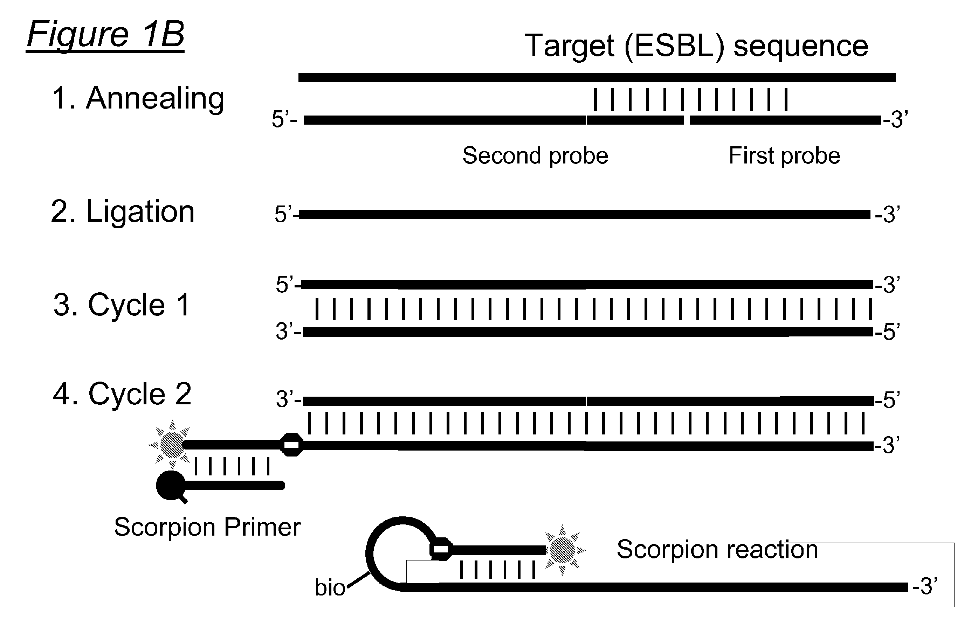

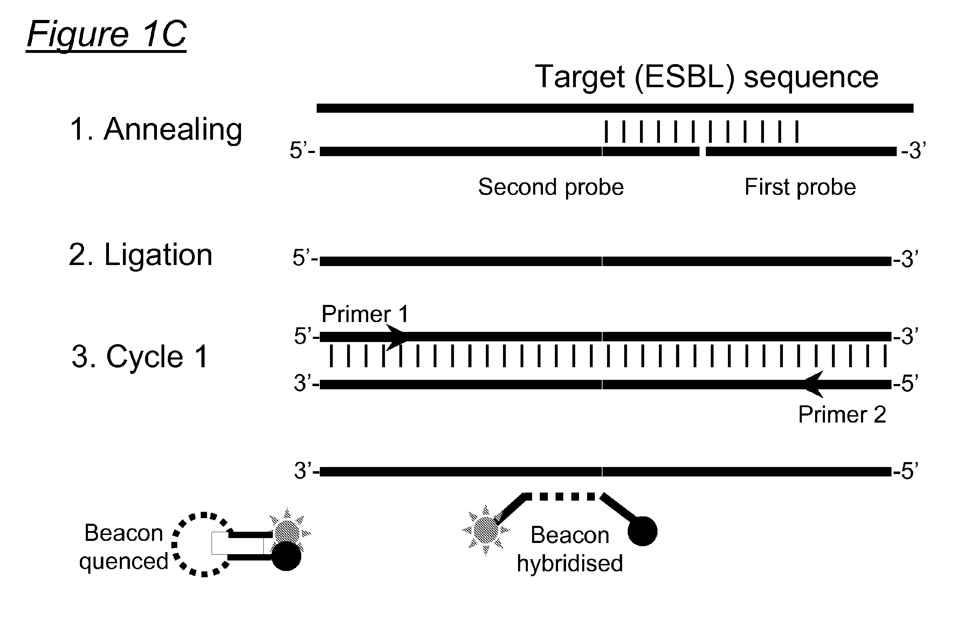

**Figure 1****:** Schematic representation of the real time detection of the amplification reaction. Figure 1a: Taqman probes; Figure 1b: Scorpion probes; and Figure 1c: Molecular Beacon probes.
**Figure 2** **Lay out of micro-array**
   Example of microarray picture (upper panel). The microarray contains 3 zones, each with 30 identical probe targets, allowing parallel analysis of 3 isolates. The layout of zone 1 is shown (lower panel). Each zone contains 24 probe targets for ESBL detection and 6 control spots.
   The control spots were designed to assess the following steps:
   *HybC*: Biotinylated probe complementary to spotted oligonucleotide at indicated array Position.
   *Pos C:* Internal reaction control consisting of DNA probe and complementary target DNA. Upon successful completion of the A-step a small amount of product will be amplified in the subsequent PCR that will be detected at the indicated Positions on the microarray.
   *Neg C:* Internal reaction control consisting of DNA probe but lacking any complementary target DNA. The intensity of the signal at the indicated array Positions specify the background noise
   *DNA C:* 2 DNA probes targeted at highly conserved sequences in *Enterobacteriaceae* and non-fermenters. At least one of these probes is expected to give a spot at the indicated Positions in case DNA of *Enterobacteriaceae* or non-fermenters is present in the reaction.

   The Positions in column 1 and 12 (upper panel) contain biotinylated oligonucleotides spotted on the microarray that are used as control spots for the staining process and reference spots for image analysis software. To allow visual inspection of the DNA microarray pictures spots interpreted as Positive by the software are marked with a green circle (not shown). The isolates analysed in the picture were ESBL negative (zone 1), CTX-M-1 Positive (zone 2) and CTX-M-9 Positive (zone 3).

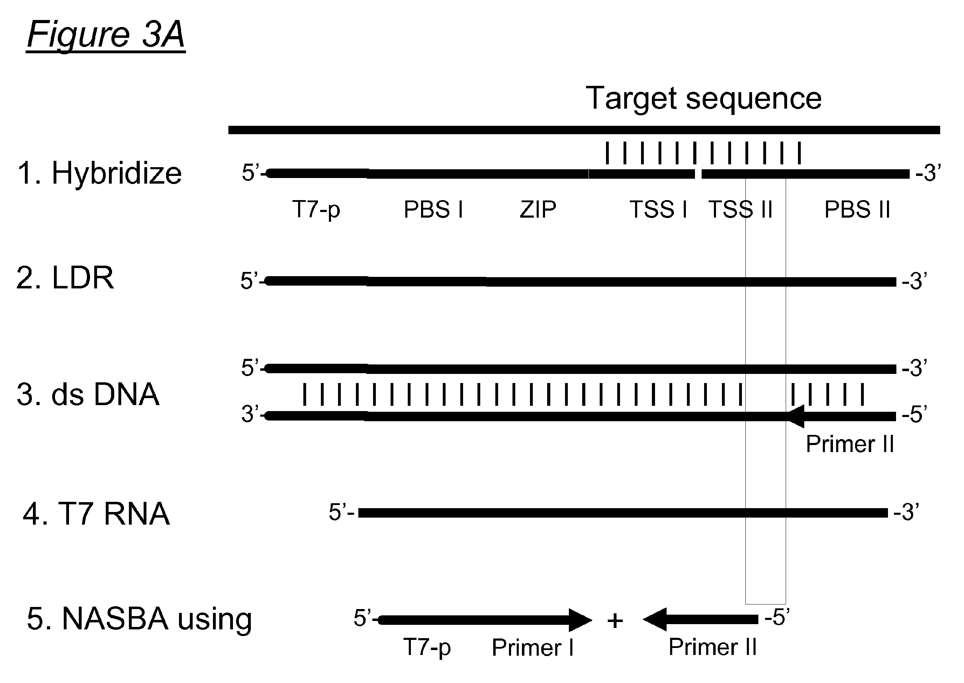

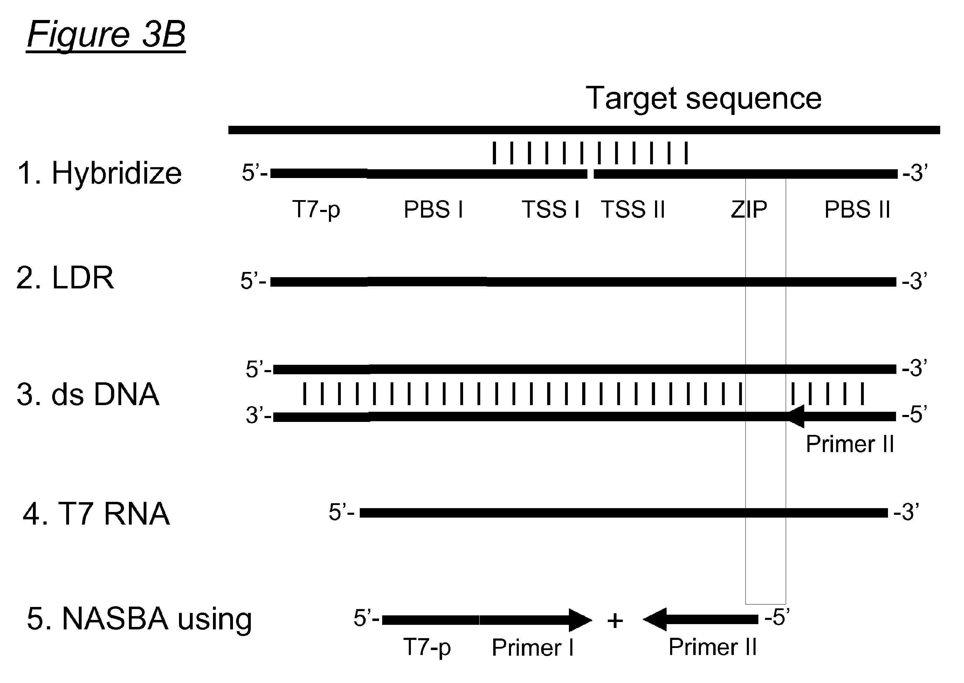

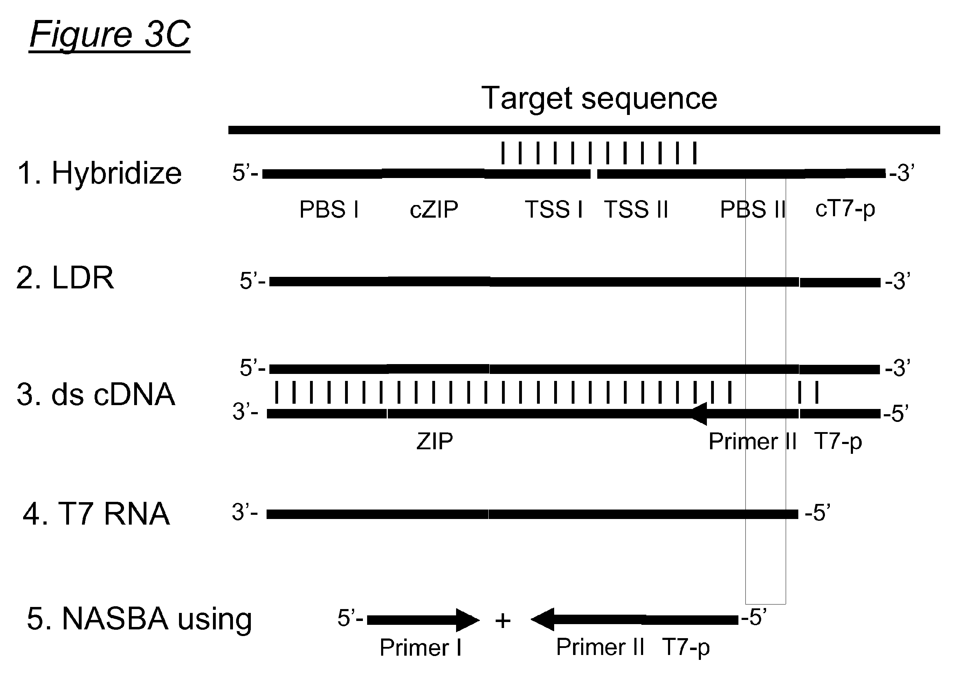

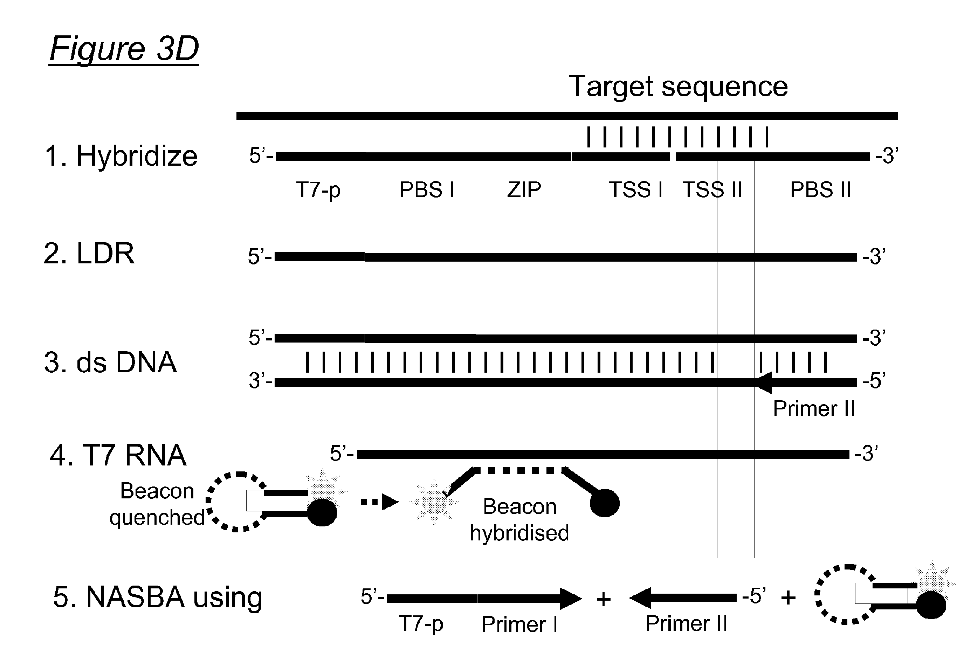

**Figure 3** **Schematic representation of RNA-based amplification techniques.**
   Figure 3A refers to the combination in which the first nucleic acid probe and primer I comprise the T7 promoter and ZIP is located on the first nucleic acid probe; Figure 3B refers to the combination in which the first nucleic acid probe and primer I comprise the T7 promoter and ZIP is located on the second nucleic acid probe; Figure 3C refers to the combination in which primer II comprises the T7 promoter, the second nucleic acid probe comprises a region that is complementary to the T7 promoter (cT7-p) and cZIP is located on the first nucleic acid probe. Figure 3D indicates the real time detection of the amplification reaction using molecular beacons. Molecular beacons carry a fluorescent molecule and a quencher at their extremities. The loop sequence of the beacon is specific and complementary (cDET) to the DET region on the amplicon. In this example ZIP functions as DET region. The beacon binds to the DET region and when it is opened, the quencher is distant from the fluorescent molecule and consequently allows the emission of fluorescence.

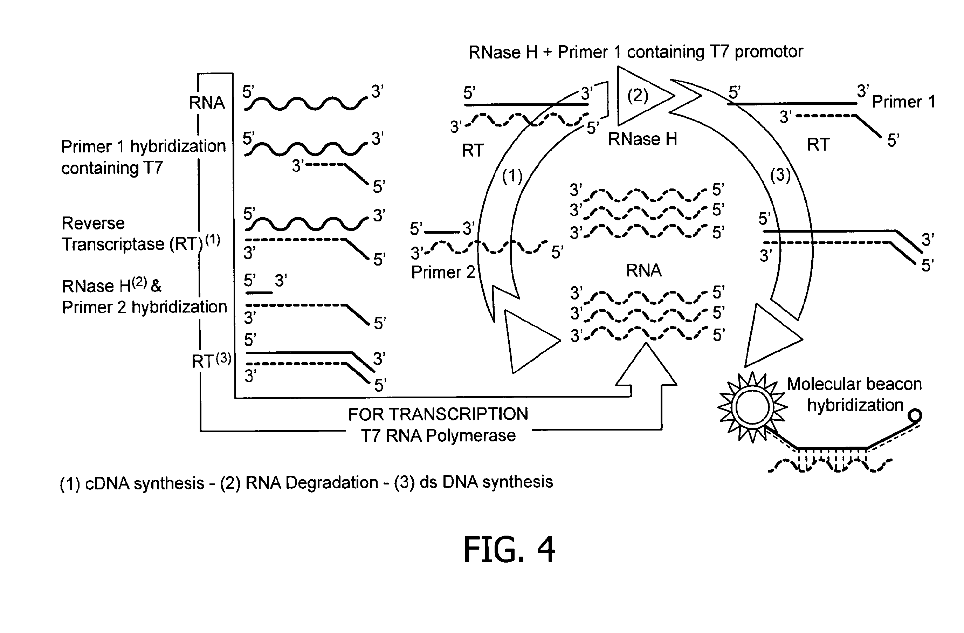

**Figure 4** **Schematic representation of the NASBA reaction**
   NASBA (Nucleic Acid Sequence Based Amplification) is an isothermal nucleic acid amplification technology resulting in single-stranded RNA molecule synthesis. NASBA technology is based on the concerted action of three enzymes: (1) a reverse transcriptase for cDNA synthesis, e.g. AMV reverse transcriptase, (2) RNase H for degradation of the RNA in the heteroduplex RNA-DNA and (3) T7 RNA polymerase for synthesis of RNA from the T7 promoter. Specific primers are used, one of them carrying the T7 promoter.

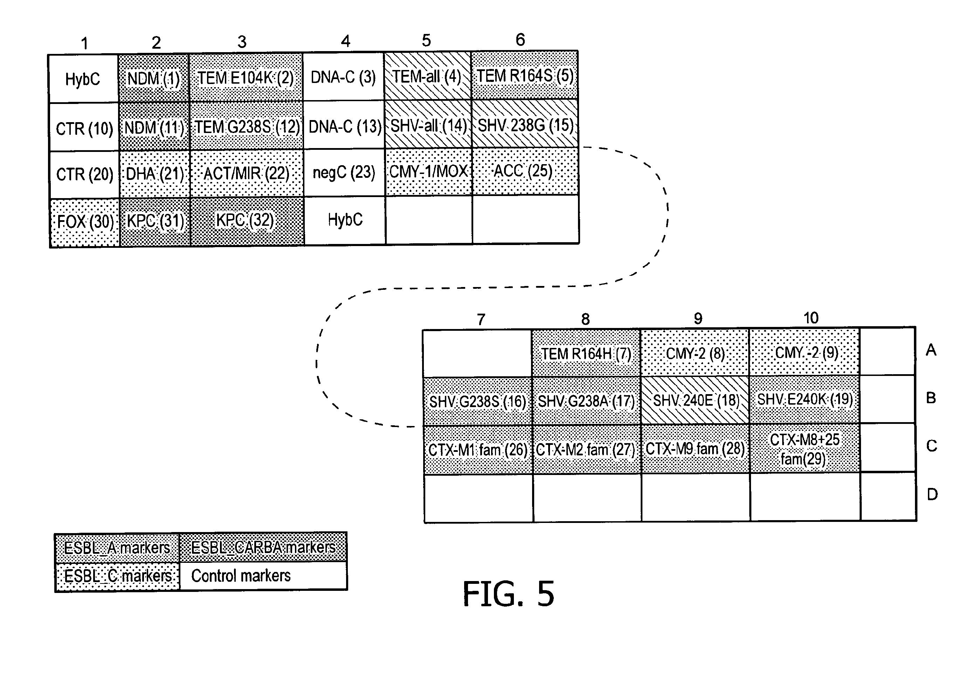

**Figure 5** **Layout of the ESBL array detecting ESBL_{A}, ESBL_{M} and/or ESBL_{CARBA}.genes.**
   Probes for the 3 ESBL groups are depicted in different shades of grey. The striped pattern probes detect TEM and SHV non-ESBL sequences. In parenthesis the array position of each of the probes is shown.

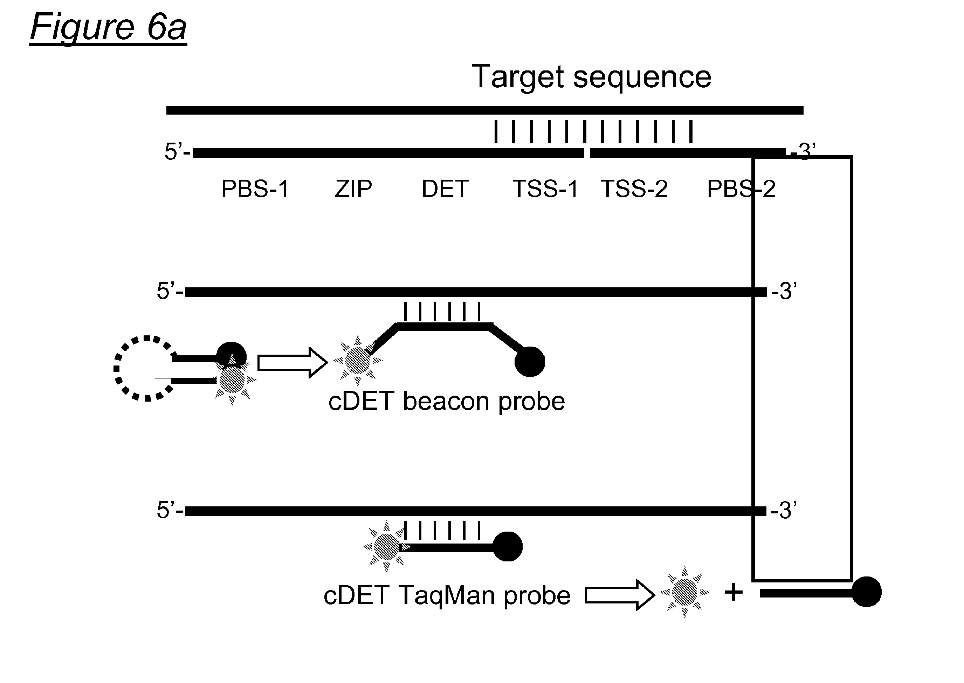

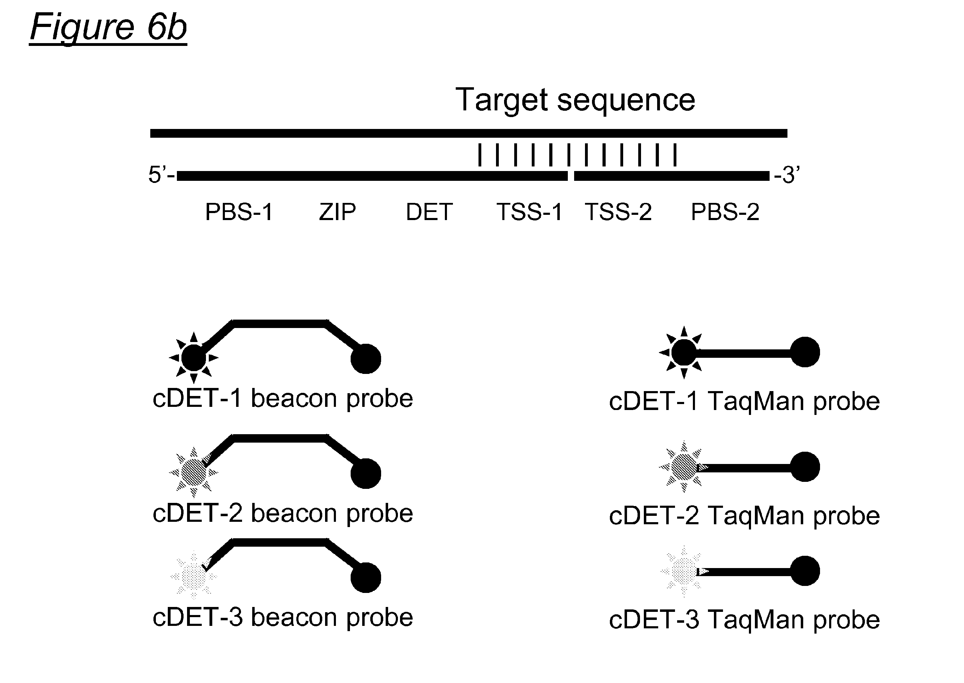

**Figure 6** **Design of probe pairs for real-time and microarray detection.**
   Figure 6a illustrates probe pairs hybridized to target sequence (top), the connected probe assembly hybridized to cDET beacon probe (middle) and the connected probe assembly hybridized to cDET TaqMan probe (bottom) and subsequent cleavage of the fluororescent reporter. Figure 6b illustrates cDET-1, cDET-2 and cDET-3 beacon and TaqMan probes used for real-time detection of ESBL_{A}, ESBL_{M} and ESBL_{CARBA}.genes of example 4.
   Exemplary sequences are:
   cDET-1 beacon: FAM-CGCTGC**CTTTCGAGAGAACCGGCTTCGAAG**GCAGCG-Dabcyl
   cDET-1 TaqMan: FAM-**CTTTCGAGAGAACCGGCTTCGAAG**-BHQ-1
   wherein FAM refers to the fluorescent reporter dye, and Dabcyl and BHQ-1 to the fluorescence quenchers. The cDET-1 sequence is depicted in bold; the beacon probe contains in addition a 5'and 3' complementary 6-mer sequence enabling the typical beacon stem structure.
   cDET-2 beacon: YY-CGCTGC**CAGTCTGAACGCAACTCTGCTGAT**GCAGCG-Dabcyl
   cDET-2 TaqMan: YY-**CAGTCTGAACGCAACTCTGCTGAT**-BHQ-1.
   wherein YY refers to the Yakima Yellow fluorescent reporter dye, and Dabcyl and BHQ-1 to the fluorescence quenchers. The cDET-2 sequence is depicted in bold; the beacon probe contains in addition a 5'and 3' complementary 6-mer sequence enabling the typical beacon stem structure.
   cDET-3 beacon: Cy5-CGCTGC**CAGTCTAGAGAACCGGCTGCTGAT**GCAGCG-BHQ-2
   cDET-3 TaqMan: Cy5-**CAGTCTAGAGAACCGGCTGCTGAT**-BHQ-2
   wherein Cy5 refers to the fluorescent reporter dye, and BHQ-2 to the fluorescence quenchers. The cDET-3 sequence is depicted in bold; the beacon probe contains in addition a 5'and 3' complementary 6-mer sequence enabling the typical beacon stem structure.

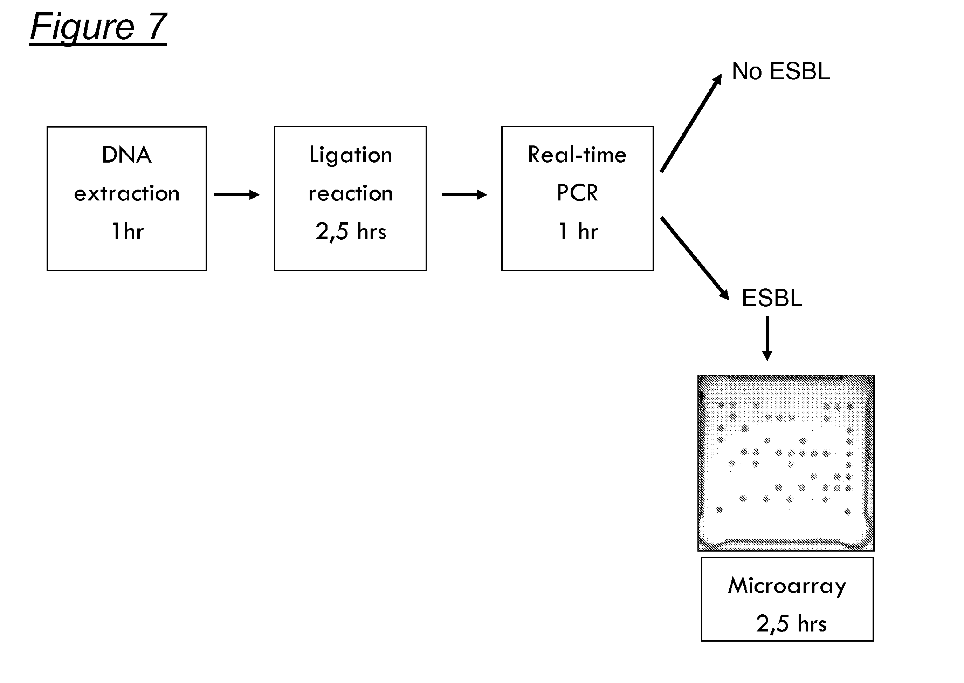

**Figure 7** **Flow scheme of the real-time detection and microarray-based typing of ESBL genes**

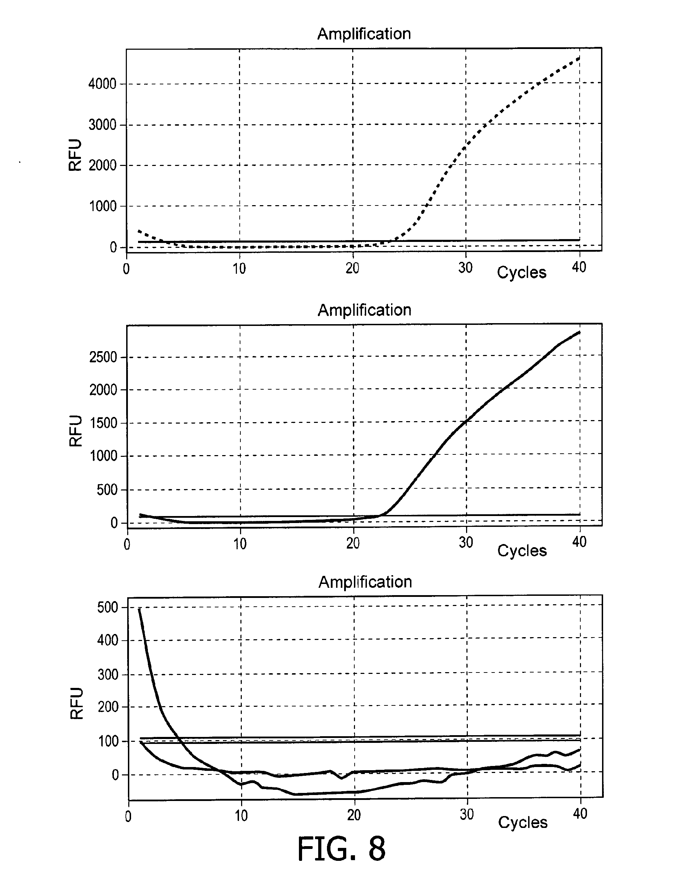

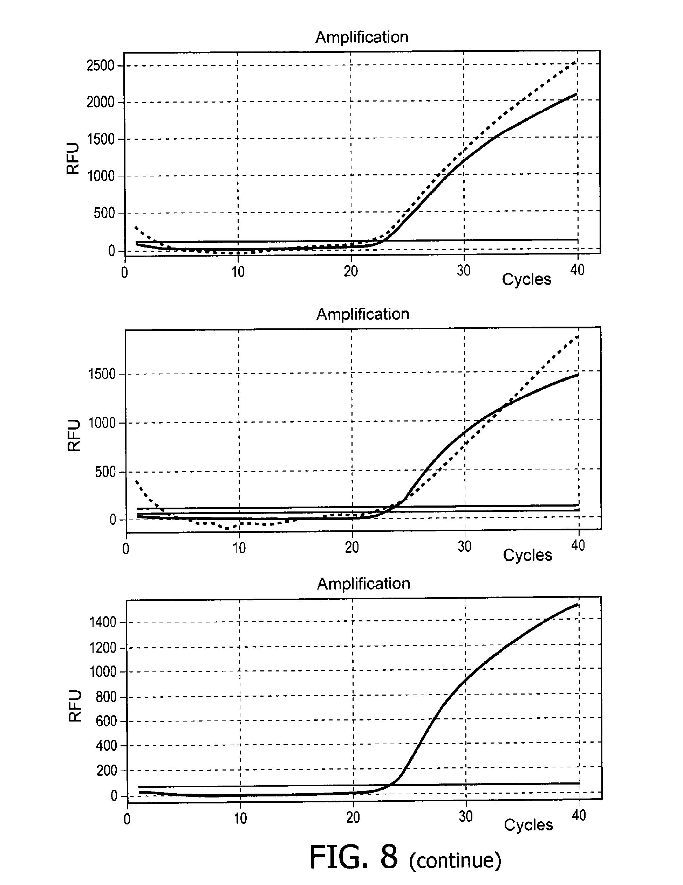

**Figure 8** **Various real-time PCR profiles.**
   The PCR profiles were obtained with the 155 bacterial strains from example 4. Y-axes indicate fluoresence units, X-axes indicate cycle numbers. Top left, FAM-ESBL_A profile; middle left YY-ESBL_M-C profile; bottom left, ESBL negative profile; top right, FAM-ESBL_A & YY-ESBL_C profiles; middle right, FAM-ESBL_A & Cy5-ESBL_CARBA profiles; bottom right, Cy5-ESBL_CARBA profile.

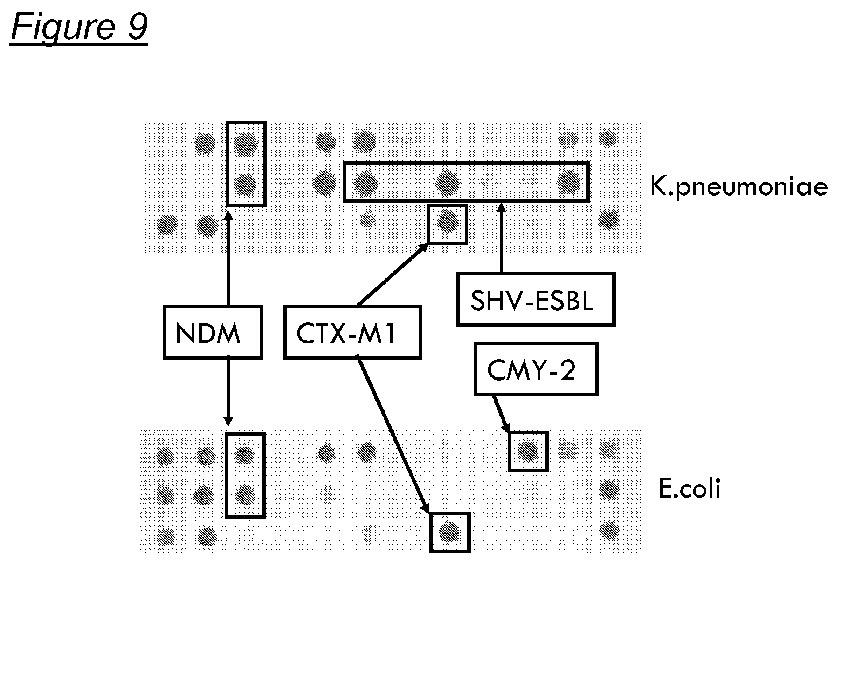

**Figure 9** **Microarray profiles**
   This shows the microarray-based typing of ESBL genes of one E.coli and one K.pneumoniae strain. The K.pneumoniae contains an ESBL_CARBA NDM gene, and two ESBL_A genes, SHV_ESBL and CTX-M1 type gene. The E.coli contains an ESBL_CARBA NDM gene, an ESBL_A CTX-M1 type gene, and an ESBL_C CMY-2 type gene.

### Detailed Description of the Invention

Before the present assays, compositions, devices and methods used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

As used herein, the terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of". The recitation of numerical ranges by endpoints includes all numbers and fractions subsumed within the respective ranges, as well as the recited endpoints. The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

In the present specification and the appended claims, the singular forms "a", "an", and "the" include the plural references, and vice versa, unless the context clearly indicates otherwise. Unless defined otherwise, all technical and scientific terms used herein have the same meaning as commonly understood by one of ordinary skill in the art.

The present invention relates to methods for detecting, identifying and characterizing ESBL micro-organisms. More specifically, the present invention relates to a method for determining the presence of ESBL nucleic acids in a sample by using a ligation detection reaction comprising a plurality of probe pairs, in which a step comprises the detection of the presence of ESBL nucleic acids via a detector molecule in Real Time detection, and/or via the identification and characterization of said ESBL nucleic acids via a labelled primer. The invention described in this application is able to detect a wide range of ESBL nucleic acids in a single test. In addition, the invention is able to discriminate between ESBL genes stricto sensu (genes for ESBL that are sensitive to inhibition by clavulanic acid), non-ESBL variants of ESBL genes, AmpC genes, metallo-beta-lactamase genes and carbapenemase genes. Presence/absence of ESBL is achieved by real-time analysis as described in this invention, further characterization is achieved by hybridization detection, e.g. to a solid phase such as a microarray, as further described in this invention.

In particular, the present invention relates to a method for determining the presence of extended spectrum beta-lactamases (ESBL) nucleic acids in a sample, comprising the steps of:
(a) extracting nucleic acids from micro-organisms, said nucleic acids comprising target ESBL nucleic acids,
(b) performing a ligase detection reaction (LDR) on said target ESBL nucleic acids, comprising:
   (b1) contacting a plurality of different probe pairs with said target ESBL nucleic acids,
      wherein each probe pair comprises a (identifiable) first nucleic acid probe and a (identifiable) second nucleic acid probe,
      wherein each probe pair being specific for a particular target ESBL nucleic acid;
   (b2) incubating said target ESBL nucleic acids with said plurality of different probe pairs under conditions allowing to hybridize said particular target ESBL nucleic acid, with at least one probe pair,
   (b3) ligating any essentially adjacent (identifiable) first nucleic acid probe and (identifiable) second nucleic acid probe [of a probe pair], to form a connected probe assembly;
   (b4) providing at least a set of two amplification primers,
   (b5) amplifying the connected probe assembly of step (b3) using the amplification primers of step (b4), thereby providing amplified target ESBL nucleic acid [ESBL amplicon],
(c) detecting the amplified target ESBL nucleic acid [ESBL amplicon], by performing a hybridization to a capture probe, or by performing Real Time (RT) detection and a hybridization to a capture probe, whereby the presence of an ESBL nucleic acid in a sample is determined.

The present invention further relates to a method according to the invention, wherein in step (c) detecting the amplified target ESBL nucleic acids [ESBL amplicon] is by performing Real Time (RT) detection and the hybridization to a capture probe.

The present invention further relates to a method according to the invention, wherein the hybridization to a capture probe is performed subsequently to RT detection.

As will be demonstrated further below, the present invention enabled for the first time simultaneous detection of ESBLs belonging to various families, e.g. the three most prevalent ESBL families: TEM, SHV and CTX-M. Evaluation of the diagnostic accuracy of the assay in 212 genotypically and phenotypically well-characterized isolates showed that the method according to the invention offers an attractive option for rapid and accurate detection or exclusion of ESBL nucleic acids. In the examples section it is shown that the method according to the present invention has a very high sensitivity (95%) while a specificity of 100%. The assay detects specific SNPs in ESBL nucleic acids. The method according to the present invention has several advantages in comparison with prior art testing. First, the time to result of the present method is shorter. For instance, results for 36 isolates could be obtained within the same working day, whereas phenotypic confirmatory tests require an overnight incubation. Secondly, the present method is accurate for species producing beta-lactamases that may interfere with phenotypic tests for ESBL production, such as *K*. *oxytoca* isolates with K1 hyperproduction and AmpC beta-lactamase producing isolates. Thirdly, the assay identifies the ESBL families involved (SHV, TEM or CTX-M) and provides information on the SNPs in the TEM and SHV ESBL gene(s). This information may be useful for infection control purposes. The flexibility of the present invention enables easy extension of the method with additional markers through the addition of new probes in case of changing epidemiology of the different ESBL families.

The present invention further relates a method according to the invention, wherein said ligation step (b3) comprises the use of a ligase.

The present invention further relates a method according to the invention, wherein the amplified target ESBL nucleic acids [ESBL amplicon] are labeled, preferably during amplification, more preferably by providing in step (b4) at least a set of two amplification primers wherein at least one primer is labelled.

The present invention further relates a method according to the invention, wherein said LDR comprises at least 4, preferably 5, 6, 7, 8, 9, 10 or even more different probe pairs.

The present invention further relates a method according to the invention, wherein said particular target ESBL nucleic acid is chosen from the group consisting of TEM, SHV and CTX.

The present invention further relates a method according to the invention, wherein said particular target ESBL nucleic acid is chosen from the group consisting of the nucleic acids characterized by sequence id of Table 4, including TEM wild type 104, TEM wild type 164, TEM wild type 238, TEM wild type 238/240, OXA 48 I, OXA 48 II, VIM 552C, VIM 246, IMP 114, IMP 480 I/II, SHV all, NDM-1 75, NDM-1 392, CMY II 304, CMY II 1199, CMY I MOX, ACC 606, MIR ACT, DHA 753, DHA 1110, TEM all, FOX, KPC I, KPC II, CTX-M8 + 25, CTX-M9 + 64, CTX-M2, and CTX-M1, preferably TEM-E104K, TEM-R164S, TEM-R164C, TEM-R164H, TEM-G238S, SHV-G238S, SHV-G238A, SHV-E240K, SHV-D179A, SHV-D179G, and SHV-D179N.

The present invention further relates a method according to the invention, wherein said different probe pairs are chosen from the group consisting of TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N and CTX-M1, CTX-M2, CTX-M9, and CTX-M8/25.

In a preferred embodiment, the present invention relates to a method according to the present invention wherein the presence of a group of related drug resistance conferring ESBL nucleic acids is determined in a sample. Many of the ESBL genes defined in the present application can be categorized into different classes, such as group A, group M-C and group CARBA, as defined in the earlier table showing the most important ESBL genes. While all individual genes may be detected and discriminated using the method according to the present invention, the detection and discrimination of a specific class of ESBL genes can be performed as well. This may be useful as it enables the simplification of the results obtained by the present method. Furthermore, it also allows the development of more simplified assays. Also, if only a limited number of real-time measuring capabilities are limited, the combination of ESBL genes into different classes and the detection of these combined groups still allows the detection of drug resistance conferred by the ESBL nucleic acid groups.

More particularly in the method according to the present invention the ESBL genes are divided in groups related to clinical relevance such as for instance Group ESBL_{A}, ESBL_{M} and/or ESBL_{CARBA}.

More particularly group ESBL_{A} comprises or consists of classical ESBL genes, conferring resistance to cephalosporines of 3rd and 4th generation, inhibited by Clavulanic Acid, and sensitive to Carbapenems, group ESBL_{M} comprises or consist of plasmid mediated AmpC genes (ESBL_{M-C}) and OXA-ESBL (ESBL_{M-D}), conferring resistance to cephalosporines of 3rd and 4th generation, not inhibited by Clavulanic Acid, and sensitive to Carbapenems and group ESBL_{CARBA} comprises or consists of ESBL genes conferring resistance to all β-lactamases including Carbapenems.

Using the method according to the present invention a plurality of different probe pairs may be used having a unique ZIP for detection and using a capture probe bound to a solid support, e.g. a microarray, as well as a DET for the detection using real-time PCR. The DET may not be a unique DET specific for a specific ESBL gene but it may be a DET specific for a specific group of ESBL genes, such as ESBL_{A}, ESBL_{M} and/or ESBL_{CARBA}. In this way the presence of any of the members of that specific group of ESBL genes can be detected in a sample using real-time PCR where fluorescence specific for the selected DET sequences of groups ESBL_{A}, ESBL_{M} and/or ESBL_{CARBA} is detected. In addition, the hybridization of the amplification products (specifically the ZIP or cZIP region) to a capture probe detects and determines what member of ESBL groups ESBL_{A}, ESBL_{M} and/or ESBL_{CARBA} is present in the sample and therefore responsible for the fluorescence in the real-time PCR. This allows the use of a limited number of real-time detection probes while still maintaining the full specificity of the detection method.

The present invention further relates a method according to the invention, wherein a probe pair being specific for a particular target ESBL nucleic acid comprises an identifier region, preferably a cZIP or ZIP.

The present invention further relates a method according to the invention, wherein said identifier region, preferably a cZIP or ZIP, is specific for TEM, SHV or CTX.

The present invention further relates a method according to the invention, wherein said identifier region, preferably a cZIP or ZIP, is specific for a target ESBL nucleic acid.

The present invention further relates a method according to the invention, wherein said identifier region, preferably a cZIP or ZIP, is specific for a particular target ESBL nucleic acid chosen from the group consisting of TEM-E104K, TEM-R164S, TEM-R164C, TEM-R164H, TEM-G238S, SHV-G238S, SHV-G238A, SHV-E240K, SHV-D179A, SHV-D179G, and SHV-D179N.

The present invention further relates a method according to the invention, wherein said identifier region, preferably a cZIP or ZIP, is specific for a probe pair chosen from the group consisting of TEM-104E, TEM-104K, TEM-164R, TEM-164S, TEM-164C, TEM-164H, TEM-841, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N and CTX-M1, CTX-M2, CTX-M9, and CTX-M8/25.

The present invention further relates a method according to the invention, wherein said identifier region hybridizes to said capture probe.

The present invention further relates a method according to the invention, wherein said capture probe comprises a ZIP, which is essentially complementary to a corresponding cZIP on said connected probe assembly, or wherein said capture probe comprises a cZIP which is essentially complementary to a corresponding ZIP on said connected probe assembly.

The present invention further relates a method according to the invention, wherein said capture probe is spatially addressable on a microarray.

The present invention further relates a method according to the invention, wherein the amplified target ESBL nucleic acids [ESBL amplicon] derived from at least two samples are hybridised to capture probes present on a single microarray.

The present invention further relates a method according to the invention, wherein the amplified target ESBL nucleic acids [ESBL amplicon] hybridised to the corresponding capture probes on a microarray results in a hybridisation pattern.

The present invention further relates a method according to the invention, wherein said Real Time (RT) detection comprises:
(i) providing at least one or more detector molecules, wherein said detector molecules detects the amplified target ESBL nucleic acids [ESBL amplicon];
(ii) monitoring the signal and/or the modulation of the signal of said detector molecule, thereby detecting the presence of said amplified target ESBL nucleic acids [ESBL amplicon].

The present invention further relates a method according to the invention, wherein said detector molecules are one or more oligonucleotide detector probes having a sequence at least partially complementary to said amplified target ESBL nucleic acids [ESBL amplicon] and including a fluorescent reporter molecule and a fluorescent quencher molecule capable of quenching the fluorescence of said reporter molecule, said oligonucleotide detector probe existing in at least one single-stranded, partially single-stranded or double-stranded conformation when unhybridized where said quencher molecule quenches the fluorescence of said reporter molecule, said oligonucleotide detector probe existing in at least one conformation when hybridized to said target nucleic acid where the fluorescence of said reporter molecule is unquenched.

The present invention further relates a method according to the invention, wherein the sequence of said oligonucleotide detector probes is at least complementary to at least one region of the first nucleic acid probe or at least one region of the second nucleic acid probe.

The present invention further relates a method according to the invention, wherein the first nucleic acid probe and/or the second nucleic acid probe comprises at least one DET region, which is
(i) essentially complementary to one or more oligonucleotide detector probes; and
(ii) essentially non-complementary to said target ESBL nucleic acid.

The present invention further relates a method according to the invention, wherein a cZIP or a ZIP on a connected probe assembly functions as a DET region.

The present specification further describes a kit for determining the presence of ESBL nucleic acids in a sample, comprising means for extracting nucleic acids from micro-organisms, means for specifically amplifying said ESBL nucleic acids, means for detecting the signal obtained from the amplified ESBL nucleic acids, means for analysing the amplified ESBL nucleic acids, and an instruction manual.

In a preferred embodiment, said drug resistance conferring nucleic acids are ESBL nucleic acids. Nevertheless, in view of the generality of the invention, in the following, the term ESBL nucleic acid may be replaced by drug resistance conferring nucleic acid, unless explicitly indicated otherwise.

Beta-lactamases are a family of enzymes that hydrolyze beta-lactam rings, such as beta-lactam rings of beta-lactam antibiotic drugs. Beta-lactamases are found in gram positive and gram negative bacteria and are responsible for the antibiotic resistance of many bacterial strains. These antibiotics have a common element in their molecular structure: a four-atom ring known as a beta-lactam. The beta-lactamase enzyme breaks that ring open, deactivating the molecule's antibacterial properties. Beta-lactam antibiotics are typically used to treat a wide variety of gram-positive and gram-negative bacteria.

Beta-lactamases can be classified on the basis of their primary structure into four molecular classes, namely classes A to D. Classes A, C and D have a serine residue at their active site and class B, or metallo-beta-lactamases, have zinc at their active site. Carbapenemases are a diverse group of beta-lactamases that include enzymes belonging to class A, B and D. Class A carbapenemases include the KPC-family, i.e. presently KPC 1 - 7 (more variants are to be expected in the near future). Class B carbapenemases include the IMP family, VIM family, NDM-1, GIM-I and SPM-I as well as others. Class D carbapenemases include OXA-23, OXA-24, OXA-40, OXA-48 and OXA-58 as well as others. AmpC beta-lactamases are class C enzymes and can be encoded by chromosomal genes or be plasmid-borne. AmpC beta-lactamases hydrolyze broad and extended-spectrum cephalosporins (i.e., cephamycins and oxyimino-beta-lactams).

Extended-spectrum beta-lactamases (ESBLs) according to the invention, is any beta-lactamase, preferably beta-lactamases that hydrolyze, *i.e.* inactivate, cephalosporins and carbapenems. In the present invention, the broad definition of ESBL including ampC enzymes and carbapenemases is contemplated, for instance, as described in Giske et al. (J. Antimicrob. Chemother. 2009, 63:1-4), that may also be considered as or a comprehensive timely review on the subject of beta-lactam resistance in gram negative bacteria. The name ESBLs will be further used in the text to indicate gram-negative bacteria harbouring beta-lactamases capable of breaking down a wide range of beta-lactam antibiotics. The name ESBL will also be used to indicate an extended spectrum beta-lactamase enzyme. Thus ESBLs may confer resistance to these antibiotics and related oxyimino-beta lactams. Typically, ESBLs derive from genes for TEM-1, TEM-2, or SHV-1 by mutations that alter the amino acid configuration around the active site of these β-lactamases. This extends the spectrum of β-lactam antibiotics susceptible to hydrolysis by these enzymes.

ESBLs according to the invention include the TEM family, SHV family as well as others, and CTX-M family, which are class A enzymes. The ESBLs of the present invention include, but are not limited to TEM beta-lactamases (class A), SHV beta-lactamases (class A), CTX-M beta-lactamases (class A), OXA beta-lactamases (class D), AmpC-type β-lactamases (Class C), Carbapenemases, IMP-type carbapenemases (metallo-beta-lactamases), VIM (Verona integron-encoded metallo-beta-lactamase), NDM-type carbapenemases (New Delhi Metallo-beta-lactamases), OXA (oxacillinase) group of β-lactamases (Class D), and KPC (K. pneumoniae carbapenemase) (Class A). Hence, the ESBL genes can be grouped, such as, according to class, according to gene family, according to resistance to specific antibiotics, or according to mutation. The present invention relates in particular to carbapenemases, preferably chosen from the group consisting of KPC, NDM, VIM, OXA-48 and IMP. The present invention relates in particular to the CTX-M1 family, in particular CTX-M15 in E.coli strain ST-131, which is endemic in Europe. The present invention further relates in particular to the CTX-M9 family, including CTX-M9 en CTX-M14, the SHV-family, the TEM-family and the CTX-M2 family. The present invention also relates to AmpC beta-lactamases.

ESBLs are frequently plasmid encoded. Plasmids responsible for ESBL production frequently carry genes encoding resistance to other drug classes (for example, aminoglycosides). In this regard it will be appreciated that different ESBL genes may have sequences in common.

ESBL nucleic acids according to the invention are nucleic acids derived from genes encoding ESBLs. ESBL nucleic acids may characterize part of a coding region for an ESBL. The ESBL nucleic acids may be located on chromosomes, extra-chromosomally, on plasmids, on transposons, etc. The target ESBL nucleic acid is the ESBL nucleic acid to be detected, i.e. of which the presence is to be determined. ESBL nucleic acids encompass DNA, mRNA, and total RNA.

The term "nucleic acid" as used herein means a polymer composed of nucleotides, e.g. deoxyribonucleotides or ribonucleotides. The terms "ribonucleic acid" and "RNA" as used herein means a polymer composed of ribonucleotides. The terms "deoxyribonucleic acid" and "DNA" as used herein means a polymer composed of deoxyribonucleotides. The terms "oligonucleotide", "primer" and "probe" as used herein denotes single stranded nucleotide multimers of from about 10 to about 250 nucleotides in length. The term "polynucleotide" as used herein refers to single or double stranded polymer composed of nucleotide monomers of from about 10 to about 250 nucleotides in length, usually of greater than about 250 nucleotides in length up to about 2000 nucleotides in length.

TEM-1 is the most commonly-encountered beta-lactamase in gram-negative bacteria. Up to 90% of ampicillin resistance in E. coli is due to the production of TEM-1. Also responsible for the ampicillin and penicillin resistance that is seen in *H. influenzae* and *N. gonorrhoeae* in increasing numbers. Although TEM-type beta-lactamases are most often found in *E. coli* and *K*. *pneumoniae,* they are also found in other species of gram-negative bacteria with increasing frequency. The amino acid substitutions responsible for the ESBL phenotype cluster around the active site of the enzyme and change its configuration, allowing access to oxyimino-beta-lactam substrates. Opening the active site to beta-lactam substrates also typically enhances the susceptibility of the enzyme to beta-lactamase inhibitors, such as clavulanic acid. Single amino acid substitutions at positions 104, 164, 238, and 240 produce the ESBL phenotype, but ESBLs with the broadest spectrum usually have more than a single amino acid substitution. Based upon different combinations of changes, currently 140 TEM-type enzymes have been described. SHV-1 shares 68 percent of its amino acids with TEM-1 and has a similar overall structure. The SHV-1 beta-lactamase is most commonly found in *K*. *pneumoniae* and is responsible for up to 20% of the plasmid-mediated ampicillin resistance in this species. ESBLs in this family also have amino acid changes around the active site, most commonly at positions 238 and/or 240, less frequently at 179. More than 60 SHV varieties are known. They are the predominant ESBL type in the United States and are found worldwide. SHV-5 and SHV-12 are among the most common.

CTX-M beta-lactamases (class A) were named for their greater activity against cefotaxime than other oxyimino-beta-lactam substrates (eg, ceftazidime, ceftriaxone, or cefepime). Rather than arising by mutation, they represent examples of plasmid acquisition of beta-lactamase genes normally found on the chromosome of *Kluyvera* species, a group of rarely pathogenic commensal organisms. These enzymes are not very closely related to TEM or SHV beta-lactamases in that they show only approximately 40% identity with these two commonly isolated beta-lactamases. More than 40 CTX-M enzymes are currently known. Despite their name, a few are more active on ceftazidime than cefotaxime. Presently, they are mainly been found in strains of *E. coli,* but have also been described in other species of Enterobacteriaceae. They are the predominant ESBL type in Europe and parts of South America.

OXA beta-lactamases were long recognized as a less common but also plasmid-mediated beta-lactamase variety that could hydrolyze oxacillin and related anti-staphylococcal penicillins. These beta-lactamases differ from the TEM and SHV enzymes in that they belong to molecular class D and functional group 2d. The OXA-type beta-lactamases confer resistance to ampicillin and cephalothin and are characterized by their high hydrolytic activity against oxacillin and cloxacillin and the fact that they are poorly inhibited by clavulanic acid. Amino acid substitutions in OXA enzymes can also give the ESBL phenotype. The OXA beta-lactamase family was originally created as a phenotypic rather than a genotypic group for a few beta-lactamases that had a specific hydrolysis profile. Therefore, there is as little as 20% sequence homology among some of the members of this family. However, recent additions to this family show some degree of homology to one or more of the existing members of the OXA beta-lactamase family.

AmpC type β-lactamases are commonly isolated from extended-spectrum cephalosporin-resistant Gram-negative bacteria. AmpC β-lactamases (also termed class C or group 1) are typically encoded on the chromosome of many Gram-negative bacteria including *Citrobacter, Serratia* and *Enterobacter* species where its expression is usually inducible; it may also occur on *Escherichia coli* but is not usually inducible, although it can be hyperexpressed. AmpC type β-lactamases may also be carried on plasmids. AmpC β-lactamases, in contrast to ESBLs, hydrolyse broad and extended-spectrum cephalosporins (cephamycins as well as to oxyimino-β-lactams) but are not inhibited by β-lactamase inhibitors such as clavulanic acid.

Carbapenems are famously stable to AmpC β-lactamases and extended-spectrum-β-lactamases. Carbapenemases are a diverse group of beta-lactamases that are active not only against the oxyimino-cephalosporins and cephamycins but also against the carbapenems. Aztreonam is stable to the metallo-β-lactamases but many IMP and VIM producers are resistant, owing to other mechanisms. Carbapenemases were formerly believed to derive only from classes A, B, and D, but a class C carbapenemase has been described.

NDM-type carbapenemases (New Delhi Metallo-b-lactamases) are plasmid mediated carbapenemases, which were only recently discovered in India, and presently is spreading to Europe, particularly the United Kingdom.

The OXA group of β-lactamases (Class D) mainly occur in Acinetobacter species and are divided into two clusters. Recently, however, a specific variant, OXA-48, have also been found in E.coli and K.pneumoniae, and seems to be spreading rapidly. OXA carbapenemases hydrolyse carbapenems very slowly *in vitro,* and the high MICs seen for some Acinetobacter hosts (>64 mg/L) may reflect secondary mechanisms. They are sometimes augmented in clinical isolates by additional resistance mechanisms, such as impermeability or efflux. OXA carbapenemases also tend to have a reduced hydrolytic efficiency towards penicillins and cephalosporins.

The method of the present invention can detect specifically the phenotypes of the TEM variants and the corresponding amino acid substitutions as depicted in Table 1 a. The method of the present invention can detect specifically the phenotypes of the SHV variants and the corresponding amino acid substitutions as depicted in Table 1 b.

The present invention relates particularly to the detection of ESBL associated substitutions as depicted in Table 2 and supplementary Table 2.

ESBL micro-organisms according to the invention are gram-negative and/or gram-positive bacteria carrying one or more ESBL genes. Examples of gram-negative bacteria that frequently carry ESBL include (sub)species of *Escherichia, Klebsiella, Enterobacter, Salmonella, Shigella, Citrobacter, Hafnia, Serratia, Morganella, Proteus, Providencia, Pseudomonas, Acinetobacter and Kluyvera.*

The present invention relates to a method as described herein, wherein said micro-organism is selected from the group consisting of human and/or animal parasitic, symbiotic, commensals and/or pathogenic micro-organisms. The present invention relates to a method as described herein, wherein said micro-organism is selected from the group of bacteria and (sub)species thereof consisting of *Escherichia, Klebsiella, Enterobacter, Salmonella, Shigella, Citrobacter, Hafnia, Serratia, Morganella, Proteus, Providencia, Pseudomonas, Acinetobacter and Kluyvera.* The present invention relates to a method as described herein, wherein said micro-organism is selected from the group consisting of E.coli, Klebsiella pneumoniae, Klebsiella oxytoca, Enterobacter species, Salmonella species, Shigella species, Citrobacter freundii, Hafnia alvei, Serratia species, Morganella morganii, Proteus mirabilis, Providencia species, Pseudomonas aeruginosa, Acinetobacter baumanni and N. gonorrhoeae.

The present invention relates also to a method as described herein, wherein said micro-organism is selected from the group consisting of *Averyella, Pantoea, Photorhabdus, Pleosimonas, Raoultella, Edwardsiella, Ewingella, Cedecea, Leclercia, Leminorella, Moellerella, Rahnella, Tatumella, Yokenella, Yersinia, Nocardia, Rhodococcus, Gordonia, Actinomadura, Streptomyces, Mycobacterium, Propionibacterium, Actinomyces, Lactobacillus, Eurobacterium, Eggerthella, Olsenella, Bifidobacterium, Mobiluncus, Alistipes, Bacteroides, Cetobacterium, Desulfovibrio, Dialister, Faecalibacterium, Fusobacterium, Porphyromonas, Prevotella, Sneathia, Tannerella Lactococcus, Listeria, Erysipelothrix, Leuconostoc, Bacillus, Staphylococcus, Clostridium, Vibrio, Enterococcus, Legionella, Campylobacter, Arcobacter, Helicobacter, Leptospira, Borrelia, Treponema, Mycoplasma, Ureoplasma, Chlamydia, Chlamydophila, Rickettsia, Orientia, Ehrlichia, Anaplasma, Neorickettsia, Aegyptianella, Coxiella, Tropheryma, Streptococcus, Micrococcus, Flavobacterium, Alcaligenes, Microbacterium, Neisseria, Actinobacillus, Capnocytophaga, Eikenella, Kingella, Pasteurella, Haemophilus, Aeromonas, Burkholderia, Stenotropomonas, Ralstonia, Cupriavidus, Pandoraea, Brevundimonas, Comamonas, Delftia, Acidovorax, Achromobacter, Chryseobacterium, Moraxella, Bordetella, Psychrobacter, Oligella, Haematobacter, Alcaligenes, Advenella, Alishewanella, Aquaspirillum, Laribacter, Myroides, Shewanella, Ochrobactrum, Rhizobium, Halomonas, Herbaspirillum, Inquilinus, Massilia, Sphingobacterium, Pedobacter, Para coccus, Asaia, Methylobacterium, Roseomonas, Azospirillum, Elizabethkingia, Empedobacter, Weeksella, Bergeyella, Balneatrix, Bordetella, Francisella, Brucella, Bartonella, Peptostreptococcus, Finegoldia, Anaerococcus, Peptoniphilus, Veillonella, Gallicola, Sackia, Atopobium, Ruminococcus, Aerococcus, Abiotrophia,*

In a particular preferred embodiment, the present invention relates to a method as described herein, wherein said micro-organism is *E.coli, K.pneumoniae* and/or *Enterobacter,* and even more preferably, wherein said ESBL is a carbapenemase.

In general, a sample or specimen will be taken as a part of anything. For clinical applications the sample or specimen can be any kind of bodily solid, semi-solid or fluid substance such as, but not limited to faeces, blood, blood plasma, serum, urine, bodily liquid, rectal swabs, nasal swabs, sputum, licor, infected tissue, etc, but also from the environment.

In one aspect of the invention, the present method is applicable to the micro-organisms which are known to cause (opportunistic) infections and maladies.

As such, the present invention relates to a method for determining the presence of ESBL micro-organisms in a sample, comprising the steps of optionally collecting said micro-organisms if present, extracting nucleic acids from said micro-organisms, specifically amplifying said nucleic acids thereby detecting the amplified target nucleic acids, whereby the presence of said micro-organisms is determined. For samples where the presence of said ESBL micro-organisms is detected, the amplified nucleic acids may be further analyzed, thereby identifying and characterizing specifically the ESBL nucleic acid. As a consequence, only the positive samples are subjected to a further analysis which constitutes in a time, work and/or material saving method.

In a particular embodiment of the present invention the micro-organisms are captured or collected prior to extracting the nucleic acids from micro-organisms (step (a)) of the method of the present invention. The capturing or collection of the micro-organisms prior to step (a) of the method provides a concentration step which allow a concentration of the micro-organisms prior to the method of the invention, thereby providing an even more accurate method.

In order to increase the amount of micro-organisms present in a sample, said micro-organisms, if present, may be grown on media. Accordingly, the present invention relates to a method as described herein, wherein said method, for instance step (a) of above, is preceded by an enrichment of micro-organisms, comprising (i) growth of said micro-organisms on selective media, or (ii) growth of said micro-organisms on non-selective media. Growth of said micro-organisms on selective media will preferably favour the growth of micro-organisms of interest, while the growth on non-selective media will sustain growth of most micro-organisms, e.g. not especially favouring the growth of a particular micro-organism.

Although the sample can be used directly for nucleic acid isolation, some techniques require the growth and collection of the micro-organisms prior to the nucleic acid isolation. According to the method of the invention the growth and collection of the micro-organisms prior to the nucleic acid isolation is neither required nor essential, thereby providing a faster detection method compared to the prior art methods. The growth and collection of the micro-organisms prior to the nucleic acid-isolation may however be optionally included in the method of the invention. Accordingly, the present invention relates to a method as described herein, wherein said method, for instance step (a) of above, is preceded by an enrichment of micro-organisms, comprising concentrating the micro-organisms. Typical collection strategies known in the art are for instance, but not limited to, plating out the sample on a suitable solid culture medium, adding the sample in a suitable liquid culture medium or first providing the sample in a suitable liquid culture medium followed by plating it out on a suitable solid culture medium. From a solid culture medium, micro-organisms can be directly collected for DNA-isolation, while a liquid culture medium in general requires first a centrifugation step to collect the micro-organisms. The collection and/or capturing of said micro-organisms may be performed by means of centrifugation, filtration, such as filtering of an aqueous or liquid solution, whereby all particles larger than the sieving size are being captured, sedimentation, electrostatic forces, coagulation, flocculation, capturing of micro-organisms by antibodies, and/or capturing of micro-organisms by ligands.

In order to characterise the ESBL nucleic acid from a micro-organism, *i.e.* the target ESBL nucleic acid, said ESBL nucleic acid is normally isolated from the micro-organism after said organism has optionally been collected or captured. The collection or capturing of the contaminating organism and the isolation, e.g. extracting, of the nucleic acids are performed using generally known techniques. The present invention relates to a method as described herein, wherein said extracting nucleic acids employs a treatment with a lysozyme, a pectinolytic, or guanidinium thiocyanate or by a mechanical treatment such as sonication or the use of a bead beater, by injecting the micro-organisms in hot phenol, and snap freezing the micro-organisms in liquid nitrogen followed by a mechanical treatment. A convenient method for isolating RNA from Gram negative organisms is to resuspend the cells in water and boil the water for at least one minute. Optionally EDTA and/or a detergent can be added to the water. The techniques are common in the art and described in e.g. Selinger et al. (2000, Nature Biotechnol. 18, 1262-1268), Current Protocols in Molecular Biology, Wiley & Co, USA, Boom et al. (1999; J. Clin. Microbiol. 37: 615-619), Ye et al. (2001, J.Microbiol. Methods 47, 257-272). A variety of RNA isolation kits are available from different commercial sources, e.g. from Ambion, Qiagen, Sigma-Aldrich and others, the use of the RNAlater® solution (Ambion and Qiagen) may all successfully be used in the method of the present invention. A variety of genomic DNA isolation kits are available from different commercial sources, e.g. from Gentra, Promega, Qiagen and others, all of which may successfully be used in the methods of the present invention.

A convenient way to estimate the concentration of the isolated nucleic acid is by spectrophotometry at 260 nm, which is well known in the art.

After nucleic acids have been extracted or isolated from the micro-organisms, said nucleic acids need to be detected and possibly analysed. In general, only minute amounts of contaminating micro-organisms are present. Therefore, the isolated ESBL nucleic acids or a specific portion thereof, *i.e.* the target ESBL nucleic acid, may be amplified. In case of the target ESBL nucleic acid being RNA, said RNA may first be converted to cDNA before analysis. Therefore, the present invention relates to a method as described herein, wherein said nucleic acid is mRNA or total RNA, wherein said mRNA or total RNA is converted to cDNA, e.g. by the activity of a reverse transcriptase, as is well known in the art.

It will be understood that the terms "amplified target ESBL nucleic acids", "ESBL amplicon" and "amplified nucleic acid mixture" as used throughout the invention have essentially the same meaning. It will be understood that the term "ESBL amplicon" encompasses RNA transcripts generated via RNA polymerase activity.

In order to determine the presence of ESBL nucleic acids, the present invention may employ known techniques identifying the ESBL nucleic acid of the micro-organism at issue. The present invention relates preferably to multiplexed amplification and labelling technique described below. Multiplexing provides the opportunity to perform multiple analyses during a single process step providing faster analysis times and lower amounts of consumables to be used.

An embodiment of the invention relates to Ligase Detection Reaction (LDR). The Ligase Detection Reaction (LDR) is a sensitive assay for detecting, among others Single Nucleotide Polymorphisms (SNPs), as described by Favis et al., (2000, Nature Biotechnology 18: 561-564). Single nucleotide differences along the drug resistance conferring genes, such as ESBL genes may be employed to distinguish between sequences of different drug resistance conferring genes, such as different ESBL nucleic acids. Similarly, any nucleotide difference between two ESBL nucleic acids in any type of DNA or RNA, such as chromosomal DNA, plasmid DNA, or any other organelle DNA, mRNA, total RNA or any other RNA molecule such as described *infra,* may be employed to determine the specific ESBL nucleic acid.

In step (b1) of the method according to the present invention the ESBL nucleic acid and/or ESBL cDNA may be detected using the Ligase Detection Reaction. In the LDR according to the invention, a plurality of different probe pairs is contacted with a target ESBL nucleic acid.

Each probe pair comprises a (identifiable) first nucleic acid probe and a (identifiable) second nucleic acid probe. The probes may be distinguished individually by their specific sequence, e.g. a sequence complementary to a particular target ESBL nucleic acid sequence, or by a tag, e.g. a tag-sequence, including a ZIP, chip or DET sequence. Preferably, the (identifiable) first nucleic acid probe is complementary to a distinct part of said target ESBL nucleic acid and the (identifiable) second nucleic acid probe is complementary to an essentially adjacent located second part of said target ESBL nucleic acid. Essentially adjacent in this context means 0 (no intervening), 1 or 2 nucleotides apart, based on the target ESBL nucleic acid. Optionally, the first and/or second nucleic acid probe is identifiable. The term "identifiable" in this context connotes probes which are individually distinguishable from other probes.

The term "plurality" in plurality of different probe pairs relates to more than one probe pair, preferably, 2, 3, 4, 5, 6, 7, 8, 9, 10, 11, 12, 13, 14, 15, 16, 17, 18, 19, 20, or even more, such as, 25, 30, 32, 35, 40, 45, 50, 60, 70, 80, 90, 100 or still even more different probe pairs. As demonstrated in the examples, the method according to the invention is particularly suited for the rapid detection of a wide variety of ESBL nucleic acids in a single test by using the plurality of different probe pairs. Because of the versatility of the present method, newly discovered ESBL can easily be incorporated in the test by adding or adjusting the probe pairs. Since the "probe pairs" are made of a (identifiable) first nucleic acid probe and a (identifiable) second nucleic acid probe, the person skilled in the art will understand that the term "different probe pairs" relates to probe pairs in which either the (identifiable) first nucleic acid probe, either the (identifiable) second nucleic acid probe, or both the (identifiable) first nucleic acid probe and the (identifiable) second nucleic acid probe, differ, e.g. have a different (complementary) target ESBL nucleic acid sequence, from another probe pair. Hence, each probe pair may be specific for a particular target ESBL nucleic acid.

A set or pair of two probes (a (identifiable) first nucleic acid probe and a (identifiable) second nucleic acid probe or probe I and II, respectively) may be designed, based on the target ESBL nucleic acid sequence to be detected, of which at least a part is known. Both probes contain a region at the end (the 3' and the 5' end of the respective probes I and II) that is capable of hybridizing to the known section of the target ESBL nucleic acid sequence.

Preferred sets of probe pairs are provided in supplementary Table 1. Hence, the present invention relates particularly to at least one probe pair of Table 1, or more than 1 probe pair, such as, 2, 3, 4, 5, 6, 7, 8, 9, 10, 11, 12, 13, 14, 15, 16, 17, 18, 19, 20 or even more probe pairs. Also, the present invention relates to at least 1, such as, 2, 3, 4, 5, 6, 7, 8, 9, 10, 11, 12, 13, 14, 15, 16, 17, 18, 19, 20 or even more (identifiable) first nucleic acid probes and/or (identifiable) second nucleic acid probe making up the probe pairs of supplementary Table 1

In other words, one probe (probe I) comprises a region Target-Specific Sequence I or TSS(I) (specifically hybridising to a target ESBL region, said region TSS(I) being located at the ultimate 3' end of probe I. Said probe I may further comprise a primer binding section PBS(I), located 5' from the region TSS(I). Said probe I may contain a stuffer region, a ZIP region (ZIP or cZIP) and/or a particular DET region. For instance, said stuffer region and/or a ZIP on probe I may be located between region TSS(I) and PBS(I). Said probe I may further or alternatively comprise an RNA polymerase promoter region, such as eg a T7 promoter region (T7-p), located at the ultimate 5' end of probe I and adjacent to PBS(I), such as for instance depicted in Figures 3a and 3b.

The probe II comprises a region TSS(II) specifically hybridising to a target region, said region TSS(II) being located at the ultimate 5' end of probe II. Said probe II further comprising a primer binding section PBS(II) located 3' from the region TSS(II). Probe II may further comprise a ZIP region (ZIP or cZIP) and/or a DET region, located in-between the region TSS(II) and PBS(II). Said probe II may further or alternatively comprise an RNA polymerase promoter region, such as eg a T7 promoter region (cT7-p) located at the ultimate 3' end of probe II and adjacent to PBS(II), such as for instance depicted in Figure 3c.

It will be appreciated that the TSS of the probes may be of any length provided a specific hybridisation is possible with the target ESBL nucleic acid sequence under the conditions of the method according to the invention, such as, 5, 6, 7, 8, 9, 10, 11, 12, 13, 14, 15, 16, 17, 18, 19, 20, 21, 22, 23, 24, 25, 26, 27, 28, 29 or 30 nucleotides, or even more nucleotides, such as for instance 35, 40, 45, or 50. Preferred sequences are depicted in supplementary Table 1.

With appreciation of the present invention, TSS can be determined with standard methods. Preferably, a TSS is located in the target ESBL sequences provided in Table 4. The sequences of Table 4 were exceptionally suited for detecting a broad range of ESBL nucleic acids, with any suitable method. Hence, the present invention relates preferably to TSS chosen from at least one but preferably more than one, such as, 2, 3, 4, 5, 6, 7, 8, 9, 10, 11, 12, 13, 14, 15, 16, 17, 18, 19, 20, 21, 22, 23, 24, 25, 26, 27 or 28 sequences provided in Table 4. Even more preferably, the present invention relates to sequences of TEM wild type 104, TEM wild type 164, TEM wild type 238, TEM wild type 238/240, OXA 48 I, OXA 48 II, VIM 552C, VIM 246, IMP 114, IMP 480 I/II, SHV all, NDM-1 75, NDM-1 392, CMY II 304, CMY II 1199, CMY I MOX, ACC 606, MIR ACT, DHA 753, DHA 1110, TEM all, FOX, KPC I, KPC II, CTX-M8 + 25, CTX-M9 + 64, CTX-M2, and CTX-M1 of Table 4, even more preferably TEM-E104K, TEM-R164S, TEM-R164C, TEM-R164H, TEM-G238S, SHV-G238S, SHV-G238A, SHV-E240K, SHV-D179A, SHV-D179G, and SHV-D179N. In a further preferred embodiment the present invention relates to TSS located in the target ESBL sequences provided in Table 4 of (i) TEM wild type 104, TEM wild type 164, TEM wild type 238, TEM wild type 238/240 (ii) OXA 48 I, OXA 48 II; (iii) VIM 552C, VIM 246; (iv) IMP 114, IMP 480 I/II; (v) SHV all; (vi) NDM-1 75, NDM-1 392; (vii) CMY II 304, CMY II 1199, CMY I MOX; (viii) ACC 606; (ix) MIR ACT; (x) DHA 753, DHA 1110; (xi) TEM all; (xii) FOX; (xiii) KPC I, KPC II; and/or (xiv) CTX-M8 + 25, CTX-M9 + 64, CTX-M2, and CTX-M1.

ZIP and cZIP refer to the DNA segments used for detection to capture probes, where cZIP has the complementary sequence of ZIP. The cZIP or its complement the ZIP is a unique sequence for identification of the eventually amplified products. cZIP will hybridize to its complement ZIP. The complement of the amplified product is present on for instance a capture probe in solution or on a solid support (capture probe; see *infra*). Upon hybridisation, the target region TSS(I) of probe I is located adjacent to the target region TSS(II) of probe II. cZIP, ZIP, DET and the PBSs are preferably not capable of hybridizing to the target sequence.

T7-p refers to the T7 promoter sequence, whereas cT7-p has the complementary sequence of T7-p.

An RNA polymerase is an enzyme that produces RNA from DNA as template in a process termed transcription. The RNA strand is complementary to the DNA template. RNA polymerases start transcription at a specific DNA sequence known as a RNA polymerase promoter. Preferred RNA polymerases are T7, SP-6, T3 and T4 RNA polymerase.

The method described herein relates to the simultaneous detection of various ESBL micro-organisms, by providing at least one set, and preferably more than one set of two probes, specifically designed to identify and/or characterise the presence of an ESBL nucleic acid, and hence ESBL micro-organism (multiplex). The different sets of probes should preferably not cross-hybridise, while on the other hand the melting temperature Tm of the different sets of probe/primers is about similar, e.g. all between 65°C and 75°C. Commonly available computer programmes, such as Probe Match, Michigan State University, East Lansing, Michigan USA, Oligo 5.0 software (PE Biosystems, Foster City, California, USA), and using Clustal W Algorithm, may facilitate the design of specific probes. Preferably, the primers/probes have a melting temperature Tm between about 37-85 °C, or 50-80 °C, or 55-75 °C, or 65-75 °C. As such, the present invention relates also to multiplex amplification.

Accordingly, the present invention relates to a method as described herein, wherein a set of two adjacent probes is provided for the ESBL nucleic acids as defined supra. Also, these probes may be coupled.

In step (b2) of the method according to the invention, the target ESBL nucleic acid is incubated with a plurality of different probe pairs under conditions allowing to hybridize said particular target ESBL nucleic acid with at least one probe pair. Hybridising specifically takes the length, G/C content and hybridisation conditions, such as salt and temperature, into account as known by the person skilled in the art. Hybridizing conditions are well known in the art, or may be determined without difficulty by the person skilled in the art, see e.g. "Molecular Cloning: A Laboratory Manual" Third Edition (Sambrook et al., 2000) and "Current Protocols in Molecular Biology" (F. M. Ausubel et al., eds., 1987, and periodic updates). General guidance is given in the Examples section. The term "hybridising specifically" relates to a perfect match between a receptor (e.g. target ESBL nucleic acid) and its ligand (e.g. identifiable first or identifiable second nucleic acid probe).

In step (b3) of the method according to the invention, the identifiable first nucleic acid probe is connected with any essentially adjacent located identifiable second nucleic acid probe of a probe pair, to form a connected probe assembly. As described supra, the first and second probe of a probe pair hybridised to an ESBL target nucleic acid, may be located 0, 1 or 2 nucleotides apart. It has been found that a "gap" of 1 or 2 nucleotides can provide for a further means for discrimination. In this case, the complementary nucleotide(s) of the gap in the presence of a polymerase activity are added to the reaction mixture. These complementary nucleotide(s) may be labeled. In the presence of a perfectly matching template, the probes may be connected, e.g. ligated by the action of a ligase. Thus, the present invention relates to a method as described herein, wherein said ligation step comprises the use or activity of a ligase, such as T4 DNA ligase, T4 RNA ligase, *E.coli* DNA ligase, or a thermostable ligase such as *Taq* DNA ligase, *Pfu* DNA ligase, *Tth* DNA ligase or Ampligase™. Conditions under which a ligation reaction may occur are well known in the art. In step (b4) of the method according to the invention, the at least a set of two amplification primers is provided, wherein at least one primer of said set of two amplification primers is preferably labelled when performing a hybridisation reaction with a capture probe. The set of two amplification primers comprises at least a forward and a reverse primer as is well known in the art. The forward primer is complementary to and can bind, e.g. can hybridise, to the respective primer binding site of a nucleic acid probe of a probe pair, while the reverse primer comprises a sequence substantially identical to the primer binding site of the other nucleic acid probe of a probe pair. Preferably, the reverse primer is labelled. In this case, only amplified target ESBL nucleic acids [ESBL amplicon] are labelled.

The amplification primers may comprise a promoter for a reverse transcriptase at their ultimate 5' ends.

Only one set of two amplification primers is necessary to amplify all the connected probe assemblies, in case all of the identifiable first nucleic acid probes of each probe pair of the plurality of different probe pairs comprises the same identical primer binding site (PBS I) on the one hand and all of the identifiable second nucleic acid probes of each probe pair of the plurality of different probe pairs comprises the same identical primer binding site (PBS II) on the other hand. This reduces costs and simplifies the amplification reaction.

The person skilled in the art will understand that variations on the above are possible. For instance, all reverse primers or all forward primers can be the same (the constant primer), when either all of the identifiable first nucleic acid probes or all of the identifiable second nucleic acid probes of the plurality of different probe pairs comprises the same identical primer binding site. In this case it is preferred to have the constant primer labelled. This reduces costs as well. For instance, the nucleic acid probes can be grouped, in which the identifiable first nucleic acid probes of a group comprise the same identical primer binding site (PBS I). In addition, or in the alternative, the identifiable second nucleic acid probes of a group comprise the same identical primer binding site (PBS II). In this case it is preferred to have the constant primer of a group specifically labelled, i.e. a label which is specific for a group, and which can be used to differentiate from other groups. This facilitates identification of groups of ESBL nucleic acids. For instance, each identifiable first nucleic acid probe and each identifiable second nucleic acid probe comprises a unique primer binding site, i.e. a primer binding site not shared by any other probe. In this case, differentiation between ESBL nucleic acids can be even further enhanced. Further, for instance, a combination is possible of any of the above.

In step (b5) of the method according to the invention, the connected probe assembly of step (b3) is amplified using the amplification primers of step (b4), thereby providing amplified target ESBL nucleic acids [ESBL amplicon].

It will be appreciated that the methods of WO 2004/106547 and PCT/EP2009/064292, both in name of the present applicant, are particularly well-suited in the method according to the present invention.

Various techniques are known by the person skilled in the art to amplify nucleic acids, such as DNA and/or cDNA. All of these techniques are contemplated by the present invention. Accordingly, the present invention relates to a method as described herein, wherein said nucleic acid is DNA and/or cDNA, and wherein said DNA and/or cDNA is amplified using an amplification technique, such as, bDNA, Hybrid capture, SDA, TMA, PCR, LCR, TAS, 3SR, NASBA and Qβ amplification, as explained in Versalovic and Lupski (2002, Trends Microbiology 10: S15-S21). Further, a probe or primer may contain an RNA polymerase binding site. The ligated probes are subsequently amplified by the activity of an RNA polymerase, e.g. T4-, T7- or SP6 RNA polymerase.

The present invention especially contemplates multiplex amplification, such as multiplex PCR. Multiplex amplification, such as multiplex PCR, allows amplification, and thus analysis of two or more targets simultaneously. By routine experimentation the person skilled in the art will be able to optimize the reaction conditions, in view of having multiple primer pairs in a single reaction, which may increase the likelihood of primer-dimers and other nonspecific products that may interfere with the amplification of specific products. In addition, the concentrations of individual primer pairs often need to be optimized since different multiplex amplicons are often amplified with differing efficiencies, and multiple primer pairs can compete with each other in the reaction. The person skilled in the art will make similar considerations and optimize the conditions for the other amplification techniques described above for multiplex amplifications, *i.e.* amplification of more than one target.

In addition, the present invention relates to the direct amplification of RNA, such as, for example, via a modified Tyras method, wherein a primer/probe comprising a RNA polymerase recognition site and recognition site complementary to the target nucleic acid is used. Further preferred RNA amplification techniques of the present invention are nucleic acid sequence-based amplification (NASBA) and transcription mediated amplification (TMA).

The NASBA technology was developed by Compton in 1991 (Compton 1991, Nature 350: 91-92), who defined it as "a primer-dependent technology that can be used for the continuous amplification of nucleic acids in a single mixture at one temperature". The NASBA technology is preferably performed as described in EP0629706. A preferred embodiment of NASBA is depicted in Figure 4.

The TMA technology is performed as described by Gonzales and McDonough (Applications of Transcription-Mediated Amplification to Quantification of Gene Sequences. Gene Amplification. 1998 Ed. François Ferre, Birkhauser, Boston. PP. 189-204) and in US5399491. It uses two primers and two enzyme activities: RNA polymerase activity and reverse transcriptase activity. One primer contains a promoter sequence for RNA polymerase. In the first step of amplification, this primer hybridizes to the target RNA. Reverse transcriptase activity creates a DNA copy of the target RNA by extension from the 3' end of the promoter primer. The RNA in the resulting RNA:DNA duplex is degraded by the RNase activity of the reverse transcriptase. Next, a second primer binds to the DNA copy. A new strand of DNA is synthesized from the end of this primer by reverse transcriptase activity, creating a double-stranded DNA (dsDNA) molecule. RNA polymerase recognizes the promoter sequence in the DNA template and initiates transcription. Each of the newly synthesized RNA amplicons reenters the TMA process and serves as a template for a new round of replication.

NASBA and TMA have several differences in comparison to PCR: (i) they are isothermal, which implicates that a water bath or heat block can be used instead of a thermal cycler (ii) a more labile RNA amplicon is produced instead of a DNA amplicon, which helps reduce the possibility of carry-over contamination, (iii) more copies per cycle are produced.

The present invention also relates to LDR-NASBA and LDR-TMA techniques, wherein the connected probe assembly that is generated in step (b3) is converted into RNA before its amplified using NASBA or TMA. The conversion step involves (1) the synthesis of a complementary DNA strand of the single-stranded connected probe assembly using a reverse transcriptase activity, such as, for example AMV or MMLV reverse transcriptase, and resulting in double-stranded DNA, followed by (2) the generation of a RNA transcript using the double-stranded DNA as template and RNA polymerase activity, e.g. via T7 RNA polymerase. Accordingly, the present invention intends a probe/primer comprising a RNA polymerase recognition site .

The present invention further relates to the incorporation of dUTP and incubation of the PCR reaction with uracil-DNA-glycosylase prior to thermal cycling to prevent cross-contamination from previous amplifications.

When both probes are hybridized to the target sequence, and are located adjacent to each other, the probes can be ligated using a ligase, such as for example *Pfu* DNA ligase. After ligation, the ligated probes may be amplified using at least one primer that is capable of hybridizing to a primer binding section. Preferably, amplification is carried out by PCR, using probe I with a PBS(I) which differ from probe II with PBS(II). Hence, primer I binding to the region characterized by PBS(I) will differ from primer II binding to the region characterized by PBS(II). It will be appreciated that if primer I comprises a sequence substantially complementary to PBS(I), then primer II comprises a sequence substantially identical to PBS(II), and vice versa, that if primer I comprises a sequence substantially identical to PBS(I), then primer II comprises a sequence substantially complementary to PBS(II).

In step (c) of the method of the invention, the amplified target ESBL nucleic acids [ESBL amplicon] are detected by hybridization to capture probes during and/or after the amplification reaction. Detection of amplicons during amplification after each amplification cycle may be accomplished by e.g. Real Time (RT) PCR. Detection after amplification may be accomplished by hybridization to a capture probe bound to a solid support. In both cases the presence of an ESBL nucleic acid in a sample is determined.

In a further embodiment, the identifiable first nucleic acid probe (Probe I) or the identifiable second nucleic acid probe (Probe II) may comprise a ZIP. Since it is the object of the present invention that upon ligation of Probe I and Probe II, the ligated probe (connected probe assembly) is amplified, it can be understood that the amplified ligated probe (ESBL amplicon) should contain a cZIP for it to hybridise with a ZIP, present on a capture probe, for instance, on a microchip. Therefore providing Probe I or Probe II with a ZIP would also result in an amplified ligated probe (ESBL amplicon) comprising a cZIP.

Capture probes can be immobilized on microarrays and/or may be present in solution during the amplification reaction. In the latter case such capture probes will typically be labeled with fluorescent dye and/or quenchers. The composition of the immobilized capture probes is not critical. The only requirement is that they be capable of hybridising to a target nucleic acid of complementary sequence, e.g. the amplified nucleic acid, if any. For example, the capture probes may be composed of all natural or all synthetic nucleotide bases, or a combination of both. Non-limiting examples of modified bases suitable for use with the instant invention are described, for example, in Practical Handbook of Biochemistry and Molecular Biology, G. Fasman, Ed., CRC Press, 1989, pp. 385-392. While in most instances the polynucleotides will be composed entirely of the natural bases (A, C, G, T or U), in certain circumstances the use of synthetic bases may be preferred, see for example Uhlman & Peyman, 1990, Chemical Review 90(4):544-584; Goodchild, 1990, Bioconjugate Chem. 1(3):165-186; Egholm et al., 1992, J. Am. Chem. Soc. 114:1895-1897; Gryaznov et al., J. Am. Chem. Soc. 116:3143-3144, as well as the references cited in all of the above. As mentioned above, the capture probes may be polymers of synthetic nucleotide analogs. Such capture probes may be utilised in certain embodiments because of their superior stability under assay conditions. Modifications in the native structure, including alterations in the backbone, sugars or heterocyclic bases, have been shown to increase stability and binding affinity. As such, the capture probes may include polymers of ribonucleotides and deoxyribonucleotides, with the ribonucleotide and/or deoxy-ribonucleotides being connected together via 5' to 3' linkages. The capture probes of the invention may be ribonucleic acids, for example sense or antisense ribonucleic acids, full-length or partial fragments of cRNA, full-length or partial fragments of mRNA, and/or ribo-oligonucleotides. Alternatively, capture probes of the invention may be deoxy-ribonucleic acids, preferably single-stranded full-length or fragments of sequences encoding the corresponding mRNAs. The form of the capture probes should be chosen so that they are complimentary to and form appropriate Watson-Crick hydrogen bonds with the amplified target nucleic acid and/or ligated probes in a sample.

The immobilized capture probes may be as few as four, or as many as hundreds, or even more, nucleotides in length. Contemplated as capture probes according to the invention are nucleic acids that are typically referred to in the art as oligonucleotides and also those referred to as nucleic acids. Thus, the arrays of the present invention are useful not only in applications where amplified target nucleic acids or ligated probes are hybridised to immobilized arrays of relatively short (such as, for example, having a length of approximately 6, 8, 10, 20, 40, 60, 80, or 100 nucleotides) capture probes, but also in applications where relatively short capture probes are hybridised to arrays of immobilized target nucleic acids. The capture probes of the array can be of any desired sequence.

In a further embodiment of the invention comprises a capture probe comprising the ZIP sequence which is essentially complementary to a corresponding cZIP. The capture probe comprising the ZIP sequence may be synthetically made and spotted on a solid support, e.g. a microarray or microbead, or synthesized on a specified location on the solid support. The ZIP sequence is a unique identifier sequence, which is complementary to the cZIP sequence. The present invention relates also to capture probes and the use thereof, comprising unique 20 to 30 base oligonucleotides, for instance 20, 21, 22, 23, 24, 25, 26, 27, 28, 29 or 30 base oligonucleotides, named ZIPs that are coupled to a porous three dimensional substrate at known locations, as described by van Beuningen et al., (2001, Clinical Chemistry 47: 1931-1933). These ZIPs hybridise specifically to molecules containing sequences that are complementary to the ZIPs, i.e. the cZIPs. By linking these cZIPs to fluorescent primers via a ligation-amplification reaction, ZIP microarrays may be used to detect and identify micro-organisms, such as for example microbial specimens. Because the ZIPs represent unique artificial sequences, microarrays comprising ZIPs can be used as a universal platform for molecular recognition simply by changing the gene specific sequences linked to the cZIPs. The detection of label on a specified location on the microarray, such as the Pamchip indicates the presence of a hybridisation product between the ligated product and the ZIP sequence on the microarray.

Accordingly, the present invention relates to a method as described herein, wherein said capture probe hybridises specifically to a corresponding cZIP. The amplified target nucleic acid or nucleic acids hybridised to a corresponding capture probe or probes on a microarray may result in a hybridisation pattern. The hybridisation pattern, including the intensity of hybridisation, may be characteristic for a given micro-organism.

The present invention also relates to a method as described herein, wherein said capture probe hybridises specifically to a corresponding cZIP coupled to a microbead, such as a Luminex microbead. The amplified target nucleic acid or nucleic acids hybridised to a corresponding capture probe or probes on microbeads may be sorted using a Fluorescence-Assisted Cell Sorting apparatus, like the Luminex Analyzer. Such microbeads each have a unique identity similar to specific positions on a microarray and may result in a hybridisation pattern. The hybridisation pattern, including the intensity of hybridisation, may be characteristic for a given target ESBL nucleic acid.

It will be apparent that the present invention relates to a method as described herein, wherein a signal is detected after hybridising the specifically amplified nucleic acids or the ligated probes to the capture probe. The said signal may be a fluorescent or phosphorescent signal, and said fluorescent or phosphorescent signal may be detected by a CCD camera or by laser scanning, such as for example a PAMstation or FD10 system® (Olympus). Alternatively, the present invention relates to a method as described herein, wherein said microarray is an Arraytube® and said fluorescent or phosphorescent signal may be detected by a CCD camera and/or laser scanning. Alternatively, the present invention relates to a method as described herein, wherein the hybridization signal is a colorimetrical signal using biotin-labelled primers detected by any method known in the art such as for instance conjugation with horseradish peroxidase-streptavidine followed by a peroxidase coloring reaction. The latter reaction may be visualized using an ArrayTube Reader (Clondiag GMBH, Jena, Germany).

The price of a microarray presents the larger cost per test. In order to decrease the price per test, the microarray can be interrogated simultaneously with more than one sample, as detailed in the examples section. As such, it is contemplated that each individual sample is subjected to the method of the present invention until the hybridisation step, i.e. from each individual sample, the micro-organisms are captured, after which the nucleic acids are extracted, which subsequently undergo a ligase detection reaction. Next, the amplified target nucleic acids if present are detected and the positive samples are collectively hybridized to the capture probes on a single microarray and the hybridized target nucleic acids are detected. The probes pair used per sample may be identical, e.g. detecting the same target nucleic acid, or may differ per sample, e.g. detecting different target nucleic acids. However, in order to differentiate between amplified target ESBL nucleic acids from different samples or between different amplified target ESBL nucleic acids derived from a single sample, each probe, and thus the amplified target ESBL nucleic acid, should be individually assignable and detectable. Hence, each probe comprises a distinct and individually identifiable tag complementary to a distinct capture probe on the microarray (e.g. ZIP and cZIP). Although the nucleic acid probe pairs may detect the same target ESBL nucleic acids in different samples, each amplified target ESBL nucleic acid derived from each sample is traceable because of its discrete tag, corresponding to a specific address on the microarray. In an alternative embodiment, the probes do not comprise tags, but only the primers used for amplification comprise a distinct and individually identifiable tag, such as a ZIP or cZIP. Obviously, the same considerations as mentioned above apply, in that the tags should differ per sample, and/or per probe, making each individual sample and/or probe identifiable. Accordingly, the present invention relates to a method as described herein, wherein amplified target nucleic acids derived from at least two samples hybridized to capture probes present on a single microarray.

Similar to detection of multiple samples on a microarray the same principles may be used for detection of multiple samples using microbeads.

The person skilled in the art will understand that the ZIP region and its complementary sequence cZIP intend only to reference the complementarity i.e. the ability to hybridize to each other specifically, irrespective of the position of the ZIP and/or cZIP sequence. The capture probe on the microarray may therefore comprise a cZIP, provided that the to be detected molecules comprise the ZIP region.

The data obtained by the methods of the present invention may be further analysed, possibly in an automated fashion. For instance, the hybridisation pattern obtained may be compared to hybridisation patterns stored in a databank. In this regard, the present invention relates also to a computer program stored on computer readable medium capable of performing the comparison of the obtained hybridisation pattern with the hybridisation patterns stored in a databank. Accordingly, the present invention relates to a computer comprising a computer readable medium capable of performing the methods described above. Also, the present invention relates to a computer readable medium comprising a computer program according capable of performing the method described above. Furthermore, the present invention relates to a computer program capable of displaying a web page on a remote computer enabling the use of the method described before.

After extracting, e.g. isolating, the target ESBL nucleic acid, the probes and/or primers are hybridised to the said target ESBL nucleic acid. In a preparing step, primers may be used to amplify the target ESBL nucleic acids. The probes may be ligated and may be amplified with primers specifically recognising regions on said probes. The probes and/or primers may be labelled. Also, the label may be incorporated during the amplification step or attached after amplification. Accordingly, the present invention relates to a method as described herein, wherein the amplified nucleic acid (ESBL amplicon) is labelled. Virtually any label that produces a detectable and/or quantifiable signal and that is capable of being attached to or incorporated into the amplified nucleic acid, can be used in conjunction with the methods and arrays of the invention. Suitable labels include, by way of example and not limitation, radioisotopes, fluorophores, chromophores, chemiluminescent moieties, etc. In embodiments where the label is attached to the amplified nucleic acid, the label can be attached to any part of the nucleic acid, including the free terminus or one or more of the bases. Preferably, the position of the label will not interfere with hybridisation, detection and/or other post-hybridisation modifications of the labelled nucleic acid. A variety of different protocols may be used to generate the labelled nucleic acids, as is known in the art, where such methods typically rely on the enzymatic generation of labelled nucleic acid using an initial primer and template nucleic acid. Labelled primers can be employed to generate the labelled amplified nucleic acid. Alternatively, label can be incorporated into the nucleic acid during first strand synthesis or subsequent synthesis, labelling or amplification steps in order to produce labelled amplified nucleic acid. Label can also be incorporated directly to mRNA using chemical modification of RNA with reactive label derivatives or enzymatic modification using labelled substrates. Representative methods of producing labelled amplified nucleic acid are disclosed in U.S. Application Serial nos.: 08/859,998; 08/974,298; 09/225,998.

The amplified nucleic acids may be labelled, for example, by the labels and techniques described *supra.* Alternatively, they may be labelled by any other technique known in the art. Preferred techniques include direct chemical labelling methods and enzymatic labelling methods, such as kinasing and nick-translation. Accordingly, the present invention relates to methods as described herein, wherein the amplified target nucleic acid is labelled. Preferably, the nucleic acid is labelled during amplification, or the amplified target nucleic acid is labelled after amplification. As such, the present invention relates to methods as described herein, wherein primer I and/or primer II are labelled. Labelling during amplification provides faster analysis times as it provides the opportunity to eliminate a process step where the amplified targets are labeled.

A variety of different labels may be employed, where such labels include fluorescent labels, phosphorescent labels, isotopic labels, enzymatic labels, particulate labels, etc. For example, suitable labels include fluorochromes, e.g. fluorescein isothiocyanate (FITC), rhodamine, such as rhodamine 123, R6G, IRDyes™, Texas Red, phycoerythrin, allophycocyanin, 6-carboxyfluorescein (6-FAM), 2',7'-dimethoxy-4',5'-dichloro-6-carboxy-fluorescein (JOE), 6-carboxy-X-rhodamine (ROX), TET, JOE, NED, (ET-)ROX, 6-carboxy-2',4',7',4,7-hexachloro-fluorescein (HEX), 5-carboxyfluorescein (5-FAM) or N,N,N',N'-tetramethyl-6-carboxy-rhodamine (TAMRA), fluor 488™, cyanine dyes, e.g. Cy5, Cy3, Cy2, BODIPY dyes, e.g. Biodipy™ 630/650, Biodipy 530, Biodipy™ FL, Alexa such as Alexa542, Alexafluor™ 532, etc. Suitable isotopic labels include radioactive labels, e.g. ³²P, ³³P, ³⁵S, ³H. Other suitable labels include size particles that possess light scattering, fluorescent properties or contain entrapped multiple fluorophores. The label may be a two stage system, where the primer and/or probe is conjugated to biotin, haptens, etc. having a high affinity binding partner, e.g. avidin, specific antibodies, etc. The binding partner is conjugated to a detectable label, e.g. an enzymatic label capable of converting a substrate to a chromogenic product, a fluorescent label, an isotopic label, etc.

In certain embodiments, the primers directed to different target ESBL nucleic acids, e.g. specific groups, may be differentially labelled. By "differentially labelled" and "contain a different label" is meant that the primers directed to different target nucleic acids are labelled differently from each other such that they can be simultaneously distinguished from each other. Hence, primer I may contain a label different from primer II. For instance, primer I or primer II binding to a first pair of probes, may contain a different label from primer I or primer II binding to a second pair of probes.

Only one of the primers of a primer set may be labelled, for example at its 5' end. Either the first primer or second primer may be labelled at its 5' end. Alternatively, both primers may be labelled with the same or different labels. In a multiplex, the method may operate using one common primer, e.g. hybridising to PBS(I), and one probe specific primer, e.g. hybridising to PBS(II). It will be appreciated that the common primer may hybridise to PBS(II), while the probe specific primer hybridises to PBS(I). In a further embodiment, probe I contains a label.

It should be noted that in case a labelled primer II comprising a sequence substantially complementary to PBS(II) is used, the first and second nucleic acid probes should provide ZIPs instead of cZIPs and preferably the ZIP is positioned on the first nucleic acid probe. By providing the ZIP on the first nucleic acid probe the labelled strand of the amplified ligated probe will contain a cZIP.

In a further embodiment of the present invention, the ZIP and/or DET is provided on the non-labelled strand of the amplified ligated probe.

In a further embodiment, said first nucleic acid probe is coupled with its 5' end to the 3' end of said second nucleic acid probe, possibly via a stuffer region.

It should be noted that in the instance that either a labelled primer I or a labelled primer II is used during the amplification of the ligated probe, a double stranded amplified ligated probe will be formed wherein one of the strands will be labelled, while the other strand will not be labelled. The present invention provides that the labelled strand of the amplified ligated probe comprises a ZIP, enabling this strand to hybridise with the cZIP, present on, for instance, a solid support, such as a microarray, microchip or bead. This same ZIP may be used for detection during PCR, i.e. real-time PCR, with the same cZIP having a fluorescent dye and/or quencher thereby enabling the monitoring of the increase of amplification products as the amplification reaction proceeds. Either the labelled or the non-labelled strand of the amplified ligated probe may comprise an additional ZIP sequence, enabling the detection with two different cZIP capture probes. By providing two ZIPs on respectively the labelled and the non-labelled strand of the amplified ligated probe, the detection of both occurs separately from each other without any hindrance between both detection strategies. In first instance the detection of the first ZIP will provide information regarding the presence or absence of the amplified ligated probe and consequently the presence or absence of the target micro-organism, whereas the samples that provide a positive detection signal of the first ZIP will subsequently be hybridized in the second screening step where the labelled strand of the amplified ligated probe containing the second ZIP will be detected.

It will be appreciated by a person skilled in the art that many variations exist, e.g. the amplification products will not be labeled and detection using the ZIP will only be done in real-time during amplification using fluorescently-labelled cZIP capture probes, or one strand of the amplification products will be labeled and detection using the ZIP will be done in real-time during amplification using fluorescently-labelled cZIP capture probes, and subsequently by hybridization to cZIP or ZIP capture probes coupled to a solid support. The person skilled in the art will appreciate that various variations are possible depending on the specific purpose, but all of which within the scope of the present invention. For instance, the 1 or 2 different ZIPs may be present on the same or on different strands, i.e. the sense and antisense strand, on the labelled and/or non-labelled strands. For instance, the ZIP may be present in the labelled strand facilitating real-time detection with a ZIP capture probe and a cZIP on a solid phase. For instance, none of the strand is labelled, one of both strands is labelled, both strands are labelled, with the same or different labels. In the latter case, differentiation of the strands is facilitated. In a preferred embodiment, primer I is labelled if the cZIP is located on the first or the second nucleic acid probe.

As already set out above, it will be appreciated that the label may be attached to at least one of the primers and/or probes, or in the alternative, may be incorporated during amplification. The label is for instance a fluorescent label. Accordingly, the present invention relates to a method as described herein, wherein at least one primer contains a label, and preferably a fluorescent label. This provides a cost efficient detection method.

The present invention especially contemplates that during the amplification procedure one or more capture probes are present. The presence of capture probes during the amplification enables the (real time) detection of the accumulation of amplified target nucleic acids and therefore this step provides a first screening of the samples. Since a large amount of the samples are presumed to be negative, the first fast and cheap screening step assesses whether or not ESBL micro-organisms are present or absent in the sample. Therefore, the more complicated, time consuming and more expensive second screening step, i.e. hybidrization using capture probes coupled to a solid support can be avoided when no ESBL micro-organisms are present.

The amplification of the target ESBL nucleic acids can be detected and/or quantified using the fluorescence or phosphorescence of a dye, which fluorescence or phosphorescence is associated either directly or indirectly with the multiplication of the amplified DNA. Since the amplification of the target nucleic acids is detected using detector molecules which can either be dyes intercalating double-stranded DNA or oligonucleotide capture probes complementary to the target nucleic acids, a person skilled in the art would expect that these capture probes may compromise the hybridization reaction of the second screening step if these capture probes are present during the hybridization. Since the hybridization of DNA is a very delicate process influenced by a large variety of environmental factors, the presence of these capture probes during the hybridization reaction should be avoided. Removing these capture probes prior to the second screening step is an elaborate process. However, the inventors have surprisingly found that the presence of these capture probes during the second screening step do not disrupt the results obtained during this screening nor do they increase the number of false positive or false negative results.

A direct detection method can for instance use a dye which binds nonspecifically to double-stranded DNA and only fluoresces or phosphoresces in connection with this binding. When the target nucleic acids are amplified, said dye binds to the newly formed double-stranded DNA such that the measurable fluorescence or phosphorescence increases. Examples of such dyes include, but are not limited to, SYBR Green-I®, ethidium bromide, propidium iodide, TOTO®-1{Quinolinium,1-1'-[1,3-propanediylbis[(dimethyliminio)-3,1-propanediyl]]bis [4-[(3-methyl-2(3H)-benzothiazolylidene) methyl]]-,tetraiodide}, and YoPro® {Quinolinium,4-[(3-methyl-2(3H)-benzoxazolylidene) methyl]-1-[3-(trimethylammonio)propyl]-,diiodide}. Most preferred dye for the instant invention is a non-asymmetrical cyanide dye such as SYBR Green-I®, manufactured by Molecular Probes, Inc. (Eugene, Oreg.).

Another direct detection method that can be used in the method of the present invention is a method using oligonucleotide capture probes. Oligonucleotide capture probes are short oligonucleotides complementary to the target nucleic acids. These oligonucleotide capture probes provide a fluorescent signal upon binding to the amplified target nucleic acids. The method by which the fluorescent or phosphorescent signal is provided by the oligonucleotide capture probes can be any method known in the art.

Different oligonucleotide capture probes may be used for carrying out the method of the present invention including, but not limited to a 5' nuclease assay in which the oligonucleotide detector probe carries a fluorogenic reporter dye at its 5' end and a quencher at its 3' end, Scorpion primers in which a primer is covalently linked to the probe, the primer probe complex comprising a fluorophore and a quencher molecule each linked to either the primer or the probe, and Molecular Beacons which are hairpin shaped molecules with an internally quenched fluorophore.

A well-known method for detection of the accumulation of amplification products during PCR using capture probes is the 5' nuclease assay, also known as TaqMan assay. This assay employs capture probes that hybridize to one of the strands of the amplification product. In the present invention, such capture probes will be essentially complementary or identical to the ZIP or ZIPs present in a particular probe pair. The capture probes will typically have a fluorescent dye at the 5'end, and a fluorescent quencher at the 3'end. Such capture probes will excite a low fluorescence because of quenching of fluorescence of the 5'dye by the quencher at the 3'end. These capture probes will hybridize to the amplicons during PCR together with the PCR primer of the strand to which the capture probe is hybridized. After binding of the PCR primer, the polymerase will extend the PCR primer, thereby removing the 5'nucleotide with its dye from the capture probe using its 5'exonuclease activity. The dye-labeled 5'nucleotide will be released in the reaction mixture, after which its fluorescence may be monitored.

A second well-known method for detection of the accumulation of amplification products during PCR using capture probes is the use of so called Molecular Beacons. In this approach such molecular beacon capture probes will typically hybridize to one of the strands of the amplification product. In the present invention such molecular beacon capture probes will be essentially complementary or identical to the ZIP or ZIPs present in a particular probe pair. Molecular beacon capture probes will typically have a fluorescent dye at the 5'end, and a fluorescent quencher at the 3'end. This 5'dye and 3'quencher will be held in close proximity to each other, because additional nucleotides are added to the 5'end and 3'end of the capture probes creating a panhandle (hairpin loop) structure due to internal base-pairing of these extra 5' and 3' nucleotides. Such capture probes will excite a low fluorescence because of quenching of fluorescence of the 5'dye by the quencher at the 3'end. These capture probes will hybridize to the amplicons during PCR, because the base-pairing to the amplicons is essentially stronger than the internal base-pairing of the capture probe's panhandle. Opening of the panhandle structure will result in the fluorescent 5'dye being no longer quenched by the 3'quencher, after which its fluorescence may be monitored. For a comprehensive review on molecular beacon probes see: Vet, J.A.M. and Marras, S.A.E., Methods in Molecular Biology (2004), Vol. 288, pp 273 - 290, Humana Press Inc., NJ USA, Herdewijn, P., ed. Finally, detection using molecular beacon capture probes may be combined with the monitoring of the melting behaviour of such probes as described in: El Hajj, H.H. et al., J. Clin. Microbiol. (2009), Vol. 47, pp 1190 - 1198.

A third well-known method for detection of the accumulation of amplification products during PCR using capture probes is the use of so called Scorpion primers. In this approach such capture probes are typically linked to one of the amplification primers, and the fluorescent signal is created by internal hybrization of the capture probe to the extension product of the amplification primer to which the capture probe is linked. Such a Scorpion primer-capture probe chimaera may have various structures, which are reviewed in: Carters R. et al., Methods in Molecular Biology (2008), Vol. 429, pp 99 - 115, Humana Press Inc., NJ USA, Marx A. and Seitz, O., eds.

A fourth well-known method for detection of the accumulation of amplification products during PCR using capture probes is the use of so called Fluorescence Resonance Energie Transfer probes, FRET probes. In one particular embodiment of this invention such FRET capture probes will constitute two independent probes both hybridizing adjacent to each other to one of the strands of the amplification product. At least one of the FRET probes will be complementary to the ZIP or cZIP, the other one will be complementary to a second ZIP or cZIP sequence, or to another sequence of the amplicon to be detected. The two FRET capture probes will each have a fluorescent label, one probe will have a 3'fluorescent dye, the other probe will have a 5'fluorescent dye. After hybridization of the two FRET capture probes adjacent to each other on the amplicon, the fluorescence of the dye of one of the probes will by enhanced by the close proximity of the dye of the other probe. In another embodiment of this invention a dye will be incorporated in the amplicon at a specific location, e.g. in the amplification primer, which for this purpose will be internally labeled. One FRET capture probe will be used in this embodiment and the upon hybridization of the FRET capture probe, the dye of this probe will be in close proximity to the dye incorporated in the amplicon, thereby enhancing the fluorescence of the dye of the FRET capture probe. For reference see e.g US-6174670 and US2006-6281099.

It will be appreciated by a person skilled in the art that more principle for detection of the accumulation of amplification products using capture probes exist, that are not further detailed in this invention.

Fluorophores that are frequently used as tags for FRET include fluorescein, 5-carboxyfluorescein (FAM), 2'7-dimethoxy-4'57-dichloro-6-carboxyfluorescein (JOE), rhodamine, 6-carboxyrhodamine (R6G), N,N,N7,N'-tetramethyl-6-carboxyrhodamine (TAMRA), 6-carboxy-X-rhodamine (ROX), 4-(4'-dimethylaminophenylazo)benzoic acid (DABCYL,), and 5-(2'-aminoethyl)aminonaphthalene-1-sulfonic acid (EDANS). Other potential FRET donor or acceptor molecules are known in the art (See US Pat. No. 5,866,336, Table 1). The skilled artisan will be familiar with the selection of pairs of tag molecules for FRET (US Pat. No. 5,866,336).

Besides enabling the real time detection of the amplified target nucleic acids the use of FRET oligonucleotide detector probes also enables the quantification of the target nucleic acids.

It will be apparent that the present invention relates to a method as described herein, wherein a signal is detected after and/or during amplification of the target nucleic acids. The said signal is preferably a fluorescent or phosphorescent signal, and said fluorescent or phosphorescent signal may be detected by a CCD camera or by laser scanning.

By providing detector molecules during the amplification procedure it is possible to perform in the first step of the method of the present invention a screening towards the presence of ESBL nucleic acids in the samples. Since the second step of the method of the invention, which involves the further characterization and identification of the ESBL nucleic acids by hybridizing the amplified target nucleic acids to capture probes coupled to a solid support, such as a microarray or microbead, has a higher cost per test, a reduction of the samples to be analyzed in the second step of the method of the present invention would be highly beneficial.

Therefore, the present invention provides a method wherein the amplified target nucleic acids hybridized are the target sequences providing a positive signal in the method of the present invention.

In a specific embodiment of the present invention, said detector molecules are one or more oligonucleotide detector probes and more preferably FRET oligonucleotide detector probes, having a sequence at least partially complementary to a target nucleic acid sequence to be detected and including a fluorescent reporter molecule and a fluorescent quencher molecule capable of quenching the fluorescence of said reporter molecule, said oligonucleotide probe existing in at least one single-stranded, partially single-stranded or double-stranded conformation when not hybridized where said quencher molecule quenches the fluorescence of said reporter molecule, said oligonucleotide probe existing in at least one conformation when hybridized to said target nucleic acid where the fluorescence of said reporter molecule is unquenched.

In a more specific embodiment of the present invention, the sequence of said oligonucleotide detector probes is at least complementary to at least one region, e.g. cZIP or ZIP of the first nucleic acid probe and/or at least one region, e.g. cZIP or ZIP, of the second nucleic acid probe.

The present invention provides that at least two oligonucleotide detector probes are provided with different fluorescent reporter molecules thereby providing an assay where depending on the detected type of fluorescent reporter molecule or the detected amount of each fluorescent reporter molecule information is obtained regarding the type of target ESBL nucleic acid that is detected as well as the amount of each type of target ESBL nucleic acid.

In a further embodiment of the present invention the first nucleic acid probe and/or the second nucleic acid probe further comprise at least one DET, which is (i) essentially complementary (for instance after amplification) to one or more oligonucleotide detector probes used for detecting the accumulation of the reaction products during the amplification reaction, (ii) essentially non-complementary to said target nucleic acid and/or (iii) which is located in between the target sequence and the primer binding section.

Said DET is a unique sequence for identification of the eventually amplified products. The DET will hybridize to its complementary oligonucleotide detector probe present during the amplification reaction. Consequently, when oligonucleotide detector probes are used for the detection of the amplified target nucleic acids, said oligonucleotide detector probe would comprise a complementary cDET, an oligonucleotide sequence complementary with the DET. Similar to ZIP/cZIP, DET may not be a unique DET specific for a specific ESBL gene but it may be a DET specific for a specific group of ESBL genes, such as ESBL_{A}, ESBL_{M} and/or ESBL_{CARBA}.

It will be appreciated that the cZIP and/or ZIP, being unique sequences, may function as DET. Also, DET may function as a cZIP and/or ZIP. Hence, in an embodiment the cZIP is, or functions as, DET. In a further embodiment ZIP is, or functions as, DET. In an even further embodiment the DET is, or functions as, ZIP and/or cZIP.

In a specific embodiment of the present invention, the method is provided in such a way that the entire screening process is performed in a closed system. To avoid contamination and provide highly reliable results the method of the present invention should be performed in an automated manner and in a closed system thereby reducing the risks for contamination and user errors.

After determining the ESBL nucleic acid according to the methods of the invention, the ESBL nucleic acid may be further analysed. A convenient method to analyse said amplified nucleic acid or said amplified nucleic acid mixture is by determining the sequence thereof. Techniques to determine the sequence of nucleic acids are well known in the art. Accordingly, the present invention relates to a method as described herein, wherein the analysis comprises determining the sequence of the amplified nucleic acid mixture. Said sequence may be determined via enzymatic, chemical or physical means. The sequence determined of the contaminating organism may be compared with sequences stored in a databank. Also the step of analysing in the method for characterising ESBL micro-organisms possibly present in a sample according to the present invention, may comprise providing a computer readable medium carrying computer output data having a database characterising ESBL nucleic acids, genes, proteins and micro-organisms based on nucleotide sequences, providing a computer and algorithm, processing the computer output data to determine the ESBL nucleic acids, genes, proteins and micro-organisms.

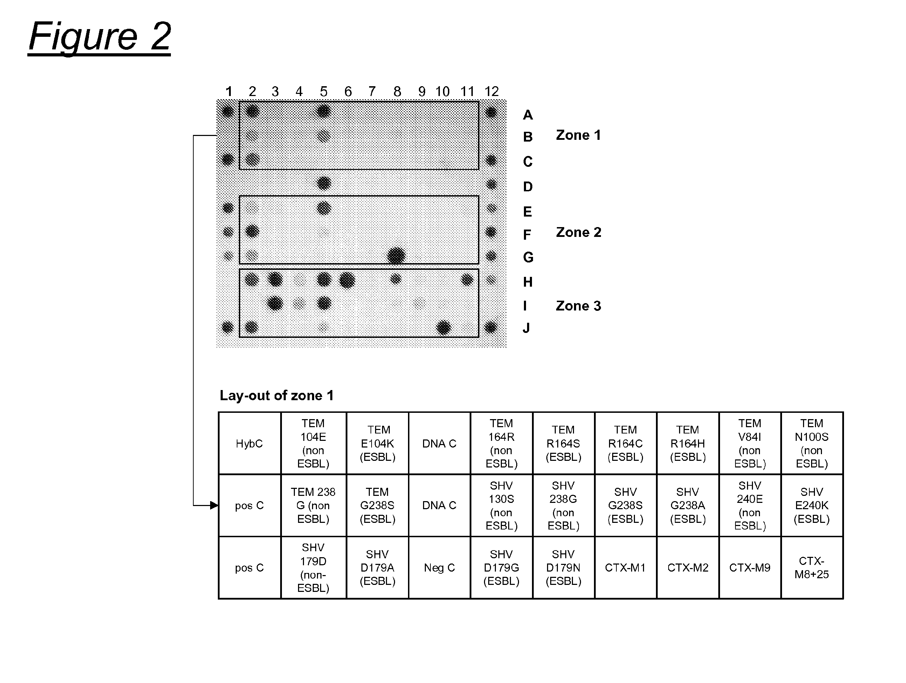

The microarrays of the present invention may be of any desired size, from two spots to 10⁶ spots or even more. The upper and lower limits on the size of the substrate are determined solely by the practical considerations of working with extremely small or large substrates. In general, microarrays contain from 2 to about 10⁶ spots, or from about 4 to about 10⁵ spots, or from about 8 to about 10⁴ spots, or between about 10 and about 2000 spots, or from about 20 to about 200 spots. Not all spots on the microarray need to be unique. Indeed, in many applications, redundancies in the spots are desirable for the purposes of acting as internal controls (see e.g. figure 2). A variety of techniques for making microarrays are known in the art, and have been described e.g. in U.S. Pat. No. 5,744,305, WO 98/31836. Preferably, the capture probes on the microarray are identifiable by their spatial addresses. The nature and geometry of the solid substrate will depend upon a variety of factors, including, among others, the type of array (e.g., one-dimensional, two-dimensional or three-dimensional) and the mode of attachment (e.g., covalent or non-covalent). The present invention relates to a method as described herein, wherein said microarray is a flow-through microarray.

Similarly, the number of microbeads of the present invention may be of any desired number, from two different beads to 10⁶ beads or even more. The upper and lower limits on the number are determined solely by the practical considerations. Not all beads need to be unique. Indeed, in many applications, redundancies in the beads are desirable for the purposes of acting as internal controls. Preferred microbeads are Luminex microbeads., which may be sorted using a Fluorescence-Assisted Cell Sorting apparatus, like the Luminex Analyzer.

The present specification describes kits for determining the presence of micro-organisms in a sample comprising the essentials of the methods of the present inventions, for instance, said kits may comprise possibly a filter, possibly means for extracting nucleic acids from said micro-organisms, means for specifically amplifying said nucleic acids, means for detecting the amplified nucleic acids, possibly means for analysing the amplified nucleic acids, e.g. microarrays, such as flow through microarrays, possibly buffers and/or an instruction manual.

It will be evident to the person skilled in the art that the present invention relates to the use of a microarray as mentioned herein in the method of the present invention. Also, the present invention relates to the use of at least one pair of a first nucleic acid probe and a second nucleic acid probe as defined supra, including coupled probes, and/or the use of at least one set of two primers as defined above, in the methods according to the invention.

Before the subject invention is described further, it is to be understood that the invention is not limited to the particular embodiments of the invention described herein, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

The following examples and figures are offered by way of illustration and not by way of limitation. Nevertheless, the content of said examples and figures may be generalised in the concept of the present invention.

### EXAMPLES

### EXAMPLE 1 MATERIALS and METHODS

### 1.1 Probe design

To identify essential SNPs associated with the ESBL phenotype for inclusion in the assay, sequences of all listed TEM variants (n=175) and SHV variants (n=128, SHV-1 through SHV-127 including SHV-2 and SHV-2a) in the Lahey database (http://www.lahey.org/Studies/) as of 6 September 2009 were analyzed and related to phenotypic characteristics as described in the literature (www.pubmed.gov).

### 1.1.1 TEM variants:

The Lahey database contained 175 TEM variants. According to this database, no data were available for 12 variants (reserved, withdrawn or not listed), 24 variants had an unknown phenotype, 51 variants had an non-ESBL beta-lactamase phenotype, and 88 had an ESBL phenotype of which 95% (84/88) had at least one of the following amino acid substitutions: R164 S/H/C (n= 50 variants), G238 D/N/S (n= 30 extra variants), E104K n=4 extra variants) (Table 1 a). These substitutions were not observed in non-ESBL TEM variants.

Based on these findings, 10 oligonucleotide probes (Supplementary Table 1) were designed for identification of TEM variants (3 probes for non-ESBL variants, 5 for ESBL variants, and 2 for identification of TEM-116). Two probes for detection of TEM-116 (presence of V84I substitution and absence of N100S substitution) were incorporated in the assay, because TEM-116 has been reported as an ESBL in several publications and >50 isolates with this TEM variant have been reported.The microarray system reports TEM-116 as ESBL-positive and was able to detect and identify TEM-116 in one isolate (data not shown). However, the Lahey database categorizes TEM-116 as an unknown phenotype and publications on the phenotype of TEM-116 are controversial. Contamination of several *Taq* polymerases by TEM-116 may have played a role in experiments attempting to establish the ESBL phenotype of this TEM variant. Probes for detection of substitutions G238D and G238N, described exclusively in TEM-111 and TEM-142, (unknown phenotypes according to the Lahey database; no published data available) were not incorporated in the assay, because these two TEM variants also harbour the E104K substitution, allowing detection by the microarray system. Four ESBL TEM variants (TEM-126, -157, -164 and -169) do not contain any of the ESBL-associated substitutions mentioned above, and are therefore not detected by the array (Table 1a). In the literature, TEM-126 and TEM 164, have each been reported in one clinical isolate. TEM-157 and TEM-169 also have an ESBL phenotype according to Lahey database, but published evidence is insufficient to determine the phenotype of TEM-157, and no published data were found on TEM-169 in Pubmed or Embase. Finally, 5 TEM variants (TEM-111, -123, -124, -142, and -165) with unknown phenotypes according to the Lahey database contain at least one ESBL-associated substitution and will therefore be identified as ESBL positive by the assay. In the literature, SNPs at TEM positions 237 and 240 have also been associated with the ESBL phenotype. However, since ESBLs with substitutions at position 237 and 240 also have substitutions at position 104, 164 or 238 (http://www.lahey.org/Studies/), probes for detection of these SNPs were not included in the assay.

### 1.1.2 SHV Variants:

The Lahey database contained 128 SHV variants. According to this database, no data were available for 20 variants, 38 variants had an unknown phenotype, 35 variants had a non-ESBL phenotype, and 35 had an ESBL phenotype of which 77% (27/35) had at least one of the following mutations on amino acid position: D179 A/N/G (n=3 variants), G238 S/A (n=22 extra variants) and E240K (n=2 extra variants) (Table 1b). SHV-10 has a non-ESBL phenotype, although it harbours the G238S and E240K substitutions due to a unique compensatory mutation at amino-acid position 130 (S to G) precluding the extension of the beta-lactamase spectrum.

For identification of SHV variants, 10 oligonucleotide probes were designed (3 probes for non ESBL variants, one for exclusion of SHV-10, and 6 probes for ESBL variants)(Supplementary Table 1).

Since ESBL variants SHV-16, 57, and 70 do not possess one of the ESBL associated substitutions mentioned above, the microarray system does not identify these SHV variants as ESBL. Five other SHV-genes (SHV-27, -38, -40, -41, -42) with an ESBL phenotype according to the Lahey database also lack these substitutions and are consequently not identified as ESBL. However, the published evidence on the phenotype is contradictory for four of these variants: SHV-27, SHV-40, SHV 41, SHV 42, and SHV-38 is a chromosomally encoded Class A carbapenemase. On the other hand, SHV-20, 21, and 22 are categorized as all non-ESBL in the Lahey database, while they are reported as ESBL in the literature including the reference from the Lahey database. In line with the published data, these three SHV variants are detected as ESBL-positive by the microarray system, since they all harbour at least one ESBL-associated mutation (Table 1 b).

### 1.1.3 CTX-M variants:

An alignment was made of the CTX-M sequences 171 mentioned in the Lahey database using ClustalX Software and 4 probes were designed based on conserved regions, where near sequence identity is observed within each group and there is strong discrimination between the groups (CTX-M-1, CTX-M-2, CTX-M-9, and CTX-M 8/25 group). The detection of the CTX-M-8 and -25 groups was combined because of the low prevalence of these gene clusters.

### 1.2 Test isolates

To evaluate the diagnostic accuracy of the microarray system, a total of 212 genotypically and phenotypically well-characterized isolates were tested. The characteristics of the test isolates (bacterial species, beta-lactamases, genotype and phenotype, result of the array system) are presented in supplementary Table 2. The collection contained the following bacterial species: *Escherichia coli* (n=139), 184 *Enterobacter cloacae* (n=12), *Citrobacter freundii* (n=2), *Citrobacter werkmanii* (n=1), 185 *Klebsiella oxytoca* (n=9), *Klebsiella pneumoniae* (n=16), *Proteus mirabilis* (n=7), 186 *Salmonella enterica* (n=26).

In total, 106 ESBL-positive isolates and 106 ESBL-negative non-repeat isolates of human (n=132) and veterinary (n=80) origin were included. An overview of the ESBL genes in the ESBL-positive isolates is presented in Table 2. The 106 ESBL-positive test isolates contained 110 ESBL genes (4 isolates harboured 2 ESBL genes), including 29 TEM ESBLs, 35 SHV ESBLs and 46 CTX-M genes. The 106 ESBL negative isolates included 17 isolates with plasmid-mediated AmpC, 6 isolates withOXA beta-lactamases, 7 were *K*. *oxytoca* K1 hyperproducers, 7 were *E. coli* with chromosomal AmpC hyperproduction, and 17 isolates harboured a non-ESBL TEMor SHV variant. The remaining 52 isolates consisted of 50 beta-lactam-susceptible *E.coli* blood culture isolates, and 2 susceptible *P. mirabilis* isolates lacking TEM, SHV or CTX-M genes. With this collection, 20 of the 24 probes for ESBL detection could be evaluated (Table 2). For detection of TEM ESBLs, one of the 10 probes was not evaluated because no isolate with the TEM R164C substitution was included in the test collection. For detection of SHV ESBLs, 3 of the 10 probes were not evaluated because no isolates were available with the SHV D179A, D179G or D179N substitutions.

### 1.3 Phenotypic characterization

To compare the results of the microarray system with phenotypic susceptibility testing, the Phoenix automated system with the NMIC-ID75 card (Becton Dickinson Diagnostic Systems, Baltimore, MD, USA) was used to determine of MICs. Confirmatory tests for ESBL production were performed if the ESBL screentest was positive (MIC of ceftriaxone and/or ceftazidime >1 mg/L). Confirmation of ESBL production was performed using Etests (AB Biodisk, Solna, Sweden) with ceftazidime +/- clavulanic acid, cefotaxime +/- clavulanic acid according to the CLSI Performance Standards for Antimicrobial Susceptibility Testing. In addition, to detect ESBL production in isolates co-expressing an AmpC beta-lactamase, a cefepime +/- clavulanic acid ESBL Etest was performed in parallel.

### 1.4 DNA isolation

DNA isolation was performed using Nucleospin Tissue Columns (Macherey-Nagel, Düren, Germany) according to the instructions of the manufacturer.

### 1.5 PCR and Sequencing

The presence of TEM, SHV, CTX-M was determined using PCR and sequencing of the same batch of isolated DNA as used in the microarray, using primers listed in Table 3. Sequencing of the amplicon with the selected primer pairs for TEM and SHV allows for discriminating between all TEM and SHV ESBL genes described in the Lahey database.

To detect the presence of CTX-M genes, a general CTX-M PCR was performed. Based on the sequence of the resulting 592 bp amplicon the CTX-M group (CTX-M-1, CTX-M-2, CTX-M-8, CTX-M-9 and CTX-M-25) was determined. To identify the members within these CTX-M groups, specific primers for amplification and sequencing were used. However, for members from the CTX-M-25 group (CTX-M-25, 26, 39 and 41) sequencing of the amplicon of the general CTX-M PCR was sufficient.

To detect the presence of plasmid-encoded AmpC genes, a multiplex PCR was performed. Hyperproduction of chromosomal AmpC in *E. coli* isolates was deduced from the phenotype, combined with a negative result of the plasmid AmpC multiplex PCR. Hyperproduction of chromosomal K1 in *K*. *oxytoca* isolates was deduced from the phenotype in combination with negative TEM, SHV and CTX-M PCR reaction. The OXA-positive isolates have been described in Paauw, et al. (2007 Emerg.Infect.Dis. 12:807-812).

### 1.6 Ligation mediated amplification and microarray analysis

Using the probes described above the protocol of Wattiau et al. (2008 Int. J. Food Microbiol. 123:293-298) was used. The resulting ESBL Array (Check-Points, Wageningen, The Netherlands) was supplied as a kit containing reaction buffers, the probes mentioned above (Supplementary Table 1), ArrayTubes 246 (Clondiag Chip Technologies, Jena, Germany), reagents and buffers for the microarray hybridization and staining. Standard laboratory equipment was used including two thermocyclers (MyCycler, Biorad, La Jolla, CA), and a rotating heating block (Eppendorf, Hamburg, Germany).

The assay was performed in two separate laboratory rooms. In the pre-PCR-room, DNA isolation, ligation and preparation for amplification were performed, and in the PCR room, amplification, hybridization and detection were performed as previously described in the protocol of Wattiau et al. *(ibid).*

Microarray images were generated using a microarray reader (ArrayTube Reader, ClonDiag Chip Technologies, Jena, Germany) connected to a standard personal computer running dedicated software for analysis of the images.

The software reported the isolate as "ESBL" if a) at least one of the ESBL-associated substitutions is detected in TEM (E104K, R164S, R164C, R164H, G238S) or SHV (G238S, G238A, E240K, D179A, D179G, D179N) b) a CTX-M gene is detected c) a TEM-116 was identified based on presence of the V84I substitution in TEM without the N100S substitution (Table 1, Figure 2). The software indicated whether a (combination of) TEM-, SHV- or CTX-M ESBL had been detected, and specified the CTX-M group (CTX-M group 1, 2, 9, or combined 8/25). If SHV or TEM genes were detected without any of the ESBL-associated substitutions, the test result was "SHV no ESBL", or "TEM no ESBL", respectively. The assay was not able to provide a Lahey number for TEM and SHV genes (e.g. TEM-6 or SHV-2), because multiple TEM and SHV ESBL variants share the same SNPs on the positions detected by the assay (Table 1, Table 2). Furthermore, if e.g. two TEM ESBL SNPs are detected, it is impossible to distinguish between the presence of two TEM genes 270 with one SNP per gene versus the presence of one TEM gene with two SNPs.

The software reported "No ESBL" if no TEM, SHV and CTX-M genes was detected. The software advised to repeat the assay when a) the result was "ESBL suspected" i.e. in case one of the ESBL-associated spots on the array appeared positive but the pattern of spots was otherwise not consistent with a known ESBL, b) when artifacts e.g. air bubbles hampered microarray reading and interpretation, or c) when the amount of DNA input was insufficient.

As shown in Figure 2, an array contains 3 zones, allowing parallel analysis of 3 isolates. Performance time of the microarray was 8 hours (hrs) per 36 isolates (3 hrs DNA isolation; 5 hrs ligation, amplification and detection).

### 1.7 Statistics

Test characteristics (sensitivity and specificity) are presented with 95% confidence intervals.

### EXAMPLE 2 RESULTS

### 2.1 Test characteristics of the microarray

Using molecular characterization by PCR and sequencing, and the corresponding 299 phenotype according to the Lahey database as the reference test, the sensitivity of the microarray was 101/106 (95%; 95%CI 89-98%), the specificity was 106/106 (100%; 95%CI 97-100%).

A false-negative result was obtained in 5 ESBL positive isolates (supplementary table 2). In two isolates, TEM-5 and TEM-7 positive, the R164S mutation was not detected and in one isolate, TEM-72 positive, the G238S mutation was not detected. Detection of these mutations is essential since the difference with non-ESBL TEM variants is based on these single mutations (Table 1). In one isolate, a CTX-M-39 gene (CTX-M group 8/25) was not detected. Finally, as expected, the SHV-57 ESBL was not detected because the SHV-57 does not have amino acid substitutions at positions detected by the array system (Table 1b and 2).

### 2.2 Accuracy of individual probes to identify specific SNPs

The sensitivity per probe (pair) to identify specific amino acid substitutions conferring ESBL in TEM and SHV and to identify CTX-M groups is shown in Table 2. Visual inspection of the microarray pictures showed that the R164S substitution in TEM was not detected in 4 out of 10 isolates containing TEMs with this substitution. For the TEM-5 and TEM-7 positive isolates this resulted in a false-negative outcome, whereas two TEM-9 isolates tested ESBL-positive, due to detection of the E104K substitution also present in TEM-9.(Table 1a and 2). The TEM G238S substitution was missed in 1 of 17 isolates with that substitution (Table 2), leading to the false ESBL-negative result of a TEM-72 positive isolate (see above). 320 The SHV G238S was not detected in one (a *K*. *pneumoniae* with SHV-12) of 33 isolates with this substitution but this did not result in a false-negative assay result, because the E240K substitution of SHV-12 was detected.

The specificity of the probes was high, since in only 2 test isolates spots were detected that were not expected based on the sequencing results. In one isolate with a TEM-19 an additional TEM E104K spot was detected and in another isolate with a TEM-18 an additional TEM G238S spot was detected.

### 2.3 Test characteristics of the phenotypic assay

Using the molecular characterization by PCR and sequencing, and the corresponding phenotype according to the Lahey database as the reference test, the sensitivity of the phenotypic ESBL testing was 104/106 (98%; 95% CI 93-99%). The test result for one *E. cloacae* isolate, containing a CTX-M-9 was out of range (non-determinable) in all 3 ESBL Etests and for one *E.coli,* containing a SHV-2 ESBL and an ACC-1 plasmid-mediated AmpC the Etest results were ESBL negative. Using the microarray system, the CTX-M-9 and SHV-2 were correctly detected in these 2 isolates.

For the 7 *K*. *oxytoca* isolates with K1 hyperproduction without genotypic evidence of TEM ESBL, SHV ESBL or CTX-M ESBL genes the phenotypic ESBL testing was positive while these isolates, as expected, tested negative in the microarray system. One *E. coli* isolate had a positive phenotypic ESBL test, although no TEM, SHV or CTX-M genes were detected by either PCR or the microarray system.

### EXAMPLE 3 RNA-BASED AMPLIFICATION TECHNIQUES

The present invention also relates to RNA-based amplification techniques for amplifying the connected probe assemblies. The present example illustrates the LDR-NASBA and LDR-TMA technique. These techniques require the conversion of DNA and/or cDNA into RNA through the combined action of reverse transcriptase and RNA polymerase activities, e.g. T7 RNA polymerase. As schematically represented in Figure 3, the nucleic acid probes bind to the target sequence via TSS-1 and TSS-2 in the first step (1). One of the probes additionally comprises the T7 promoter at its 5' terminus. Then, a conventional LDR step is performed resulting in the formation of a single-stranded connected probe nucleic acid (2). Subsequently, a reverse transcriptase activity synthesizes the complementary DNA strand of the single strand connected probe DNA formed in step 2 resulting in double-stranded DNA (3). Next, RNA polymerase activity (T7 RNA polymerase) catalyzes the formation of RNA from the double-stranded DNA template formed in step 3 (4). Finally, (the cyclic phase of) NASBA or TMA amplification is performed using the RNA transcribed in step 4 as template (5).

It is found that the following combinations exclude false positives during a subsequent detection step, in which the amplified connected probe nucleic acid is detected on a microarray system: (a) the combination in which the first nucleic acid probe and primer I comprise the T7 promoter (T7-p) and ZIP is located on the first nucleic acid probe (see Figure 3A), (b) the combination in which the first nucleic acid probe and primer I comprise the T7 promoter (T7-p) and ZIP is located on the second nucleic acid probe (see Figure 3B), or (c) the combination in which primer II comprises the T7 promoter, the second nucleic acid probe comprises a region that is complementary to the T7 promoter (cT7-p) and cZIP is located on the first nucleic acid probe (see Figure 3C).

These specific combinations are further detailed below, with reference to the Figures 3A-3C.
(1.) If the target is present, the nucleic acid probes anneal to the target sequence via TSS-1 and TSS-2.
(2.) Subsequently, the adjacent nucleic acid probes are connected via a ligation reaction to form a connected probe nucleic acid.
(3.) Next, only one primer (primer II) can hybridize to the connected probe nucleic acid, and can be used as a primer for synthesis of the complementary DNA strand using a reverse transcriptase. This results in the formation of double-stranded connected probe DNA.
(4.) The T7 RNA polymerase can only transcribe RNA in the 5' → 3' direction, downstream of the T7 promoter (T7-p) and from the double-stranded connected probe DNA as template. The generated RNA comprises ZIP, which is complementary to cZIP on a micro-array.
(5.) The RNA is used as template for amplification during a NASBA or TMA step using primers I and II.

Thus, RNA molecules are generated that comprise ZIP that is complementary to cZIP on a micro-array and can thus be detected.

Other combinations of locations of ZIP/cZIP and T7-p/cT7-p on the nucleic acid probes/primers result in either no positive amplicons or a high number of false positives.

### EXAMPLE 4 REAL-TIME DETECTION AND MICROARRAY TYPING OF ESBL GENES

### 4.1 Bacterial strains

89 E.coli and 66 K.pneumoniae strains were selected for analysis. Strains contained up to 5 beta-lactamase genes.

### 4.2 DNA isolation

DNA isolation was performed using Nucleospin Tissue Columns (Macherey-Nagel, Düren, Germany) according to the instructions of the manufacturer.

### 4.3 Probe and Array design

10 oligonucleotide probe pairs for TEM-ESBL, SHV-ESBL and CTX-M ESBL's (Supplementary Table 1) were selected for identification of ESBL_{A} variants. Following similar principles 7 probe pairs for ESBL_{M} and 4 probe pairs for ESBL_{CARBA} were designed and included in the assay. Probe pairs were detected with a unique ZIP for detection on the ArrayTube and one of three DET sequences specific for either ESBL_{A}, ESBL_{M} and/or ESBL_{CARBA}. Figure 5 displays the Array Tube format for typing of ESBL genes, and includes reaction controls and probe pairs for TEM and SHV non-ESBL gene variants.

### 4.4 DET sequences and cDET real-time TaqMan probes

Figures 6a and 6b display the probe design, and various DET sequences and cDET real-time beacon and TaqMan probes used for real-time ESBL_{A}, ESBL_{M} and/or ESBL_{CARBA}.detection and subsequent microarray typing.

### 4.5 Ligation mediated real-time PCR amplification

Using the probes described above the ligation step as described in the protocol of Wattiau et al. (2008 Int. J. Food Microbiol. 123:293-298) was used with each of the 155 bacterial DNA samples. After ligation, 5 µl of each ligation reaction mixes was transferred to a clean PCR tube. Next, 7.5 ul of a mix containing 1 nM Eco- and Mse-primer (Wattiau et al. 2008 Int. J. Food Microbiol. 123:293-298) and 0,5 nM cDET-1, c-DET-2 and c-DET-3 Beacon or TaqMan probe was added. Finally, 12,5 µl of TaqMan MasterMix (Applied Biosystems, Foster City, CA) was added and the reaction mixtures were amplified in a ABI-7500 thermocycler using 1 cycle of 10 minutes at 95°C and 40 cycles at 10 seconds 95°C and 30 seconds 65°C.

### 4.6 ArrayTube analysis of real-time PCR amplification products

10 µl of real-time PCR reaction products were used for ArrayTube ESBL typing as described by Wattiau et al. (2008 Int. J. Food Microbiol. 123:293-298).

Microarray images were generated using a microarray reader (ArrayTube Reader, ClonDiag Chip Technologies, Jena, Germany) connected to a standard personal computer running dedicated software for analysis of the images.

### 4.7 Results of the real-time PCR detection

Figure 7 displays the flow scheme of the LDR real-time PCR and Array detection. Table 4 displays the results obtained with the 89 E.coli and 66 K.pneumoniae strains. The real-time tests showed a total of 65 ESBL_{A} genes, 83 ESBL_{M} genes and 30 ESBL_{CARBA} genes in the 155 strains. These strains displayed from 0 to 3 ESBL genes. Figure 8 shows 6 real-time amplification profiles obtained with 6 of the 155 bacterial strains.

### 4.8 Results of the microarray typing of real-time PCR products

Table 5 displays the typing results of the 155 bacterial strains. A large variety of ESBL genes were found. The ESBL real-time PCR detection and microarray-based typing of 89 E.coli and 66 K.pneumoniae strains are provided_ESBL_{A}, ESBL_{M} and ESBL_{CARBA} refer to the various ESBL groups categorized by beta-lactam degrading characteristics as depicted on the table of page 2 of this application. The presence of one or more of these ESBL groups was detected by real-time PCR. "total" refers to the total number of strains belonging to either ESBL_{A}, ESBL_{M} or ESBL_{CARBA}. When positive for any of the ESBL groups, real-time PCR products were further typed by microarray analysis. Gene types distinguished for time ESBL_{A} included TEM, SHV, CTX-M1 type, CTX-M2 type and CTX-M9 type, for ESBL_{M}, CMY-2, FOX, CMY-1/MOX, DHA, ACC and MIR/ACT, and for ESBL_{CARBA}, KPC and NDM. Figure 9 displays two of the 155 ESBL microarray images.

**Table 1a: Phenotypes of TEM variants and corresponding amino acid substitutions that may be detected by the array system.**

| **Phenotype according to Lahey database** | **TEM Variants** | **ESBL Associated Amino Acid Substitutions at Positions Detected by Array** | | | **Expected Array Result** |
|---|---|---|---|---|---|
| | | **E 104 K** | **R 164 S/C/H** | **G 238 S** | |
| Non ESBL | TEM-1, 2, 13, 30-40, 44, 45, 51, 54, 55, 57-59, 65, 67, 73, 74, 76-84, 90, 95, 97-99, 103, 110, 122, 127, 128, 135, 141, 145, 159, 160, 163 | - | - | - | Negative |
| ESBL | TEM-17, 18, 56, 106, 124 | + | - | - | Positive |
| ESBL | TEM-9, 24, 26, 46, 60, 63, 121, 129, 130, 131, 133, 149 | + | + S | - | Positive |
| ESBL | TEM-87 | + | + C | - | Positive |
| ESBL | TEM-6, 16, 43, 109 | + | + H | - | Positive |
| ESBL | TEM-167 | + | - | + | Positive |
| ESBL | TEM-3, 4, 15, 21, 22, 50, 52, 66, 88, 89, 92, 94, 113, 123, 138, 139 | + | - | + | Positive |
| ESBL | TEM-8 | + | + S | + | Positive |
| ESBL | TEM-107, 134 | + | + H | + | Positive |
| ESBL | TEM-11, 27, 28, 29, 61, 75, 115, 118, 132, 147, 151, 152 | - | + H | - | Positive |
| ESBL | TEM-91, 143, 144 | - | + C | - | Positive |
| ESBL | TEM-5, 7, 10, 12, 53, 85, 86, 102, 114, 125, 136, 137, 155, 158 | - | + S | - | Positive |
| ESBL | TEM-19, 20, 25, 42, 47, 48, 49, 68, 71, 72, 93, 101, 112, 120 | - | - | + | Positive |
| **ESBL** | **TEM-126, 157¹, 164¹, 169¹** | - | - | - | **Negative** |
| Unknown | TEM-111, 124, 142 | + | - | - | Positive |
| Unknown | TEM-123 | + | - | + | Positive |
| Unknown | TEM-165 | - | + S | - | Positive |
| Unknown | TEM-116 | - | - | - | Positive |
| Unknown | TEM-70, 96, 104, 105, 108, 117, 146, 148, 150,161, 162, 164¹, 166, 169¹, 170-174 | - | - | - | Negative |
| TEM number reserved or withdrawn from Lahey database | TEM-14, 23, 41, 62, 69, 100, 119, 140, 153, 154, 156, 175 | Not applicable | | | |

| | | | | | |
|---|---|---|---|---|---|
| 1: ESBL phenotype according to Lahey database, but published evidence insufficient to determine phenotype (References: TEM-157; TEM-164 and TEM-169: no published data found in Pubmed or Embase). | | | | | |

**Table 1b: Phenotypes of SHV variants and corresponding amino acid substitutions that may be detected by the array system.**

| **Phenotype according to Lahey database** | **SHV Variants** | **ESBL Associated Amino Acid Substitutions at Positions Detected by Array*** | | | **Expected Array Result** |
|---|---|---|---|---|---|
| | | **D D179A/N/G** | **G 238 S/A** | **E 240 K** | |
| Non ESBL | SHV-1, 11, 14, 19, 25, 26, 32, 33, 43, 44, 48, 49, 56, 60-62, 71-83, 85, 89 | - | - | - | Negative |
| **Non ESBL** | **SHV-10¹** | - | **+ S** | **+ K** | **Positive** |
| Non ESBL | SHV-20², 21² | | + S | | Negative |
| Non ESBL | SHV-22² | | + S | + K | Negative |
| ESBL | SHV-6 | + A | - | - | Positive |
| ESBL | SHV-8 | + N | - | - | Positive |
| ESBL | SHV-24 | + G | - | - | Positive |
| ESBL | SHV-2, 2a, 3, 30, 34, 39, 86 | - | + S | - | Positive |
| ESBL | SHV-13, 102 | - | + A | - | Positive |
| ESBL | SHV-4, 5, 7, 9, 12, 15, 22², 45, 46, 55, 64, 66, 105 | - | + S | + K | Positive |
| ESBL | SHV-18 | - | + A | + K | Positive |
| ESBL | SHV-31, 115 | - | - | + K | Positive |
| ESBL | **SHV-16, 27³, 38⁴, 40³, 41³, 42³, 57, 70,** | - | - | - | **Negative** |
| Unknown | SHV-29 | - | + A | - | Positive |
| Unknown | SHV-106 | - | + S | - | Positive |
| Unknown | SHV-123, 124 | - | + S | + K | Positive |
| Unknown | SHV-97, 126 | - | - | + K | Positive |
| Unknown, detected as Non-ESBL. | SHV-28, 35-37, 50-53, 59, 63, 65, 67, 69, 92-96, 101, 103, 104, 107, 108, 110, 111, 113, 114, 116, 117, 121, 125, 127 | - | - | - | Negative |
| SHV number reserved or withdrawn | SHV-17, 23, 47, 54, 58, 68, 84, 87, 88, 90, 91, 98-100, 109, 112, 118-120, 122 | | | | |

| | | | | | |
|---|---|---|---|---|---|
| 1: The microarray system is designed to detect the S130G substitution in SHV-10, that reverses the extension of the beta-lactamase spectrum conferred by G238S and E240K. However, the software reports ESBL Positive if one or more of the ESBL associated substitutions is detected, even if the S130G substitution is detected as well. 2: Non-ESBL phenotype according the Lahey database, but ESBL phenotype according to literature including the reference from the Lahey database. 3: ESBL phenotype according to Lahey database, but published evidence contradictory (SHV-27; SHV-40; SHV 41; SHV 42). 4: Chromosomally encoded Class A carbapenemase. | | | | | |

**Supplementary Table 1: Nucleotide sequences of probes for ligation mediated amplification**

| | |
|---|---|
| **TEM-104E** | |
| **TEM-104-K** | |
| **TEM-164R** | |
| **TEM-164S** | |
| **TEM-164C** | |
| **TEM-164H** | |
| **TEM-84I** | |
| **TEM-100S** | |
| **TEM-238G** | |
| **TEM-238S** | |
| **SHV-130S** | |
| **SHV-238G** | |
| **SHV-238S** | |
| **SHV-238A** | |
| **SHV-240E** | |
| **SHV-240K** | |
| **SHV-179D** | |
| **SHV-179A** | |
| **SHV-179G** | |
| **SHV-179N** | |
| **CTX-M1** | **GATGTCACTGGCTGAGCTTAGCGC** GGCCGCGCTACAGTACAGCGATA |
| **CTX-M2** | **GGCGGTGCTTAAACAGAGCGAGA** GCGATAAGCACCTGCTAAATCAGCG |
| **CTX-M9** | **TGCGCCGCTGGTTCTGGTGAC** CTATTTTACCCAGCCGCAACAGAACG |
| **CTX-M8&25** | **GGGWGTGGCGTTGATTGACACC** GCCGATAACGCGCAGACGCTCTA |

**Table 2. Detection of ESBL associated substitutions by microarray in 106 ESBL positive isolates¹**

| **TEM ESBL genes in isolate collection:** | **Number of isolates** | **ESBL associated amino acid substitutions detected by array** | | | | |
|---|---|---|---|---|---|---|
| | | E104K | R164S | R164C | R164H | G238S |
| TEM-18 | 1 | + | - | | - | - |
| TEM-9, 63 | 3 | + | + | | - | - |
| TEM-6, 43 | 2 | + | - | | + | - |
| TEM-3, 4, 52 | 11 | + | - | | - | + |
| TEM-8 | 1 | + | + | | - | + |
| TEM-5, 7, 10, 12 | 6 | - | + | | - | - |
| TEM-19, 20, 72 | 5 | - | | | - | + |
| Individual probe sensitivity (# of positive isolates per probe / total # of isolates with the specific substitution) | | 18/18 (100%) | 6/10 (60%) | not tested | 2/2 (100%) | 16/17 (94%) |
| | | | | | | |

| **SHV ESBL Genes in Isolate Collection:** | **Number of isolates** | **ESBL associated amino acid substitutions detected by array** | | | | |
|---|---|---|---|---|---|---|
| | | D179A/N/G | | G238S | G238A | E240K |
| SHV-2, 2a, 3 | 11 | | | + | - | - |
| SHV-4, 5, 12 | 22 | | | + | - | + |
| SHV-18 | 1 | | | - | + | + |
| SHV-57 | 1 | | | - | - | - |
| Individual probe sensitivity (# of positive isolates per probe / total # of isolates with the specific substitution) | | not tested | | 32/33 (97%) | 1/1 (100%) | 23/23 (100%) |
| | | | | | | |

| **CTX-M Genes in the Isolate Collection** | **Number of isolates** | **CTX-M groups detected by Array** | | | | |
|---|---|---|---|---|---|---|
| | | CTX-M-1 | | CTX-M-2 | CTX-M-9 | CTX-M 8/25 |
| 1,3,10,15,28 | 21 | + | | - | - | - |
| 2,5 | 6 | - | | + | - | - |
| 9,14,16,27 | 16 | - | | - | + | - |
| 8,39 | 3 | - | | - | - | + |
| Individual probe sensitivity (# of positive isolates per probe / total # of isolates with the specific substitution) | | 21/21 (100%) | | 6/6 (100%) | 16/16 (100%) | 2/3 (67%) |

| | | | | | | |
|---|---|---|---|---|---|---|
| ¹: Four isolates contained 2 ESBL genes | | | | | | |

**Supplementary Table 2: Characteristics of the 213 test isolates (bacterial species, beta-lactamases, ESBL genotype, ESBL phenotype, and Array Result). Supplementary Table 2a: ESBL Positive isolates (n=106); Supplementary Table 2b: ESBL negative isolates (n=107).**

**Supplementary Table 2a: ESBL Positive test isolates (n=106)**

| **Isolate number** | **species** | **Beta-lactamase** | **Beta-lactamase type** | **ESBL genotype** | **ESBL phenotype** | **ESBL Array** |
|---|---|---|---|---|---|---|
| 1-3 | *E. coli* | CTX-M-1 | ESBL CTX-M-Family 1 | Pos | Pos | Pos |
| 4 | *E. coli* | CTX-M-10 | ESBL CTX-M-Family 1 | Pos | Pos | Pos |
| 5-9 | *E. coli* | CTX-M-15 | ESBL CTX-M-Family 1 | Pos | Pos | Pos |
| 10 | *E. coli* | CTX-M-3 | ESBL CTX-M-Family 1 | Pos | Pos | Pos |
| 11, 12 | *K. pneumoniae* | CTX-M-15 | ESBL CTX-M-Family 1 | Pos | Pos | Pos |
| 13 | *Salmonella species* | CTX-M-1 | ESBL CTX-M-Family 1 | Pos | Pos | Pos |
| 14 | *Salmonella species* | CTX-M-15 | ESBL CTX-M-Family 1 | Pos | Pos | Pos |
| 15 | *Salmonella species* | CTX-M-28 | ESBL CTX-M-Family 1 | Pos | Pos | Pos |
| 16 | *Salmonella species* | CTX-M-3 | ESBL CTX-M-Family 1 | Pos | Pos | Pos |
| 17 | *E. coli* | CTX-M-15, TEM-1 | ESBL CTX-M-Family 1, Non ESBL TEM | Pos | Pos | Pos |
| 18-20 | *E. coli* | CTX-M-2 | ESBL CTX-M-Family 2 | Pos | Pos | Pos |
| 21 | *P. mirabilis* | CTX-M-2 | ESBL CTX-M-Family 2 | Pos | Pos | Pos |
| 22 | *Salmonella species* | CTX-M-5 | ESBL CTX-M-Family 2 | Pos | Pos | Pos |
| 23 | *Salmonella species* | CTX-M-2, SHV-2 | ESBL CTX-M-Family 2, ESBL SHV | Pos | Pos | Pos |
| 24 | *P. mirabilis* | CTX-M-39 | ESBL CTX-M-Family 25 | Pos | Pos | **Neg** |
| 25 | *Salmonella species* | CTX-M-3 | ESBL CTX-M-Family 3 | Pos | Pos | Pos |
| 26 | *E. coli* | CTX-M-8 | ESBL CTX-M-Family 8 | Pos | Pos | Pos |
| 27 | *Salmonella species* | CTX-M-8 | ESBL CTX-M-Family 8 | Pos | Pos | Pos |
| 28 | *E. cloacae* | CTX-M-9 | ESBL CTX-M-Family 9 | Pos | **Non det** | Pos |
| 29, 30 | *E. cloacae* | CTX-M-9 | ESBL CTX-M-Family 9 | Pos | Pos | Pos |
| 31 - 33 | *E. coli* | CTX-M-14 | ESBL CTX-M-Family 9 | Pos | Pos | Pos |
| 34, 35 | *E. coli* | CTX-M-16 | ESBL CTX-M-Family 9 | Pos | Pos | Pos |
| 36 | *E. coli* | CTX-M-27 | ESBL CTX-M-Family 9 | Pos | Pos | Pos |
| 37-40 | *E. coli* | CTX-M-9 | ESBL CTX-M-Family 9 | Pos | Pos | Pos |
| 41 | *Salmonella species* | CTX-M-14 | ESBL CTX-M-Family 9 | Pos | Pos | Pos |
| 42 | *Salmonella species* | CTX-M-9 | ESBL CTX-M-Family 9 | Pos | Pos | Pos |
| 43 | C. *freundii* | SHV-2a | ESBL SHV | Pos | Pos | Pos |
| 44-48 | *E. cloacae* | SHV-12 | ESBL SHV | Pos | Pos | Pos |
| 49 | *E. cloacae* | SHV-2 | ESBL SHV | Pos | Pos | Pos |
| 50 | *E. coli* | SHV 5a, SHV-12 | ESBL SHV | Pos | Pos | Pos |
| 51 -53 | *E. coli* | SHV-12 | ESBL SHV | Pos | Pos | Pos |
| 54 | *E. coli* | SHV-3 | ESBL SHV | Pos | Pos | Pos |
| 55, 56 | *E. coli* | SHV-4 | ESBL SHV | Pos | Pos | Pos |
| 57-59 | *E. coli* | SHV-5 | ESBL SHV | Pos | Pos | Pos |
| 60 | *E. coli* | SHV-57 | ESBL SHV | Pos | Pos | **Neg** |
| 61 -63 | *K. pneumoniae* | SHV-12 | ESBL SHV | Pos | Pos | Pos |
| 64 | *K. pneumoniae* | SHV-18 | ESBL SHV | Pos | Pos | Pos |
| 65, 66 | *K. pneumoniae* | SHV-2 | ESBL SHV | Pos | Pos | Pos |
| 67, 68 | *K. pneumoniae* | SHV-5 | ESBL SHV | Pos | Pos | Pos |
| 69, 70 | *Salmonella species* | SHV-2 | ESBL SHV | Pos | Pos | Pos |
| 71 | *Salmonella species* | SHV-4 | ESBL SHV | Pos | Pos | Pos |
| 72 | *K. pneumoniae* | CTX-M-15, SHV-5 | ESBL SHV, CTX-M-Family 1 | Pos | Pos | Pos |
| 73, 74 | *K. pneumoniae* | SHV-12, CTX-M-1 | ESBL SHV, CTX-M-Family 1 | Pos | Pos | Pos |
| 75 | *K. oxytoca* | SHV-5, TEM-1 | ESBL SHV, Non ESBL TEM | Pos | Pos | Pos |
| 76 | C. *freundii* | TEM-52 | ESBL TEM | Pos | Pos | Pos |
| 77 | *E. cloacae* | TEM-19 | ESBL TEM | Pos | Pos | Pos |
| 78 | *E. coli* | TEM-10 | ESBL TEM | Pos | Pos | Pos |
| 79 | *E. coli* | TEM-12 | ESBL TEM | Pos | Pos | Pos |
| 80, 81 | *E. coli* | TEM-3 | ESBL TEM | Pos | Pos | Pos |
| 82, 83 | *E. coli* | TEM-4 | ESBL TEM | Pos | Pos | Pos |
| 84 | *E. coli* | TEM-5 | ESBL TEM | Pos | Pos | Neg |
| 85 | *E. coli* | TEM-52 | ESBL TEM | Pos | Pos | Pos |
| 86 | *E. coli* | TEM-52 | ESBL TEM | Pos | Pos | Pos |
| 87 | *E. coli* | TEM-6 | ESBL TEM | Pos | Pos | Pos |
| 88 | *E. coli* | TEM-7 | ESBL TEM | Pos | Pos | **Neg** |
| 89 | *E. coli* | TEM-7 | ESBL TEM | Pos | Pos | Pos |
| 90 | *E. coli* | TEM-7 | ESBL TEM | Pos | Pos | Pos |
| 91 | *E. coli* | TEM-8 | ESBL TEM | Pos | Pos | Pos |
| 92, 93 | *E. coli* | TEM-9 | ESBL TEM | Pos | Pos | Pos |
| 94 | *K. pneumoniae* | TEM-18 | ESBL TEM | Pos | Pos | Pos |
| 95 | *K. pneumoniae* | TEM-19 | ESBL TEM | Pos | Pos | Pos |
| 96 | *K. pneumoniae* | TEM-3 | ESBL TEM | Pos | Pos | Pos |
| 97 | *P. mirabilis* | TEM-43 | ESBL TEM | Pos | Pos | Pos |
| 98 | *P. mirabilis* | TEM-52 | ESBL TEM | Pos | Pos | Pos |
| 99 | *P. mirabilis* | TEM-72 | ESBL TEM | Pos | Pos | **Neg** |
| 100, 101 | *Salmonella species* | TEM-20 | ESBL TEM | Pos | Pos | Pos |
| 102, 103 | *Salmonella species* | TEM-52 | ESBL TEM | Pos | Pos | Pos |
| 104 | *Salmonella species* | TEM-63 | ESBL TEM | Pos | Pos | Pos |
| 105 | *E. coli* | CMY-10, CTX-M-9 | Plasmid mediated ampC, ESBL CTX-M-Family 9 | Pos | Pos | Pos |
| 106 | *E. coli* | ACC-1, SHV-2a | Plasmid mediated AmpC, ESBL SHV | Pos | **Neg** | Pos |

| | | | | | | |
|---|---|---|---|---|---|---|
| Pos = Positive; neg = negative; Non det = non determinable | | | | | | |

**Supplementary Table 2b: ESBL negative isolates (n=106)**

| **Isolate number** | **species** | **Beta-lactamase** | **Beta-lactamase type** | **ESBL genotype** | **ESBL phenotype** | **ESBL Array** |
|---|---|---|---|---|---|---|
| 107 | C. *werkmanii* | Chromosomal AmpC | Chromosomal AmpC | Neg | Neg | Neg |
| 108 | *E. cloacae* | Chromosomal AmpC | Chromosomal AmpC | Neg | Neg | Neg |
| 109-113 | *E. coli* | Chromosomal AmpC | Chromosomal AmpC | Neg | Neg | Neg |
| 114-120 | *K. oxytoca* | K1 hyperproducer | K1 | Neg | Pos | Neg |
| 121 | *E. coli* | No TEM, SHV or CTX-M genes | No TEM, SHV or CTX-M genes | Neg | Pos | Neg |
| 122-170 | *E. coli* | No TEM, SHV or CTX-M genes | No TEM, SHV or CTX-M genes | Neg | neg | Neg |
| 171, 172 | *P. mirabilis* | No TEM, SHV or CTX-M genes | No TEM, SHV or CTX-M genes | Neg | Neg | Neg |
| 173, 174 | *E. coli* | SHV-1 | Non ESBL SHV | Neg | Neg | Neg |
| 175 | *K. oxytoca* | SHV-11 | Non ESBL SHV | Neg | Neg | Neg |
| 176 | *E. cloacae* | TEM-1 | Non ESBL TEM | Neg | Neg | Neg |
| 177-184 | *E. coli* | Tem-1 | Non ESBL TEM | Neg | Neg | Neg |
| 185-187 | *E. coli* | TEM-2 | Non ESBL TEM | Neg | Neg | Neg |
| 188, 189 | *Salmonella species* | TEM-1b | Non ESBL TEM | Neg | Neg | Neg |
| 190 | *E. coli* | OXA-2 | OXA | Neg | Neg | Neg |
| 191 | *E. coli* | OXA-23 | OXA | Neg | Neg | Neg |
| 192 | *E. coli* | OXA-30 | OXA | Neg | Neg | Neg |
| 193 | *E. coli* | OXA-5 | OXA | Neg | Neg | Neg |
| 194 | *E. coli* | OXA-7 | OXA | Neg | Neg | Neg |
| 195 | *E. coli* | OXA-9 | OXA | Neg | Neg | Neg |
| 196, 197 | *E. coli* | ACC-1 | plasmid mediated AmpC | Neg | Neg | Neg |
| 198, 199 | *E. coli* | ACT-1 | plasmid mediated AmpC | Neg | Neg | Neg |
| 200 | *E. coli* | CMY-1 | plasmid mediated AmpC | Neg | Neg | Neg |
| 201, 202 | *E. coli* | CMY-2 | plasmid mediated AmpC | Neg | Neg | Neg |
| 203, 204 | *E. coli* | DHA-1 | plasmid mediated AmpC | Neg | Neg | Neg |
| 205 | *E. coli* | MIR-1 | plasmid mediated AmpC | Neg | Neg | Neg |
| 206 | *E. coli* | MOX-1 | plasmid mediated AmpC | Neg | Neg | Neg |
| 207, 208 | *Salmonella species* | ACC-1 | plasmid mediated AmpC | Neg | Neg | Neg |
| 209 | *Salmonella species* | CMY-18 | plasmid mediated AmpC | Neg | Neg | Neg |
| 210-212 | *Salmonella species* | CMY-2 | plasmid mediated AmpC | Neg | Neg | Neg |

**Table 3: Primers for PCR and sequencing of TEM, SHV and CTX-M genes.**

| Gene | Primer | Sequence | Reference |
|---|---|---|---|
| TEM | TEM-F | 5'-GCGGAACCCCTATTTG-3' | (27) |
| TEM | TEM-R | 5'-ACCAATGCTTAATCAGTGAG-3' | (27) |
| SHV | SHV-F1 | 5'-CTTTACTCGCCTTTATCG-3' | (26) |
| SHV | SHV-R1 | 5'-TCCCGCAGATAAATCACCA-3' | (26) |
| CTX-M | CTX-M-F | 5'-ATGTGCAGYACCAGTAARGTKATGGC-3' | (24) |
| CTX-M | CTX-M-R | 5'-TGGGTRAARTARGTSACCAGAAYSAGCGG-3' | (24) |
| CTX-M1 group | CTX-M-10-1F | 5'-ATGGTTAAAAAATCACTGCG-3' | (28) |
| CTX-M1 group | CTX-M-10-4R | 5'-AAACCGTTGGTGACGAT-3' | (28) |
| CTX-M2 group | CTX-M-2F | 5'-ATGATGACTCAGAGCATTCG-3' | (35) |
| CTX-M2 group | CTX-M-2R | 5'-TTATTGCATCAGAAACCGTG-3' | (35) |
| CTX-M8 group | CTX-Mgp8-F | 5'-TGATGAGACATCGCGTTAAG-3' | (14) |
| CTX-M8 group | CTX-Mgp8-R | 5'-TAACCGTCGGTGACGATTTT-3' | (14) |
| CTX-M9 group | CTX-M-9-1F | 5'-TGGTGACAAAGAGAGTGCAACG-3' | (28) |
| CTX-M9 group | CTX-M-9-MF | 5'-GGAGGCGTGACGGCT TTT-3' | (28) |

**Table 4**

| **SEQ. ID.** | **GenBank accession number** | **Sequence** | **Position** | **Remarks** |
|---|---|---|---|---|
| TEM wild type 104 | GU734697 | | 201-300 | |
| TEM wild type 164 | GU734697 | | 381-480 | |
| TEM wild type 238 | GU595196 | | 28706-28805 | reverse |
| SHV wild type 238/240 | AB551737 | | 650-749 | reverse |
| OXA 48 I | AY236073 | | 2168-2267 | |
| OXA 48 II | AY236073 | | 2867-2966 | |
| VIM 552 C | GU724871 | | 1488-1587 | reverse |
| VIM 246 | GU731077 | | 982-1081 | |
| IMP 114 | GU831553 | | 64-163 | reverse |
| IMP 4801/II | GQ302617 | | 518-617 | |
| SHV all | HM048880 | | 222-321 | |
| NDM-1 75 | FN396876 | | 2412-2511 | |
| NDM-1 392 | FN396876 | | 2749-2848 | |
| CMY II 304 | GU393330 | | 1-100 | |
| CMY II 1199 | FJ437066 | | 944-1043 | |
| CMY I MOX | AF381626 | | 384-483 | |
| ACC 660 | EF554600 | | 652-751 | |
| MIR ACT | DQ986958 | | 411-510 | |
| DHA 753 | EF406115 | | 704-803 | |
| DHA 1110 | EF406115 | | 1023-1122 | |
| TEM all | GU734697 | | 502-601 | |
| FOX | DQ478715 | | | |
| KPC I | GQ229417 | | 38-137 | |
| KPC II | GQ229417 | | | |
| CTX-M 8 +25 | AB543595 | | 1944-2043 | |
| CTX-M9 +64 | AB545872 | | 268-367 | Reverse |
| CTX-M2 | FJ973571 | | 729-828 | |
| CTX-M 1 | HM002660 | | 373-472 | |

**Table 5:**

| **Species** | **ESBL_A** | | | | | | |
|---|---|---|---|---|---|---|---|
| | TEM | SHV | CTX-M1 | CTX-M2 | CTX-M9 | **total** | |
| E.coli | 5 | 5 | 14 | 0 | 0 | **24** | |
| K.pneumoniae | 4 | 13 | 12 | 9 | 3 | **41** | |
| | | | | | | | |

| **Species** | **ESBL_C** | | | | | | |
|---|---|---|---|---|---|---|---|
| | CMY-2 | DHA | ACC | MIR/ACT | FOX | CMY-1/MOX | **total** |
| E.coli | 50 | 1 | 1 | 0 | 2 | 2 | **56** |
| K.pneumoniae | 12 | 8 | 0 | 3 | 3 | 1 | **27** |
| | | | | | | | |

| **Species** | **ESBL_CARBA** | | | | | | |
|---|---|---|---|---|---|---|---|
| | KPC | NDM | **total** | | | | |
| E.coli | 3 | 2 | **5** | | | | |
| K.pneumoniae | 12 | 13 | **25** | | | | |

## Claims

1. A method for determining the presence of extended spectrum beta-lactamases (ESBL) nucleic acids in a sample, comprising the steps of:
(a) extracting nucleic acids from micro-organisms, said nucleic acids comprising target ESBL nucleic acids,
(b) performing a ligase detection reaction (LDR) on said target ESBL nucleic acids, comprising:
(b1) contacting a plurality of different probe pairs with said target ESBL nucleic acid(s),
wherein each probe pair comprises a first nucleic acid probe and a second nucleic acid probe,
wherein each probe pair being specific for a particular target ESBL nucleic acid,
wherein for each probe pair, the first nucleic acid probe and the second nucleic acid probe are configured to be essentially adjacent when hybridized with the particular target ESBL nucleic acid;
(b2) incubating said target ESBL nucleic acid with said plurality of different probe pairs under conditions allowing to hybridize said particular target ESBL nucleic acid with at least one probe pair,
(b3) ligating any essentially adjacent identifiable first nucleic acid probe and identifiable second nucleic acid probe of a probe pair, to form a connected probe assembly;
(b4) providing at least a set of two amplification primers configured to amplify the connected probe assembly of step (b3),
(b5) amplifying the connected probe assembly of step (b3) using the amplification primers of step (b4), thereby providing amplified target ESBL nucleic acids [ESBL amplicon],
(c) detecting the amplified target ESBL nucleic acids [ESBL amplicon] by performing Real Time (RT) detection and a hybridization to a capture probe, wherein the hybridization to the capture probe is performed when the RT detection determines that ESBL nucleic acid(s) is present, whereby the presence of an ESBL nucleic acid in a sample is determined.

2. The method according to claim 1, wherein said ligation step (b3) comprises the use of a ligase.

3. The method according to claim 1 or 2, wherein the amplified target ESBL nucleic acids [ESBL amplicon] are labeled, preferably during amplification, more preferably by providing in step (b4) at least a set of two amplification primers wherein at least one primer is labelled.

4. The method according to any one claims 1 to 3, wherein said LDR comprises at least 4, preferably 5, 6, 7, 8, 9, 10 or even more different probe pairs.

5. The method according to any one claims 1 to 4, wherein said particular target ESBL nucleic acid is chosen from the group consisting of TEM, SHV and CTX.

6. The method according to any one of claims 1 to 5, wherein said particular target ESBL nucleic acid is chosen from the group consisting of the nucleic acids **characterized by** sequence id of Table 4, including TEM wild type 104, TEM wild type 164, TEM wild type 238, TEM wild type 238/240, OXA 48 I, OXA 48 II, VIM 552C, VIM 246, IMP 114, IMP 480 I/II, SHV all, NDM-1 75, NDM-1 392, CMY II 304, CMY II 1199, CMY I MOX, ACC 606, MIR ACT, DHA 753, DHA 1110, TEM all, FOX, KPC I, KPC II, CTX-M8 + 25, CTX-M9 + 64, CTX-M2, and CTX-M1, preferably TEM-E104K, TEM-R164S, TEM-R164C, TEM-R164H, TEM-G238S, SHV-G238S, SHV-G238A, SHV-E240K, SHV-D179A, SHV-D179G, and SHV-D179N.

7. The method according to any one of claims 1 to 6, wherein said different probe pairs are chosen from the group consisting of TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N and CTX-M1, CTX-M2, CTX-M9, and CTX-M8/25.

8. The method according to any of the claims 1 to 7, wherein a probe pair being specific for a particular target ESBL nucleic acid comprises an identifier region.

9. The method according to claim 8, wherein said identifier region is specific for TEM, SHV or CTX.

10. The method according to claim 8 or 9, wherein said identifier region is specific for a target ESBL nucleic acid.

11. The method according to any one of claims 8 to 10, wherein said identifier region is specific for a particular target ESBL nucleic acid chosen from the group consisting of TEM-E104K, TEM-R164S, TEM-R164C, TEM-R164H, TEM-G238S, SHV-G238S, SHV-G238A, SHV-E240K, SHV-D179A, SHV-D179G, and SHV-D179N.

12. The method according to any one of claims 9 to 11, wherein said identifier region is specific for a probe pair chosen from the group consisting of TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N and CTX-M1, CTX-M2, CTX-M9, and CTX-M8/25.

13. The method according to any one of claims 9 to 12, wherein said identifier region hybridizes to said capture probe.

14. The method according to any of the claims 9 to 13, wherein said capture probe comprises a region ZIP, which is essentially complementary to a corresponding identifier region cZIP on said connected probe assembly, or wherein said capture probe comprises a region cZIP which is essentially complementary to a corresponding identifier region ZIP on said connected probe assembly.

15. The method according to any one of the claims 1 to 14, wherein said capture probe is spatially addressable on a microarray.

16. The method according to any one of claims 1 to 15, wherein the amplified target ESBL nucleic acids [ESBL amplicon] derived from at least two samples are hybridised to capture probes present on a single microarray.

17. The method according to any one of claims 15 or 16, wherein the amplified target ESBL nucleic acids [ESBL amplicon] hybridised to the corresponding capture probes on a microarray results in a hybridisation pattern.

18. The method according to any one of claims 1 to 17, wherein said Real Time (RT) detection comprises:
(i) providing at least one or more detector molecules, wherein said detector molecules detects the amplified target ESBL nucleic acids [ESBL amplicon];
(ii) monitoring the signal and/or the modulation of the signal of said detector molecule, thereby detecting the presence of said amplified target ESBL nucleic acids [ESBL amplicon].

19. The method according to claim 18, wherein said detector molecules are one or more oligonucleotide detector probes having a sequence at least partially complementary to said amplified target ESBL nucleic acids [ESBL amplicon] and including a fluorescent reporter molecule and a fluorescent quencher molecule capable of quenching the fluorescence of said reporter molecule, said oligonucleotide detector probe existing in at least one single-stranded, partially single-stranded or double-stranded conformation when unhybridized where said quencher molecule quenches the fluorescence of said reporter molecule, said oligonucleotide detector probe existing in at least one conformation when hybridized to said target nucleic acid where the fluorescence of said reporter molecule is unquenched.

20. The method according to claim 19, wherein the sequence of said oligonucleotide detector probes is at least complementary to at least one region of the first nucleic acid probe or at least one region of the second nucleic acid probe.

21. The method according to any of the claims 1 to 20, wherein the first nucleic acid probe and/or the second nucleic acid probe comprises at least one region (DET), which is
(i) essentially complementary to one or more oligonucleotide detector probes; and
(ii) essentially non-complementary to said target ESBL nucleic acid.

22. The method according to any of claims 14 to 21, wherein a cZIP or a ZIP on a connected probe assembly functions as a DET region.

## Patentansprüche

1. Verfahren zur Bestimmung des Vorliegens von ESBL(Extended Spectrum Beta-Lactamases)-Nukleinsäuren in einer Probe, bei dem man in den Schritten:
(a) Nukleinsäuren aus Mikroorganismen extrahiert, wobei die Nukleinsäuren Ziel-ESBL-Nukleinsäuren umfassen,
(b) die Ziel-ESBL-Nukleinsäuren einer Ligase-Nachweisreaktion (LDR) unterzieht, bei der man:
(b1) mehrere unterschiedliche Sondenpaare mit der bzw. den Ziel-ESBL-Nukleinsäure(n) in Kontakt bringt,
wobei die Sondenpaare jeweils eine erste Nukleinsäuresonde und eine zweite Nukleinsäuresonde umfassen,
wobei jedes Sondenpaar für eine bestimmte Ziel-ESBL-Nukleinsäure spezifisch ist,
wobei bei jedem Sondenpaar die erste Nukleinsäuresonde und die zweite Nukleinsäuresonde so konfiguriert sind, dass sie bei Hybridisierung mit der bestimmten Ziel-ESBL-Nukleinsäure im Wesentlichen nebeneinander liegen;
(b2) die Ziel-ESBL-Nukleinsäure mit den mehreren unterschiedlichen Sondenpaaren unter Bedingungen inkubiert, die das Hybridisieren der bestimmten Ziel-ESBL-Nukleinsäure mit wenigstens einem Sondenpaar gestatten,
(b3) jede im Wesentlichen nebeneinander liegende identifizierbare erste Nukleinsäuresonde und identifizierbare zweite Nukleinsäuresonde eines Sondenpaars unter Bildung einer verbundenen Sondenanordnung ligiert;
(b4) wenigstens einen Satz von zwei Amplifikationsprimern mit einer Konfiguration zum Amplifizieren der verbundenen Sondenanordnung aus Schritt (b3) bereitstellt,
(b5) die verbundene Sondenanordnung aus Schritt (b3) unter Verwendung der Amplifikationsprimer aus Schritt (b4) amplifiziert, womit amplifizierte Ziel-ESBL-Nukleinsäuren [ESBL-Amplifikat] bereitgestellt werden,
(c) die amplifizierten Ziel-ESBL-Nukleinsäuren [ESBL-Amplifikat] nachweist, indem man einen RT(Real Time)-Nachweis und/oder eine Hybridisierung an eine Fängersonde durchführt, wobei die Hybridisierung an die Fängersonde durchgeführt wird, wenn durch den RT-Nachweis bestimmt wird, dass ESBL-Nukleinsäure(n) vorliegt bzw. vorliegen, wodurch das Vorliegen einer ESBL-Nukleinsäure in einer Probe bestimmt wird.

2. Verfahren nach Anspruch 1, wobei der Ligationsschritt (b3) die Verwendung einer Ligase umfasst.

3. Verfahren nach Anspruch 1 oder 2, wobei die amplifizierten Ziel-ESBL-Nukleinsäuren [ESBL-Amplifikat] markiert werden, bevorzugt während der Amplifikation, stärker bevorzugt indem in Schritt (b4) wenigstens ein Satz von zwei Amplifikationsprimern bereitgestellt wird, wobei wenigstens ein Primer markiert ist.

4. Verfahren nach einem der Ansprüche 1 bis 3, wobei die LDR wenigstens 4, vorzugsweise 5, 6, 7, 8, 9, 10 oder noch mehr unterschiedliche Sondenpaare umfasst.

5. Verfahren nach einem der Ansprüche 1 bis 4, wobei die bestimmte Ziel-ESBL-Nukleinsäure aus der aus TEM, SHV und CTX bestehenden Gruppe gewählt ist.

6. Verfahren nach einem der Ansprüche 1 bis 5, wobei die bestimmte Ziel-ESBL-Nukleinsäure aus der aus den durch Sequenz-Id in Tabelle 4 gekennzeichneten Nukleinsäuren, einschließlich TEM-Wildtyp 104, TEM-Wildtyp 164, TEM-Wildtyp 238, TEM-Wildtyp 238/240, OXA 48 I, OXA 48 II, VIM 552C, VIM 246, IMP 114, IMP 480 I/II, SHV alle, NDM-1 75, NDM-1 392, CMY II 304, CMY II 1199, CMY I MOX, ACC 606, MIR ACT, DHA 753, DHA 1110, TEM alle, FOX, KPC I, KPC II, CTX-M8 + 25, CTX-M9 + 64, CTX-M2 und CTX-M1, vorzugsweise TEM-E104K, TEM-R164S, TEM-R164C, TEM-R164H, TEM-G238S, SHV-G238S, SHV-G238A, SHV-E240K, SHV-D179A, SHV-D179G und SHV-D179N, bestehenden Gruppe gewählt ist.

7. Verfahren nach einem der Ansprüche 1 bis 6, wobei die unterschiedlichen Sondenpaare aus der aus TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N und CTX-M1, CTX-M2, CTX-M9 und CTX-M8/25 bestehenden Gruppe gewählt sind.

8. Verfahren nach einem der Ansprüche 1 bis 7, wobei ein für eine bestimmte Ziel-ESBL-Nukleinsäure spezifisches Sondenpaar einen Identifikatorbereich umfasst.

9. Verfahren nach Anspruch 8, wobei der Identifikatorbereich für TEM, SHV oder CTX spezifisch ist.

10. Verfahren nach Anspruch 8 oder 9, wobei der Identifikatorbereich für eine Ziel-ESBL-Nukleinsäure spezifisch ist.

11. Verfahren nach einem der Ansprüche 8 bis 10, wobei der Identifikatorbereich für eine aus der aus TEM-E104K, TEM-R164S, TEM-R164C, TEM-R164H, TEM-G238S, SHV-G238S, SHV-G238A, SHV-E240K, SHV-D179A, SHV-D179G und SHV-D179N bestehenden Gruppe gewählte bestimmte Ziel-ESBL-Nukleinsäure spezifisch ist.

12. Verfahren nach einem der Ansprüche 9 bis 11, wobei der Identifikatorbereich für ein aus der aus TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N und CTX-M1, CTX-M2, CTX-M9 und CTX-M8/25 bestehenden Gruppe gewähltes Sondenpaar spezifisch ist.

13. Verfahren nach einem der Ansprüche 9 bis 12, wobei der Identifikatorbereich an die Fängersonde hybridisiert.

14. Verfahren nach einem der Ansprüche 9 bis 13, wobei die Fängersonde einen Bereich ZIP umfasst, der im Wesentlichen zu einem entsprechenden Identifikatorbereich cZIP auf der verbundenen Sondenanordnung komplementär ist, oder wobei die Fängersonde einen Bereich cZIP umfasst, der im Wesentlichen zu einem entsprechenden Identifikatorbereich ZIP auf der verbundenen Sondenanordnung komplementär ist.

15. Verfahren nach einem der Ansprüche 1 bis 14, wobei die Fängersonde auf einem Mikroarray räumlich adressierbar ist.

16. Verfahren nach einem der Ansprüche 1 bis 15, wobei die aus wenigstens zwei Proben stammenden, amplifizierten Ziel-ESBL-Nukleinsäuren [ESBL-Amplifikat] an auf einem einzelnen Mikroarray vorliegende Fängersonden hybridisiert werden.

17. Verfahren nach einem der Ansprüche 15 oder 16, wobei die an die entsprechenden Fängersonden auf einem Mikroarray hybridisierten amplifizierten Ziel-ESBL-Nukleinsäuren [ESBL-Amplifikat] ein Hybridisierungsmuster ergeben.

18. Verfahren nach einem der Ansprüche 1 bis 17, wobei man bei dem RT(Real Time)-Nachweis:
(i) wenigstens ein oder mehrere Detektormoleküle bereitstellt, wobei durch die Detektormoleküle die amplifizierten Ziel-ESBL-Nukleinsäuren [ESBL-Amplifikat] nachgewiesen werden;
(ii) das Signal und/oder die Modulation des Signals des Detektormoleküls beobachtet, womit das Vorliegen der amplifizierten Ziel-ESBL-Nukleinsäuren [ESBL-Amplifikat] nachgewiesen wird.

19. Verfahren nach Anspruch 18, wobei es sich bei den Detektormolekülen um eine oder mehrere Oligonukleotid-Detektorsonden, die eine wenigstens teilweise zu den amplifizierten Ziel-ESBL-Nukleinsäuren [ESBL-Amplifikat] komplementäre Sequenz aufweisen und ein Fluoreszenz-Reportermolekül und ein Fluoreszenz-Quenchermolekül, das zum Löschen der Fluoreszenz des Reportermoleküls fähig ist, enthalten, handelt, wobei die Oligonukleotid-Detektorsonde in wenigstens einer einzelsträngigen, teilweise einzelsträngigen oder doppelsträngigen Konformation existiert, wenn sie nicht hybridisiert ist, wobei das Quenchermolekül die Fluoreszenz des Reportermoleküls löscht, wobei die Oligonukleotid-Detektorsonde in wenigstens einer Konformation existiert, wenn sie an die Zielnukleinsäure hybridisiert ist, wobei die Fluoreszenz des Reportermoleküls nicht gelöscht wird.

20. Verfahren nach Anspruch 19, wobei die Sequenz der Oligonukleotid-Detektorsonden wenigstens zu wenigstens einem Bereich der ersten Nukleinsäuresonde oder wenigstens einem Bereich der zweiten Nukleinsäuresonde komplementär ist.

21. Verfahren nach einem der Ansprüche 1 bis 20, wobei die erste Nukleinsäuresonde und/oder die zweite Nukleinsäuresonde wenigstens einen Bereich (DET) umfasst, der:
(i) zu einer oder mehreren Oligonukleotid-Detektorsonden im Wesentlichen komplementär ist; und
(ii) zu der Ziel-ESBL-Nukleinsäure im Wesentlichen nichtkomplementär ist.

22. Verfahren nach einem der Ansprüche 14 bis 21, wobei ein cZIP oder ein ZIP auf einer verbundenen Sondenanordnung als ein DET-Bereich fungiert.

## Revendications

1. Procédé de détermination de la présence d'acides nucléiques de bêta-lactamases à large spectre (ESBL) dans un échantillon, comprenant les étapes consistant à :
(a) extraire des acides nucléiques à partir de microorganismes, lesdits acides nucléiques comprenant des acides nucléiques d'ESBL cibles,
(b) exécuter une réaction de détection par ligase (RDL) sur lesdits acides nucléiques d'ESBL cibles, comprenant les étapes consistant à :
(b1) mettre en contact une pluralité de paires de sondes différentes avec ledit (lesdits) acide(s) nucléique(s) d'ESBL cible(s),
dans laquelle chaque paire de sondes comprend une première sonde d'acide nucléique et une deuxième sonde d'acide nucléique,
dans laquelle chaque paire de sondes est spécifique d'un acide nucléique d'ESBL cible particulier,
dans laquelle pour chaque paire de sondes, la première sonde d'acide nucléique et la deuxième sonde d'acide nucléique sont configurées pour être essentiellement adjacentes quand elles s'hybrident avec l'acide nucléique d'ESBL cible particulier ;
(b2) incuber ledit acide nucléique d'ESBL cible avec ladite pluralité de paires de sondes différentes, dans des conditions permettant d'obtenir une hybridation dudit acide nucléique d'ESBL cible particulier avec au moins une paire de sondes,
(b3) ligaturer une première sonde d'acide nucléique identifiable et une deuxième sonde d'acide nucléique identifiable quelconques, étant essentiellement adjacentes, d'une paire de sondes pour former un assemblage de sondes reliées ;
(b4) fournir au moins un ensemble de deux amorces d'amplification étant configurées pour amplifier l'assemblage de sondes reliées de l'étape (b3),
(b5) amplifier l'assemblage de sondes reliées de l'étape (b3) en utilisant les amorces d'amplification de l'étape (b4), en fournissant de ce fait des acides nucléiques d'ESBL cibles amplifiés [amplicon d'ESBL],
(c) détecter les acides nucléiques d'ESBL cibles amplifiés [amplicon d'ESBL] en effectuant une détection en temps réel (TR) et une hybridation avec une sonde de capture, dans laquelle l'hybridation avec la sonde de capture est exécutée quand la détection en TR détermine qu'un (des) acide(s) nucléique(s) d'ESBL est (sont) présent(s), moyennant quoi la présence d'un acide nucléique d'ESBL est déterminée dans un échantillon.

2. Procédé selon la revendication 1, dans laquelle ladite étape de ligature (b3) comprend l'utilisation d'une ligase.

3. Procédé selon la revendication 1 ou la 2, dans laquelle les acides nucléiques d'ESBL cibles amplifiés [amplicon d'ESBL] sont marqués, de préférence lors de l'amplification, mieux préféré en fournissant dans l'étape (b4) au moins un ensemble de deux amorces d'amplification, dans lequel au moins une amorce est marquée.

4. Procédé selon l'une quelconque des revendications 1 à 3, dans laquelle ladite RDL comprend au moins 4, de préférence 5, 6, 7, 8, 9, 10 paires de sondes différentes ou même plus.

5. Procédé selon l'une quelconque des revendications 1 à 4, dans laquelle ledit acide nucléique d'ESBL cible particulier est choisi dans le groupe constitué par les TEM, SHV et CTX.

6. Procédé selon l'une quelconque des revendications 1 à 5, dans laquelle ledit acide nucléique d'ESBL cible particulier est choisi dans le groupe constitué par les acides nucléiques **caractérisés par** l'id. de séquence du Tableau 4, y compris les TEM de type sauvage 104, TEM de type sauvage 164, TEM de type sauvage 238, TEM de type sauvage 238/240, OXA 48 I, OXA 48 II, VIM 552C, VIM 246, IMP 114, IMP 480 I/II, SHV all, NDM-1 75, NDM-1 392, CMY II 304, CMY II 1199, CMY I MOX, ACC 606, MIR ACT, DHA 753, DHA 1110, TEM all, FOX, KPC I, KPC II, CTX-M8 + 25, CTX-M9 + 64, CTX-M2 et CTX-M1, de préférence les TEM-E104K, TEM-R164S, TEM-R164C, TEM-R164H, TEM-G238S, SHV-G238S, SHV-G238A, SHV-E240K, SHV-D179A, SHV-D179G et SHV- D179N.

7. Procédé selon l'une quelconque des revendications 1 à 6, dans laquelle lesdites paires de sondes différentes sont choisies dans le groupe constitué par les TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N ainsi que les CTX-M1, CTX-M2, CTX-M9 et CTX-M8/25.

8. Procédé selon l'une quelconque des revendications 1 à 7, dans laquelle une paire de sondes étant spécifique d'un acide nucléique d'ESBL cible particulier comprend une région d'identification.

9. Procédé selon la revendication 8, dans laquelle ladite région d'identification est spécifique des TEM, SHV ou CTX.

10. Procédé selon la revendication 8 ou la 9, dans laquelle ladite région d'identification est spécifique d'un acide nucléique d'ESBL cible.

11. Procédé selon l'une quelconque des revendications 8 à 10, dans laquelle ladite région d'identification est spécifique d'un acide nucléique d'ESBL cible particulier, choisi dans le groupe constitué par les TEM-E104K, TEM-R164S, TEM-R164C, TEM-R164H, TEM-G238S, SHV-G238S, SHV-G238A, SHV-E240K, SHV-D179A, SHV-D179G et SHV- D179N.

12. Procédé selon l'une quelconque des revendications 9 à 11, dans laquelle ladite région d'identification est spécifique d'une paire de sondes, choisies dans le groupe constitué par les TEM-104E, TEM-104K, TEM-164R, TEM-164S, TEM-164C, TEM-164H, TEM-84I, TEM-100S, TEM-238G, TEM-238S, SHV-130S, SHV-238G, SHV-238S, SHV-238A, SHV-240E, SHV-240K, SHV-179D, SHV-179A, SHV-179G, SHV-179N ainsi que les CTX-M1, CTX-M2, CTX-M9 et CTX-M8/25.

13. Procédé selon l'une quelconque des revendications 9 à 12, dans laquelle ladite région d'identification s'hybride avec ladite sonde de capture.

14. Procédé selon l'une quelconque des revendications 9 à 13, dans laquelle ladite sonde de capture comprend une région ZIP, qui est essentiellement complémentaire à une région cZIP d'identification correspondante sur ledit assemblage de sondes reliées, ou dans laquelle ladite sonde de capture comprend une région cZIP, qui est essentiellement complémentaire à une région ZIP d'identification correspondante sur ledit assemblage de sondes reliées.

15. Procédé selon l'une quelconque des revendications 1 à 14, dans laquelle ladite sonde de capture est adressable, dans l'espace, sur un micro-arrangement.

16. Procédé selon l'une quelconque des revendications 1 à 15, dans laquelle les acides nucléiques d'ESBL cibles amplifiés [amplicon d'ESBL] dérivés d'au moins deux échantillons sont hybridés avec des sondes de capture présentes sur un seul micro-arrangement.

17. Procédé selon l'une quelconque des revendications 15 ou 16, dans laquelle les acides nucléiques d'ESBL cibles amplifiés [amplicon d'ESBL] hybridés avec les sondes de capture correspondantes sur un micro-arrangement conduisent à un modèle d'hybridation.

18. Procédé selon l'une quelconque des revendications 1 à 17, dans laquelle ladite détection en temps réel (TR) comprend les étapes consistant à :
(i) fournir au moins une ou plusieurs molécule(s) de détection, dans laquelle lesdites molécules de détection détectent les acides nucléiques d'ESBL cibles amplifiés [amplicon d'ESBL] ;
(ii) suivre le signal et/ou la modulation du signal de ladite molécule de détection, en détectant de ce fait la présence desdits acides nucléiques d'ESBL cibles amplifiés [amplicon d'ESBL].

19. Procédé selon la revendication 18, dans laquelle lesdites molécules de détection sont une ou plusieurs sonde(s) d'oligonucléotides de détection, ayant une séquence qui est au moins partiellement complémentaire auxdits acides nucléiques d'ESBL cibles amplifiés [amplicon d'ESBL] et comprenant une molécule fluorescente rapporteur ainsi qu'une molécule fluorescente d'extinction, qui est capable d'éteindre la fluorescence de ladite molécule rapporteur, ladite sonde d'oligonucléotides de détection existant dans au moins une conformation en simple brin, partiellement en simple brin ou en double brin, quand elle est n'est pas hybridée là où ladite molécule d'extinction éteint la fluorescence de ladite molécule rapporteur, ladite sonde d'oligonucléotides de détection existant dans au moins une conformation quand elle est hybridée avec ledit acide nucléique cible, là où la fluorescence de ladite molécule rapporteur n'est pas éteinte.

20. Procédé selon la revendication 19, dans laquelle la séquence desdites sondes d'oligonucléotides de détection est au moins complémentaire à au moins une région de la première sonde d'acide nucléique ou à au moins une région de la deuxième sonde d'acide nucléique.

21. Procédé selon l'une quelconque des revendications 1 à 20, dans laquelle la première sonde d'acide nucléique et/ou la deuxième sonde d'acide nucléique comprennent au moins une région (DET), qui est
(i) essentiellement complémentaire à une ou plusieurs sonde(s) d'oligonucléotides de détection ;
et
(ii) essentiellement non complémentaire au dit acide nucléique d'ESBL cible.

22. Procédé selon l'une quelconque des revendications 14 à 21, dans laquelle un cZIP ou un ZIP sur un assemblage de sondes reliées fonctionne comme une région DET.


### SEQUENCE LISTING

<110> CHECK-POINTS HOLDING B.V.
<120> ASSAYS, COMPOSITIONS AND METHODS FOR DETECTING DRUG RESISTANT MICRO-ORGANISMS
<130> CHE-006-PCT
<150> PCT/EP2010/056122
   <151> 2010-05-05
<150> PCT/EP2010/059105
   <151> 2010-06-25
<160> 85
<170> PatentIn version 3.5
<210> 1
   <211> 27
   <212> DNA
   <213> artificial sequence
<220>
   <223> Primer: TEM-104E
<400> 1
   tacactattc tcagaatgac ttggttg 27
<210> 2
   <211> 27
   <212> DNA
   <213> artificial sequence
<220>
   <223> Primer: TEM-104E
<400> 2
   agtactcacc agtcacagaa aagcatc 27
<210> 3
   <211> 27
   <212> DNA
   <213> artificial sequence
<220>
   <223> Primer: TEM-104K
<400> 3
   tacactattc tcagaatgac ttggtta 27
<210> 4
   <211> 23
   <212> DNA
   <213> artificial sequence
<220>
   <223> Primer: TEM-164R
<400> 4
   ggatcatgta actcgccttg atc 23
<210> 5
   <211> 24
   <212> DNA
   <213> artificial sequence
<220>
   <223> Primer: TEM-164R
<400> 5
   gttgggaacc ggagctgaat gaag 24
<210> 6
   <211> 23
   <212> DNA
   <213> artificial sequence
<220>
   <223> Primer: TEM-164S
<400> 6
   ggatcatgta actcgccttg ata 23
<210> 7
   <211> 23
   <212> DNA
   <213> artificial sequence
<220>
   <223> Primer: TEM-164C
<400> 7
   ggatcatgta actcgccttg att 23
<210> 8
   <211> 99
   <212> DNA
   <213> artificial sequence
<220>
   <223> Primer: CTX-M2
<400> 8
   gatatcgcgg ttatctggcc ggaaaaccac gcaccgctgg ttctggtgac ctactttacc 60
   caaccggagc agaaggcgga aagccgtcgg gatattctg 99
<210> 9
   <211> 100
   <212> DNA
   <213> artificial sequence
<220>
   <223> Primer: CTX-M1
<400> 9
   ctatggcacc accaacgata tcgcggtgat ctggccaaaa gatcgtgcgc cgctgattct 60
   ggtcacttac ttcacccagc ctcaacctaa ggcagaaagc 100
<210> 10
   <211> 24
   <212> DNA
   <213> artificial sequence
<220>
   <223> Primer: TEM-164H
<400> 10
   attgggaacc ggagctgaat gaag 24
<210> 11
   <211> 23
   <212> DNA
   <213> artificial sequence
<220>
   <223> Primer: TEM-84I
<400> 11
   atgtggcgcg gtattatccc gta 23
<210> 12
   <211> 22
   <212> DNA
   <213> artificial sequence
<220>
   <223> Primer: TEM-84I
<400> 12
   ttgacgccgg gcaagagcaa ct 22
<210> 13
   <211> 23
   <212> DNA
   <213> artificial sequence
<220>
   <223> Primer: TEM-100S
<400> 13
   cgccgcatac actattctca gag 23
<210> 14
   <211> 23
   <212> DNA
   <213> artificial sequence
<220>
   <223> Primer: TEM-100S
<400> 14
   tgacttggtt gagtactcac cag 23
<210> 15
   <211> 24
   <212> DNA
   <213> artificial sequence
<220>
   <223> Primer: TEM-238G
<400> 15
   gataccgcga gatccacgct cacc 24
<210> 16
   <211> 26
   <212> DNA
   <213> artificial sequence
<220>
   <223> Primer: TEM-238G
<400> 16
   ggctccagat ttatcagcaa taaacc 26
<210> 17
   <211> 24
   <212> DNA
   <213> artificial sequence
<220>
   <223> Primer: TEM-238S
<400> 17
   gataccgcga gatccacgct cact 24
<210> 18
   <211> 21
   <212> DNA
   <213> artificial sequence
<220>
   <223> Primer: SHV-130S
<400> 18
   gtgccgccgc cattaccgtg a 21
<210> 19
   <211> 23
   <212> DNA
   <213> artificial sequence
<220>
   <223> Primer: SHV-130S
<400> 19
   gcgataacag cgccgccaat ctg 23
<210> 20
   <211> 18
   <212> DNA
   <213> artificial sequence
<220>
   <223> Primer: SHV-238G
<400> 20
   cgcgcgcacc ccgttygc 18
<210> 21
   <211> 23
   <212> DNA
   <213> artificial sequence
<220>
   <223> Primer: SHV-238G
<400> 21
   cagctccggt cttatcggcg ata 23
<210> 22
   <211> 23
   <212> DNA
   <213> artificial sequence
<220>
   <223> Primer: SHV-238S
<400> 22
   tagctccggt cttatcggcg ata 23
<210> 23
   <211> 18
   <212> DNA
   <213> artificial sequence
<220>
   <223> Primer: SHV-238A
<400> 23
   cgcgcgcacc ccgttygg 18
<210> 24
   <211> 17
   <212> DNA
   <213> artificial sequence
<220>
   <223> Primer: SHV-240E
<400> 24
   ccgcgcgcac cccgttc 17
<210> 25
   <211> 20
   <212> DNA
   <213> artificial sequence
<220>
   <223> Primer: SHV-240E
<400> 25
   gcyagctccg gtcttatcgg 20
<210> 26
   <211> 17
   <212> DNA
   <213> artificial sequence
<220>
   <223> Primer: SHV-240K
<400> 26
   ccgcgcgcac cccgttt 17
<210> 27
   <211> 19
   <212> DNA
   <213> artificial sequence
<220>
   <223> Primer: SHV-179D
<400> 27
   ttcccggcga cgcccgcga 19
<210> 28
   <211> 22
   <212> DNA
   <213> artificial sequence
<220>
   <223> Primer: SHV-179D
<400> 28
   caccactacc ccggccagca tg 22
<210> 29
   <211> 19
   <212> DNA
   <213> artificial sequence
<220>
   <223> Primer: SHV-179A
<400> 29
   ttcccggcga cgcccgcgc 19
<210> 30
   <211> 19
   <212> DNA
   <213> artificial sequence
<220>
   <223> Primer: SHV-179G
<400> 30
   ttcccggcga cgcccgcgg 19
<210> 31
   <211> 19
   <212> DNA
   <213> artificial sequence
<220>
   <223> Primer: SHV-179N
<400> 31
   ttcccggcga cgcccgcaa 19
<210> 32
   <211> 24
   <212> DNA
   <213> artificial sequence
<220>
   <223> Primer: CTX-M1
<400> 32
   gatgtcactg gctgagctta gcgc 24
<210> 33
   <211> 23
   <212> DNA
   <213> artificial sequence
<220>
   <223> Primer: CTX-M1
<400> 33
   ggccgcgcta cagtacagcg ata 23
<210> 34
   <211> 23
   <212> DNA
   <213> artificial sequence
<220>
   <223> Primer: CTX-M2
<400> 34
   ggcggtgctt aaacagagcg aga 23
<210> 35
   <211> 25
   <212> DNA
   <213> artificial sequence
<220>
   <223> Primer: CTX-M2
<400> 35
   gcgataagca cctgctaaat cagcg 25
<210> 36
   <211> 21
   <212> DNA
   <213> artificial sequence
<220>
   <223> Primer: CTX-M9
<400> 36
   tgcgccgctg gttctggtga c 21
<210> 37
   <211> 26
   <212> DNA
   <213> artificial sequence
<220>
   <223> Primer: CTX-M9
<400> 37
   ctattttacc cagccgcaac agaacg 26
<210> 38
   <211> 22
   <212> DNA
   <213> artificial sequence
<220>
   <223> Primer: CTX-M8&25
<400> 38
   gggwgtggcg ttgattgaca cc 22
<210> 39
   <211> 23
   <212> DNA
   <213> artificial sequence
<220>
   <223> Primer: CTX-M8&25
<400> 39
   gccgataacg cgcagacgct cta 23
<210> 40
   <211> 16
   <212> DNA
   <213> artificial sequence
<220>
   <223> Primer: TEM-F
<400> 40
   gcggaacccc tatttg 16
<210> 41
   <211> 20
   <212> DNA
   <213> artificial sequence
<220>
   <223> Primer: TEM-R
<400> 41
   accaatgctt aatcagtgag 20
<210> 42
   <211> 18
   <212> DNA
   <213> artificial sequence
<220>
   <223> Primer: SHV-F1
<400> 42
   ctttactcgc ctttatcg 18
<210> 43
   <211> 19
   <212> DNA
   <213> artificial sequence
<220>
   <223> Primer: SHV-R1
<400> 43
   tcccgcagat aaatcacca 19
<210> 44
   <211> 26
   <212> DNA
   <213> artificial sequence
<220>
   <223> Primer: CTX-M-F
<400> 44
   atgtgcagya ccagtaargt katggc 26
<210> 45
   <211> 29
   <212> DNA
   <213> artificial sequence
<220>
   <223> Primer: CTX-M-R
<400> 45
   tgggtraart argtsaccag aaysagcgg 29
<210> 46
   <211> 20
   <212> DNA
   <213> artificial sequence
<220>
   <223> Primer: CTX-M-10-1F
<400> 46
   atggttaaaa aatcactgcg 20
<210> 47
   <211> 17
   <212> DNA
   <213> artificial sequence
<220>
   <223> Primer: CTX-M-10-4R
<400> 47
   aaaccgttgg tgacgat 17
<210> 48
   <211> 20
   <212> DNA
   <213> artificial sequence
<220>
   <223> Primer: CTX-M-2F
<400> 48
   atgatgactc agagcattcg 20
<210> 49
   <211> 20
   <212> DNA
   <213> artificial sequence
<220>
   <223> Primer: CTX-M-2R
<400> 49
   ttattgcatc agaaaccgtg 20
<210> 50
   <211> 20
   <212> DNA
   <213> artificial sequence
<220>
   <223> Primer: CTX-Mgp8-F
<400> 50
   tgatgagaca tcgcgttaag 20
<210> 51
   <211> 20
   <212> DNA
   <213> artificial sequence
<220>
   <223> Primer: CTX-Mgp8-R
<400> 51
   taaccgtcgg tgacgatttt 20
<210> 52
   <211> 22
   <212> DNA
   <213> artificial sequence
<220>
   <223> Primer: CTX-M-9-1F
<400> 52
   tggtgacaaa gagagtgcaa cg 22
<210> 53
   <211> 18
   <212> DNA
   <213> artificial sequence
<220>
   <223> Primer: CTX-M-9-MF
<400> 53
   ggaggcgtga cggctttt 18
<210> 54
   <211> 100
   <212> DNA
   <213> artificial sequence
<220>
   <223> Primer: TEM wild type 104
<400> 54
   gcaagagcaa ctcggtcgcc gcatacacta ttctcagaat gacttggttg agtactcacc      60
   agtcacagaa aagcatctta cggatggcat gacagtaaga                          100
<210> 55
   <211> 100
   <212> DNA
   <213> artificial sequence
<220>
   <223> Primer: TEM wild type 164
<400> 55
   gctaaccgct tttttgcaca acatggggga tcatgtaact cgccttgatc gttgggaacc      60
   ggagctgaat gaagccatac caaacgacga gcgtgacacc                          100
<210> 56
   <211> 100
   <212> DNA
   <213> artificial sequence
<220>
   <223> Primer: TEM wild type 238
<400> 56
   ttaccatctg gccccagtgc tgcaatgata ccgcgagacc cacgctcacc ggctccagat      60
   ttatcagcaa taaaccagcc agccggaagg gccgagcgca                          100
<210> 57
   <211> 100
   <212> DNA
   <213> artificial sequence
<220>
   <223> Primer: SHV wild type 238/240
<400> 57
   gggccaagca gggcgacaat gccgcgcgca ccccgctcgc cagctccggt cttatcggcg      60
   ataaaccagc ccgccggcag cacggagcgg atcaacggtc                          100
<210> 58
   <211> 100
   <212> DNA
   <213> artificial sequence
<220>
   <223> Primer: OXA 48 I
<400> 58
   gcattaagca aggggacgtt atgcgtgtat tagccttatc ggctgtgttt ttggtggcat 60
   cgattatcgg aatgcctgcg gtagcaaagg aatggcaaga 100
<210> 59
   <211> 100
   <212> DNA
   <213> artificial sequence
<220>
   <223> Primer: OXA 48 II
<400> 59
   aacttgatga taatgtgtgg ttttttgcga tgaatatgga tatgcccaca tcggatggtt 60
   tagggctgcg ccaagccatc acaaaagaag tgctcaaaca 100
<210> 60
   <211> 100
   <212> DNA
   <213> artificial sequence
<220>
   <223> Primer: VIM 552 C
<400> 60
   gaacggcaca accaccgtat agcacgttcg ctgacgggac gtatacaacc agattgtcgg 60
   tcgaatgcgc agcaccagga tagaagagct ctactggacc 100
<210> 61
   <211> 100
   <212> DNA
   <213> artificial sequence
<220>
   <223> Primer: VIM 246
<400> 61
   tctacccgtc caatggtctc attgtccgtg atggtgatga gttgcttttg attgatacag 60
   cgtggggtgc gaaaaacaca gcggcacttc tcgcggagat 100
<210> 62
   <211> 100
   <212> DNA
   <213> artificial sequence
<220>
   <223> Primer: IMP 114
<400> 62
   ttgccagatt taaaaattga aaagcttgat gaaggcgttt atgttcatac ttcgtttgaa      60
   gaagttaacg ggtggggcgt tgttcctaaa catggtttgg                           100
<210> 63
   <211> 100
   <212> DNA
   <213> artificial sequence
<220>
   <223> Primer: IMP 4801/11
<400> 63
   tttttatcca ggcccagggc acactcaaga taacgtagtg gtttggttac ctgaaaagaa      60
   aattttattc ggtggttgct ttgttaaacc ggacggtctt                           100
<210> 64
   <211> 100
   <212> DNA
   <213> artificial sequence
<220>
   <223> Primer: SHV all
<400> 64
   cgcagtgctg gcgcgggtgg atgccggtga cgaacagctg gagcgaaaga tccactatcg      60
   ccagcaggat ctggtggact actcgccggt cagcgaaaaa                           100
<210> 65
   <211> 100
   <212> DNA
   <213> artificial sequence
<220>
   <223> Primer: NDM-1 75
<400> 65
   gcagcttgtc ggccatgcgg gccgtatgag tgattgcggc gcggctatcg ggggcggaat      60
   ggctcatcac gatcatgctg gccttgggga acgccgcacc                           100
<210> 66
   <211> 100
   <212> DNA
   <213> artificial sequence
<220>
   <223> Primer: NDM-1 392
<400> 66
   cgccgcaacc atcccctctt gcggggcaag ctggttcgac aacgcattgg cataagtcgc 60
   aatccccgcc gcatgcagcg cgtccatacc gcccatcttg 100
<210> 67
   <211> 100
   <212> DNA
   <213> artificial sequence
<220>
   <223> Primer: CMY II 304
<400> 67
   atgccgatat cgttaatcgc accatcaccc cgttgatgca ggagcaggct attccgggta 60
   tggccgttgc cgttatctac cagggaaaac cctattattt 100
<210> 68
   <211> 100
   <212> DNA
   <213> artificial sequence
<220>
   <223> Primer: CMY II 1199
<400> 68
   ttgaggtaaa cccgcccgcc cccgcagtga aagcctcatg ggtgcataaa acgggctcca 60
   ctggtggatt tggcagctac gtagccttcg ttccagaaaa 100
<210> 69
   <211> 100
   <212> DNA
   <213> artificial sequence
<220>
   <223> Primer: CMY I MOX
<400> 69
   tcaaggagca caggatcccg ggcatggcgg tggccgtgct caaggatggc aaggcccact 60
   acttcaatta cggggtggcc aaccgggaga gcggggccgg 100
<210> 70
   <211> 100
   <212> DNA
   <213> artificial sequence
<220>
   <223> Primer: ACC 660
<400> 70
   taggcatgca tcacagctac ttgaaggttc cggctgacca gatggaaaac tatgcgtggg 60
   gctacaacaa gaaagatgag ccagtgcacg tgaatatgga 100
<210> 71
   <211> 100
   <212> DNA
   <213> artificial sequence
<220>
   <223> Primer: MIR ACT
<400> 71
   ggatgaggtc acggataacg cctctctgtt gcgcttttat caaaactggc agccgcagtg 60
   gaagccgggc accacgcgtc tttacgccaa tgccagcatc 100
<210> 72
   <211> 100
   <212> DNA
   <213> artificial sequence
<220>
   <223> Primer: DHA 753
<400> 72
   ttgatgcgga atcttacggc gtgaaatccg cctcaaaaga tatgctgcgc tgggcggaaa 60
   tgaatatgga gccgtcacgg gccggtaatg cggatctgga 100
<210> 73
   <211> 100
   <212> DNA
   <213> artificial sequence
<220>
   <223> Primer: DHA 1110
<400> 73
   cgcctatgtc gcctttattc cggaaaaaca ggtggcgatt gtgattctgg cgaataaaaa 60
   ctacccgaat accgaaagag tcaaagctgc acaggctatt 100
<210> 74
   <211> 100
   <212> DNA
   <213> artificial sequence
<220>
   <223> Primer: TEM all
<400> 74
   gcaaactatt aactggcgaa ctacttactc tagcttcccg gcaacaatta atagactgga 60
   tggaggcgga taaagttgca ggaccacttc tgcgctcggc 100
<210> 75
   <211> 100
   <212> DNA
   <213> artificial sequence
<220>
   <223> Primer: FOX
<400> 75
   ggttccgcct tggatggtgt gaccatggcc gagcttgcca cctacagtgc gggtggtttg 60
   ccgctgcagt tccccgatga ggtggattcg aatgacaaga 100
<210> 76
   <211> 100
   <212> DNA
   <213> artificial sequence
<220>
   <223> Primer: KPC I
<400> 76
   cttgtctctc atggccgctg gctggctttt ctgccaccgc gctgaccaac ctcgtcgcgg 60
   aaccattcgc taaactcgaa caggactttg gcggctccat 100
<210> 77
   <211> 100
   <212> DNA
   <213> artificial sequence
<220>
   <223> Primer: KPC II
<400> 77
   tgacggtggc ggagctgtcc gcggccgccg tgcaatacag tgataacgcc gccgccaatt 60
   tgttgctgaa ggagttgggc ggcccggccg ggctgacggc 100
<210> 78
   <211> 100
   <212> DNA
   <213> artificial sequence
<220>
   <223> Primer: CTX-M 8 +25
<400> 78
   ccaccggcgc tgccagcatt caggctgggc tacccacatc gtgggttgtc ggggataaaa 60
   ccggcagcgg tgattatggt acgacgaatg acatcgccgt 100
<210> 79
   <211> 100
   <212> DNA
   <213> artificial sequence
<220>
   <223> Primer: CTX-M9 +64
<400> 79
   gcggccgcgc tcagttctgc cagcgtcatt gtgccgttga cgtgtttttc ggcaatcgga 60
   ttgtagttaa ccagatcggc aggcttgatc tcgacaggct 100
<210> 80
   <211> 36
   <212> DNA
   <213> artificial sequence
<220>
   <223> cDET-1 beacon
<400> 80
   cgctgccttt cgagagaacc ggcttcgaag gcagcg 36
<210> 81
   <211> 24
   <212> DNA
   <213> artificial sequence
<220>
   <223> cDET-1 TaqMan
<400> 81
   ctttcgagag aaccggcttc gaag 24
<210> 82
   <211> 36
   <212> DNA
   <213> artificial sequence
<220>
   <223> cDET-2 beacon
<400> 82
   cgctgccagt ctgaacgcaa ctctgctgat gcagcg 36
<210> 83
   <211> 24
   <212> DNA
   <213> artificial sequence
<220>
   <223> cDET-2 TaqMan
<400> 83
   cagtctgaac gcaactctgc tgat 24
<210> 84
   <211> 36
   <212> DNA
   <213> artificial sequence
<220>
   <223> cDET-3 beacon
<400> 84
   cgctgccagt ctagagaacc ggctgctgat gcagcg 36
<210> 85
   <211> 24
   <212> DNA
   <213> artificial sequence
<220>
   <223> cDET-3 TaqMan
<400> 85
   cagtctagag aaccggctgc tgat 24